(19) Europäisches Patentamt
European Patent Office
Office européen des brevets

(11) EP 2 614 161 B1

(12) **EUROPEAN PATENT SPECIFICATION**

(45) Date of publication and mention of the grant of the patent:
**04.11.2020 Bulletin 2020/45**

(21) Application number: **11824184.3**

(22) Date of filing: **09.09.2011**

(51) Int Cl.:
***G16B 20/00*** *(2019.01)*

(86) International application number:
**PCT/US2011/051017**

(87) International publication number:
**WO 2012/034030 (15.03.2012 Gazette 2012/11)**

(54) **VARIANT ANNOTATION, ANALYSIS AND SELECTION TOOL**

WERKZEUG ZUR KOMMENTIERUNG, ANALYSE UND AUSWAHL VON VARIANZEN

ANNOTATION, ANALYSE ET OUTIL DE SÉLECTION DE VARIANTS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.09.2010 US 381239 P**

(43) Date of publication of application:
**17.07.2013 Bulletin 2013/29**

(60) Divisional application:
**20198183.4**

(73) Proprietors:
- **Fabric Genomics, Inc.**
  **Oakland, CA 94612 (US)**
- **University of Utah Research Foundation**
  **Salt Lake City, UT 84108 (US)**

(72) Inventors:
- **YANDELL, Mark**
  **Salt Lake City, UT 84132 (US)**
- **REESE, Martin**
  **Oakland, CA 94611 (US)**
- **HUFF, Chad**
  **Pearland, TX 77584 (US)**
- **HU, Hao**
  **Shoreline, WA 98177 (US)**
- **MOORE, Marvin**
  **Salt Lake City, UT 84109 (US)**

(74) Representative: **Avidity IP**
**Broers Building**
**Hauser Forum**
**21 JJ Thomson Avenue**
**Cambridge CB3 0FA (GB)**

(56) References cited:
**US-A1- 2006 052 945**
**US-A1- 2006 111 849**
**US-A1- 2007 111 247**
**US-A1- 2009 183 268**
**US-A1- 2010 132 067**

- **M. YANDELL ET AL: "A probabilistic disease-gene finder for personal genomes", GENOME RESEARCH, vol. 21, no. 9, 23 June 2011 (2011-06-23), pages 1529-1542, XP055202729, ISSN: 1088-9051, DOI: 10.1101/gr.123158.111**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 614 161 B1

## Description

Manual analysis of personal genome sequences is a massive, labor-intensive task. Although much progress is being made in DNA sequence read alignment and variant calling, little software yet exists for the automated analysis of personal genome sequences. Indeed, the ability to automatically annotate variants, to combine data from multiple projects, and to recover subsets of annotated variants for diverse downstream analyses is becoming a critical analysis bottleneck.

Researchers are now faced with multiple whole genome sequences, each of which has been estimated to contain around 4 million variants. This creates a need to efficiently prioritize variants so as to efficiently allocate resources for further downstream analysis, such as external sequence validation, additional biochemical validation experiments, further target validation such as that performed routinely in a typical Biotech/Pharma discovery effort, or in general additional variant validation. Such relevant variants are also called phenotype-causing genetic variants.

M. Yandell et al, Genome Research, (20110623), vol. 21, no. 9 discloses a probabilistic disease-gene finder for personal genomes. US2009183268A1 discloses methods of identifying elements associated with a trait, such as a disease.

The claimed invention provides a computer implemented method for identifying disease-causing genetic variants within a feature, comprising:

a) providing a genome variant file from at least one individual;
b) annotating the variants, including annotation with respect to their impact on existing genome annotations, wherein the annotating is in respect to:

- whether the variant is coding, non-coding, synonymous, non-synonymous, loss-of-function, stop codon causing, changes top regulatory elements, splice sites, or
- known variant frequencies in a population;

c) selecting a feature, wherein the feature comprises any span or collection of spans of a genome sequence or transcriptome sequence, and wherein the feature comprises a gene, a transcript, an exon, an intron, a UTR, a genetic locus, an extended gene region including regulatory elements, a list of 2 or more genes, a genetic pathway, an ontology category, or a combination thereof; and
d) scoring the variants within the feature(s), wherein the scoring:

i) compares composite allele frequencies in target genome(s) for the selected features in the genome variant file with the corresponding allele frequencies in a background database for selected features;
ii) computes a statistical probability of each variant and as a composite in the selected feature; and
iii) ranks variants and their corresponding features according to the statistical probability of each variant and/or of composite feature genotype(s);

and wherein the scoring is based on calculating a composite likelihood ratio.

We also disclose methods for identifying phenotype-causing genetic variants within a feature comprising: (a) a genome variant file from at least one individual; (b) annotating the variants, including annotation with respect to their impact on existing genome annotations, eg coding, non-coding, synonymous, non-synonymous, loss-of-function, stop codon causing, changes to regulatory elements, splice sites, known variant frequencies in a population; (c) selecting a feature such as all genes, a subset of genes, their upstream regions and other functional units; and (d) scoring the variants within the feature(s) wherein the scoring: (i) compares composite allele frequencies in the target genome(s) for the chosen features in the genome variant file with the corresponding allele frequencies in a background database for chosen features; (ii) computes the statistical probability of each variant and as a composite in a feature ; and (iii) ranks variants and their corresponding features according to the statistical probability of each variant and/or of the composite feature genotype(s). In one embodiment, the genome variant file is from whole genome sequencing, exome sequencing, transcriptome sequencing, chip and bead-type genotyping or a combination of these. In one embodiment, the genome variant files include multiple individuals. In one embodiment, the multiple individuals are genetically related. In one embodiment, the pedigree information is included in the analysis. In one embodiment, the genome variant file comes from a tumor sample. In one embodiment, the reference database comes from the normal genome. In one embodiment, the genome variant file is from a set of cases all with the known phenotype. In one embodiment, the reference database is from a set of controls without the known phenotype. In one embodiment, variant frequencies are derived from various disease variant databases such as OMIM. In one embodiment, ontology and pathway information is incorporated into the scoring. In one embodiment, the phenotype is a human disease. In one embodiment, the phenotype-causing variants identify disease genes.

We also disclose methods for identifying phenotype-causing genetic variants comprising: (a) computer processing instructions that prioritize genetic variants by combining (i) variant frequency, (ii) one or more sequence characteristics and (iii) a summing procedure; and (b) automatically identifying and reporting the phenotype-causing genetic variants. In one embodiment, at least one of said sequence characteristics comprises an amino acid substitution (AAS), a splice site, a promoter, a protein binding site, an enhancer, or a repressor. In one embodiment, the summing procedure comprises calculating a log-likelihood ratio ($\lambda$). In one embodiment, the variant frequency and the sequence characteristics are aggregately scored within a genomic feature. In one embodiment, the genomic feature is one or more user-defined regions of the genome. In one embodiment, the genomic feature comprises one or more genes or gene fragments, one or more chromosomes or chromosome fragments, one or more exons or exon framents, one or more introns or intron fragments, one or more regulatory sequences or regulatory sequence fragments, or a combination thereof. In some embodiments, the method further (c) scoring both coding and non-coding variants; and (d) evaluating the cumulative impact of both types of variants simultaneously. In one embodiment, the method incorporates both rare and common variants to identify variants responsible for common phenotypes. In one embodiment, the common phenotype is a common disease.

In one embodiment, the method identifies rare variants causing rare phenotypes. In one embodiment, the rare phenotype is a rare disease. In one embodiment, the method has a statistical power at least 10 times greater than the statistical power of a method not combining variant frequency and one or more sequence characteristics. In one embodiment, the method comprises assessing the cumulative impact of variants in both coding and non-coding regions of the genome. In one embodiment, the method analyzes low-complexity and repetitive genome sequences. In one embodiment, the method comprises analyzing pedigree data. In one embodiment, the method comprises phased genome data. In one embodiment, family information on affected and non-affected individuals are included in the target and background database.

In one embodiment, the method comprising a training algorithm. In one embodiment, the method comprises comparing genomic data in a background and a target file. In one embodiment, the background and target files contain the variants observed in control and case genomes, respectively. In one embodiment, the method comprises calculating a composite likelihood ratio (CLR) to evaluate whether a genomic feature contributes to a phenotype In one embodiment, the method comprises calculating the likelihood of a null and alternative model assuming independence between nucleotide sites and then evaluating the significance of the likelihood ratio by permutation to control for LD.

In one embodiment, the method comprises carrying out a nested CLR test that depends only on differences in allele frequencies between affected and unaffected individuals. In one embodiment, sites with rare minor alleles are collapsed into one or more categories, and the total number of minor allele copies among all affected and unaffected individuals is counted rather than just the presence or absence of minor alleles within an individual. In one embodiment, a collapsing threshold is set at fewer than 5 copies of the minor allele among all affected individuals.

In one embodiment, the method comprises determining k as the number of uncollapsed variant sites among $n_i^U$ unaffected and $n_i^A$ affected individuals, with $n_i$ equal to $n_i^U$+$n_i^A$. Let $l_{k+1}..l_{k+m}$ equal the number of collapsed variant sites within m collapsing categories labeled k+1 to m, and let $l_1..l_k$ equal 1. In one embodiment, the method comprises determining $X_i$, $X_i^U$, and $X_i^A$ as the number of copies of the minor allele(s) at variant site i or collapsing category i among all individuals, unaffected individuals, and affected individuals, respectively. In one embodiment, the method comprises calculating the log-likelihood ratio as:

$$\textit{Equation 1.}\quad \lambda = \ln\left(\frac{L_{Null}}{L_{Alt}}\right) = \sum_{i=1}^{k+m} \ln\left[\frac{\left(\hat{p}_i\right)^{X_i}\left(1-\hat{p}_i\right)^{2l_i n_i - X_i}}{\left(\hat{p}_i^U\right)^{X_i^U}\left(1-\hat{p}_i^U\right)^{2l_i n_i^U - X_i^U}\left(\hat{p}_i^A\right)^{X_i^A}\left(1-\hat{p}_i^A\right)^{2l_i n_i^A - X_i^A}}\right]$$

where $p_i$, $p_i^U$, and $p_i^A$ equal the maximum likelihood estimates for the frequency of minor allele(s) at variant site i or collapsing category i among all individuals, unaffected individuals, and affected individuals, respectively. In one embodiment, when no constraints are placed on the frequency of disease-causing variants, the maximum likelihood estimates are equal to the observed frequencies of the minor allele(s). In one embodiment, the method comprises reporting $\log_e$ of the $\chi^2$ p-value as the score to provide a statistic for rapid prioritization of disease-gene candidates wherein variant sites are unlinked, and wherein $-2\lambda$ approximately follows a $\chi^2$ distribution with $k+m$ degrees of freedom. In one embodiment, the method comprises incorporating multiple categories of indels, splice-site variants, synonymous variants, and non-coding variants. In one embodiment, the method comprises incorporating information about the severity of amino acid changes. In one embodiment, the method comprises including an additional parameter in the null and alternative model for each variant site or collapsing category. In one embodiment, the parameter $h_i$ in the null model is the likelihood that the amino acid change does not contribute to disease risk. In one embodiment, the method comprises estimating $h_i$ by setting it equal to the proportion of corresponding type of amino acid change in the population background. In one embodiment, the method comprises determining $a_i$ as the likelihood that the amino acid change contributes to disease risk. In one embodiment, the method comprises estimating $a_i$ by setting it equal to the proportion of a corresponding type of amino acid change among all disease-causing mutations in a selected study population. In one embodiment, the log likelihood ration $\lambda$ is calculated as:

$$\lambda = \ln\left(\frac{L_{Null}}{L_{Alt}}\right) = \sum_{i=1}^{k+m} \ln\left[\frac{h_i(\hat{p}_i)^{X_i}(1-\hat{p}_i)^{2l_i n_i - X_i}}{a_i(\hat{p}_i^U)^{X_i^U}(1-\hat{p}_i^U)^{2l_i n_i^U - X_i^U}(\hat{p}_i^A)^{X_i^A}(1-\hat{p}_i^A)^{2l_i n_i^A - X_i^A}}\right].$$

Equation 2.

In one embodiment, scoring non-coding variants and synonymous variants within coding regions comprises estimating the relative impacts of non-coding and synonymous variants using the vertebrate-to-human genome multiple alignments. In one embodiment, for each codon in the human genome, the frequency in which it aligns to other codons in primate genomes (wherever an open reading frame (ORF) in the corresponding genomes is available) is calculated. In one embodiment, the method comprises incorporating inheritance information. In one embodiment, inheritance information is incorporated based on a recessive model, a recessive with complete penetrance model, a monogenic recessive model or a combination thereof. In one embodiment, the method comprises variant masking wherein the user excludes a list of nucleotide sites from the log-likelihood calculations based on information obtained prior to the genome analysis. In one embodiment, the method comprises excluding both *de novo* mutations and sequencing errors, for genomes with an error rate of approximately 1 in 100,000, approximately 99.9% of all Mendelian inheritance errors are genotyping errors.

Additional advantages disclosed herein will be set forth in part in the description which follows, and in part will be obvious from the description, or may be learned by practice of the methods described herein. The advantages disclosed herein can be realized and attained by means of the elements and combinations particularly pointed out in the appended claims. It is to be understood that both the foregoing general description and the following detailed description are exemplary and are not restrictive the claims.

## BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

**Figure 1** illustrates a feature-based approach to prioritization in VAAST. Variants along with frequency information, e.g. 0.5:A 0.5:T, are grouped into user-defined features (black box, gene X). These features can be genes, sliding windows, conserved sequence regions, etc. Variants within the bounds of a given feature (dashed box) are then scored to give a *composite* likelihood for the observed genotypes at that feature under a healthy and disease model by comparing variant frequencies in the cases (target) compared to control (background) genomes. Variants producing non-synonymous amino acid changes are simultaneously scored under a healthy and disease model.

**Figure 2 (A-C)** illustrates observed Amino Acid substitution frequencies compared to BLOSUM62. Amino acid substitution frequencies observed in healthy and reported for OMIM disease alleles were converted to a LOD based scores for purposes of comparison to BLOSUM62. The BLOSUM62 scores are plotted on Y-axis throughout. Boxed circles: stops; unboxed circles: all other amino acid changes. Diameter of circles is proportional to the number of changes with that score. A. BLOSUM62 scoring compared to itself. Perfect correspondence would produce the diagonally arranged circles shown. B. Frequencies of amino acid substitutions in 10 healthy genomes compared to BLOSUM62. C. OMIM non-synonymous variant frequencies compared to BLOSUM62.

**Figure 3 (A-B)** illustrates the impact of population stratification and platform bias. Numbers of false positives with and without masking. A. Effect of population stratification. B. Effect of heterogeneous platform and variant calling procedures. Dark grey line: number of false positives without masking ("No Masking"); light grey line: after masking ("Masking"). Note that although masking has little effect on population stratification, it has a much larger impact on platform bias. This is an important behavior: population stratification introduces real, but confounding signals into disease gene searches; these signals are unaffected by masking (A); in contrast, VAAST's masking option removes false positives due to noise introduced by systematic errors in platform and variant calling procedures (B).

**Figure 4** illustrates genome-wide VAAST analysis of Utah Miller Syndrome Quartet. VAAST was run in its quartet mode, using the genomes of the two parents to improve specificity when scoring the two affected siblings. Grey bars along the center of each chromosome show proportion of unique sequence along the chromosome arms, with white denoting completely unique sequence; black regions thus outline centromeric regions. Grey bars above and below the chromosomes represent each annotated gene; plus strand genes are shown above and minus strand genes below; their width is proportional their length; height of bar, their VAAST score. Genes identified are candidates identified by VAAST. Only two genes are identified in this case: *DNAH5* and *DHODH.* The superior, significant performance enhancement of VAAST versus others programs such as the one used in the original Miller Syndrome publication (>20 potential candidates) allows for "diagnostic" like clinical decision support for doctors because of the clear accuracy and signal to noise ratio of true positives to true negatives demonstrated in this figure. Causative allele incidence was set to 0.00035, and amino acid substitution frequency was used along with variant-masking. This view was generated using the VAAST report viewer. This software tool allows the visualization of a genome-wide search in easily interpretable form, graphically displaying chromosomes, genes and their VAAST scores.

**Figure 5 (A-B)** illustrates a benchmark analysis using 100 different known disease genes. In each panel the y-axis denotes the average rank of the disease-gene among 100 searches for 100 different disease genes. Heights of boxes are proportional to the mean rank, with the number above each box denoting the mean rank of the disease gene among all ReSeq annotated Human genes. Error bars encompass the maximum and minimum observed ranks for 95% of the trials. **5A. Average ranks for 100 different VAAST searches.** Left half of panel shows the results for genome-wide searches for 100 different disease-genes assuming dominance using a case cohort of 2, 4, and 6 unrelated individuals. Right half of panel shows the results for genome-wide searches for 100 different recessive disease genes using a case cohort of 1, 2, and 3. **5B. Impact of missing data on VAAST performance.** Left and right half of panel shows results for dominant and recessive gene searches as in panel A, except in this panel the case cohorts contain differing percentages of individuals with no disease causing variants in the disease-gene. **5C. Comparison of VAAST performance to that of ANNOVAR and SIFT.** Left half of panel shows the results for genome-wide searches using VAAST, ANNOVAR and SIFT to search for 100 different dominant disease genes using a case cohort of 6 unrelated individuals. Right half of panel shows the results for genome-wide searches using VAAST, ANNOVAR and SIFT to search for 100 different recessive disease genes using a case cohort of 3 unrelated individuals.

**Figure 6 (A-B)** illustrates statistical power as a function of number of target genomes for two common-disease genes. A. NOD2, using a dataset containing rare and common non-synonymous variants. B. LPL, using a dataset containing only rare non-synonymous variants. For each data point, power is estimated from 500 bootstrapped resamples of the original datasets, with $\alpha=2.4\times10^{-6}$ except where specified. Y-axis: probability of identifying gene as implicated in disease in a genome-wide search. X-axis: number of cases. The number of controls is equal to the number of cases up to a maximum of 327 for LPL (original dataset) and 163 for NOD2 (original dataset + 60 Europeans from 1000 Genomes). VAAST + OMIM: VAAST using AAS data from OMIM as its disease model; WSS: weighted sum score of Madsen and Browning, 2009, PLoS Genet 5. GWAS: single variant GWAS analysis. NOD2 and LPL datasets were taken from Lesage et al. (2002) Am J Hum Genet 70, 845-857; and Johansen, et al. (2010), Nat Genet 42, 684-687 respectively.

**Figure 7** illustrates a VAAST search procedure. One or more variant files (in VCF or GVF format) are first annotated using the VAAST annotation tool and a GFF3 file of genome annotations. Multiple target and background variant files are then combined by the VAAST annotation tool into a single condenser file; these two files, one for the background and one for the target genomes, together with a GFF3 file containing the genomic features to be searched are then passed to VAAST. VAAST outputs a simple text file, which can also be viewed in the VAAST viewer.

## DETAILED DESCRIPTION

The present disclosure may be understood more readily by reference to the following detailed description, the Examples included therein and to the Figures and their previous and following description.

Before the present methods are disclosed and described, it is to be understood that this disclosure is not limited to specific embodiments. It is also to be understood that the terminology used herein is for the purpose of describing particular embodiments only and is not intended to be limiting. The following description and examples illustrate some exemplary embodiments of the disclosure in detail. Those of skill in the art will recognize that there are numerous variations and modifications of this disclosure that are encompassed by its scope. Accordingly, the description of a certain exemplary embodiment should not be deemed to limit the scope of the present disclosure.

The present disclosure relates to methods for the identification of phenotype-causing variants. The methods can comprise the comparison of polynucleotide sequences between a case, or target cohort, and a background, or control, cohort. Phenotype-causing variants can be scored within the context of one or more features. Variants can be coding or non-coding variants. The methods can employ a feature-based approach to prioritization of variants. The feature-based approach can be an aggregative approach whereby all the variants within a given feature are considered for their cumulative impact upon the feature (*e.g.*, a gene or gene product). Therefore, the method also allows for the identification of features such as genes or gene products. Prioritization can employ variant frequency information, sequence characteristics such as amino acid substitution effect information, phase information, pedigree information, disease inheritance models, or a combination thereof.

"Nucleic acid" and "polynucleotide" can be used interchangeably herein, and refer to both RNA and DNA, including cDNA, genomic DNA, synthetic DNA, and DNA or RNA containing nucleic acid analogs. Polynucleotides can have any three-dimensional structure. A nucleic acid can be double-stranded or single-stranded (*e.g.*, a sense strand or an antisense strand). Non-limiting examples of polynucleotides include chromosomes, chromosome fragments, genes, intergenic regions, gene fragments, exons, introns, messenger RNA (mRNA), transfer RNA, ribosomal RNA, siRNA, micro-RNA, ribozymes, cDNA, recombinant polynucleotides, branched polynucleotides, nucleic acid probes and nucleic acid primers. A polynucleotide may contain unconventional or modified nucleotides.

"Nucleotides" are molecules that when joined together for the structural basis of polynucleotides, *e.g.,* ribonucleic acids (RNA) and deoxyribonucleic acids (DNA). A "nucleotide sequence" is the sequence of nucleotides in a given polynucleotide. A nucleotide sequence can also be the complete or partial sequence of an individual's genome and can therefore encompass the sequence of multiple, physically distinct polynucleotides (*e.g.*, chromosomes).

An "individual" can be of any species of interest that comprises genetic information. The individual can be a eukaryote, a prokaryote, or a virus. The individual can be an animal or a plant. The individual can be a human or non-human animal.

The "genome" of an individual member of a species can comprise that individual's complete set of chromosomes, including both coding and non-coding regions. Particular locations within the genome of a species are referred to as "loci", "sites" or "features". "Alleles" are varying forms of the genomic DNA located at a given site. In the case of a site where there are two distinct alleles in a species, referred to as "A" and "B", each individual member of the species can have one of four possible combinations: AA; AB; BA; and BB. The first allele of each pair is inherited from one parent, and the second from the other.

The "genotype" of an individual at a specific site in the individual's genome refers to the specific combination of alleles that the individual has inherited. A "genetic profile" for an individual includes information about the individual's genotype at a collection of sites in the individual's genome. As such, a genetic profile is comprised of a set of data points, where each data point is the genotype of the individual at a particular site.

Genotype combinations with identical alleles (*e.g.,* AA and BB) at a given site are referred to as "homozygous"; genotype combinations with different alleles (*e.g.,* AB and BA) at that site are referred to as "heterozygous." It has to be noted that in determining the allele in a genome using standard techniques AB and BA cannot be differentiated, meaning it is impossible to determine from which parent a certain allele was inherited, given solely the genomic information of the individual tested. Moreover, variant AB parents can pass either variant A or variant B to their children. While such parents may not have a predisposition to develop a disease, their children may. For example, two variant AB parents can have children who are variant AA, variant AB, or variant BB. For example, one of the two homozygotic combinations in this set of three variant combinations may be associated with a disease. Having advance knowledge of this possibility can allow potential parents to make the best possible decisions about their children's health.

An individual's genotype can include haplotype information. A "haplotype" is a combination of alleles that are inherited or transmitted together. "Phased genotypes" or "phased datasets" provide sequence information along a given chromosome and can be used to provide haplotype information.

The "phenotype" of an individual refers to one or more characteristics. An individual's phenotype can be driven by constituent proteins in the individual's "proteome", which is the collection of all proteins produced by the cells comprising the individual and coded for in the individual's genome. The proteome can also be defined as the collection of all proteins expressed in a given cell type within an individual. A disease or disease-state can be a phenotype and can therefore be associated with the collection of atoms, molecules, macromolecules, cells, tissues, organs, structures, fluids, metabolic, respiratory, pulmonary, neurological, reproductive or other physiological function, reflexes, behaviors and other physical characteristics observable in the individual through various means.

In many cases, a given phenotype can be associated with a specific genotype. For example, an individual with a certain pair of alleles for the gene that encodes for a particular lipoprotein associated with lipid transport may exhibit a phenotype characterized by a susceptibility to a hyperlipidemous disorder that leads to heart disease.

The "background" or "background database" can be a collection of nucleotide sequences (*e.g.,* one or more genes or gene fragments, one or more chromosomes or chromosome fragments, one or more genomes or genome fragments, one or more transcriptome sequences, *etc.*) and their variants (variant files) used to derive reference variant frequencies in the background sequences. The background database can contain any number of nucleotide sequences and can vary based upon the number of available sequences. The background database can contain about 1-10000, 1-5000, 1-2500, 1-1000, 1-500, 1-100, 1-50, 1-10, 10-10000, 10-5000, 10-2500, 10-1000, 10-500, 10-100, 10-50, 50-10000, 50-5000, 50-2500, 50-1000, 50-500, 50-100, 100-10000, 100-5000, 100-2500, 100-1000, 100-500, 500-10000, 500-5000, 500-2500, 500-1000, 1000-10000, 1000-5000, 1000-2500, 2500-10000, 2500-5000, or 5000-10000 sequences, or any included sub-range; for example, about 1, 2, 3, 4, 5, 6, 7, 8, 9, 10, 15, 20, 25, 30, 35, 40, 45, 50, 60, 70, 80, 90, 100, 150, 200, 250, 300, 350, 400, 450, 500, 600, 700, 800, 900, 1000, 1250, 1500, 1750, 2000, 2500, 3000, 3500, 4000, 4500, 5000, 6000, 7000, 8000, 9000, 10000, or more sequences, or any intervening integer.

The "target" or "case" can be a collection of nucleotide sequences (*e.g.*, one or more genes or gene fragments, one or more genomes or genome fragments, one or more transcriptome sequences, *etc.*) and their variants under study. The target can contain information from individuals that exhibit the phenotype under study. The target can be a personal genome sequence or collection of personal genome sequences. The personal genome sequence can be from an individual diagnosed with, suspected of having, or at increased risk for a disease. The target can be a tumor genome sequence. The target can be genetic sequences from plants or other species that have desirable characteristics.

The term "cohort" can be used to describe a collection of target or background sequences, and their variants, used in a given comparison. A cohort can include about 1-10000, 1-5000, 1-2500, 1-1000, 1-500, 1-100, 1-50, 1-10, 10-10000, 10-5000, 10-2500, 10-1000, 10-500, 10-100, 10-50, 50-10000, 50-5000, 50-2500, 50-1000, 50-500, 50-100, 100-10000, 100-5000, 100-2500, 100-1000, 100-500, 500-10000, 500-5000, 500-2500, 500-1000, 1000-10000, 1000-5000, 1000-2500, 2500-10000, 2500-5000, or 5000-10000 sequences, or any included sub-range; for example, about 1, 2, 3, 4, 5, 6, 7, 8, 9, 10, 15, 20, 25, 30, 35, 40, 45, 50, 60, 70, 80, 90, 100, 150, 200, 250, 300, 350, 400, 450, 500, 600, 700, 800, 900, 1000, 1250, 1500, 1750, 2000, 2500, 3000, 3500, 4000, 4500, 5000, 6000, 7000, 8000, 9000, 10000, or more sequences, or any intervening integer.

A "variant" can be any change in an individual nucleotide sequence compared to a reference sequence. The reference sequence can be a single sequence, a cohort of reference sequences, or a consensus sequence derived from a cohort of reference sequences. An individual variant can be a coding variant or a non-coding variant. A variant wherein a single nucleotide within the individual sequence is changed in comparison to the reference sequence can be referred to as a single nucleotide polymorphism (SNP) or a single nucleotide variant (SNV) and these terms are used interchangeably herein. SNPs that occur in the protein coding regions of genes that give rise to the expression of variant or defective proteins are potentially the cause of a genetic-based disease. Even SNPs that occur in non-coding regions can result in altered mRNA and/or protein expression. Examples are SNPs that defective splicing at exon/intron junctions. Exons are the regions in genes that contain three-nucleotide codons that are ultimately translated into the amino acids that form proteins. Introns are regions in genes that can be transcribed into pre-messenger RNA but do not code for amino acids. In the process by which genomic DNA is transcribed into messenger RNA, introns are often spliced out of pre-messenger RNA transcripts to yield messenger RNA. A SNP can be in a coding region or a non-coding region. A SNP in a coding region can be a silent mutation, otherwise known as a synonymous mutation, wherein an encoded amino acid is not changed due to the variant. An SNP in a coding region can be a missense mutation, wherein an encoded amino acid is changed due to the variant. An SNP in a coding region can also be a nonsense mutation, wherein the variant introduces a premature stop codon. A variant can include an insertion or deletion (INDEL) of one or more nucleotides. An INDEL can be a frame-shift mutation, which can significantly alter a gene product. An INDEL can be a splice-site mutation. A variant can be a large-scale mutation in a chromosome structure; for example, a copy-number variant caused by an amplification or duplication of one or more genes or chromosome regions or a deletion of one or more genes or chromosomal regions; or a translocation causing the interchange of genetic parts from non-homologous chromosomes, an interstitial deletion, or an inversion.

Variants can be provided in a variant file, for example, a genome variant file (GVF) or a variant call format (VCF) file. According to the methods disclosed herein, tools can be provided to convert a variant file provided in one format to another more preferred format. A variant file can comprise frequency information on the included variants.

A "feature" can be any span or a collection of spans within a nucleotide sequence (*e.g.*, a genome or transcriptome sequence). A feature can comprise a genome or genome fragment, one or more chromosomes or chromosome fragments, one or more genes or gene fragments, one or more transcripts or transcript fragments, one or more exons or exon fragments, one or more introns or intron fragments, one or more splice sites, one or more regulatory elements (*e.g.*, a promoter, an enhancer, a repressor, *etc.*) one or more plasmids or plasmid fragments, one or more artificial chromosomes or fragments, or a combination thereof. A feature can be automatically selected. A feature can be user-selectable.

A "disease gene model" can refer to the mode of inheritance for a phenotype. A single gene disorder can be autosomal dominant, autosomal recessive, X-linked dominant, X-linked recessive, Y-linked, or mitochondrial. Diseases can also be multifactorial and/or polygenic or complex, involving more than one variant or damaged gene.

"Pedigree" can refer to lineage or genealogical descent of an individual. Pedigree information can include polynucleotide sequence data from a known relative of an individual such as a child, a sibling, a parent, an aunt or uncle, a grandparent, *etc.*

"Amino acid" or "peptide" refers to one of the twenty biologically occurring amino acids and to synthetic amino acids, including D/L optical isomers. Amino acids can be classified based upon the properties of their side chains as weakly acidic, weakly basic, hydrophilic, or hydrophobic. A "polypeptide" refers to a molecule formed by a sequence of two or more amino acids. Proteins are linear polypeptide chains composed of amino acid building blocks. The linear polypeptide sequence provides only a small part of the structural information that is important to the biochemist, however. The polypeptide chain folds to give secondary structural units (most commonly alpha helices and beta strands). Secondary structural units can then fold to give supersecondary structures (for example, beta sheets) and a tertiary structure. Most of the behaviors of a protein are determined by its secondary and tertiary structure, including those that are important for allowing the protein to function in a living system.

Disclosed herein is a newly developed analysis method that can analyze personal genome sequence data. The input of the method can be a genome file. The genome file can comprise genome sequence files, partial genome sequence files, genome variant files (*e.g.,* VCF files, GVF files, *etc.*)*,* partial genome variant files, genotyping array files, or any other DNA variant files. The genome variant files can contain the variants or difference of an individual genome or a set of genomes compared to a reference genome (*e.g.*, human reference assembly). These variant files can include variants such as single nucleotide variants (SNVs), single nucleotide polymorphisms (SNPs), small and larger insertion and deletions (INDELS), rearrangements, CNV (copy number variants), Structural Variants (SVs), *etc.* The variant file can include frequency information for each variant.

The methods disclosed herein can be used to identify, rank, and score variants by relevance either individually or in sets lying within a feature. A feature can be any span or a collection of spans on the genome sequence or transcriptome sequences such as a gene, transcript, exon, intron, UTRs, genetic locus or extended gene region including regulatory elements. A feature can also be a list of 2 or more genes, a genetic pathway or an ontology category.

The methods disclosed herein can be implemented as computer executable instructions or tools. In one embodiment, VAAST is such a tool. VAAST can provide a simple means to accomplish these methods, allowing users to, for example:

1) Combine and compare whole genome variant data for diverse downstream analyses;
(2) Identify hotspots of variation within a genome and its annotations, e.g. genes, regulatory elements, etc; and
(3) Perform ontology-driven analyses in order to investigate variation within a given Gene Ontology (GO) category, disease class or metabolic pathway.

In one embodiment, VAAST comprises an FPT (Feature Prioritization Tool) .The FPT can be a general method to score variants in personal genomes for importance. In some embodiments, VAAST:

1. Prioritizes features and/or variants
2. Can prioritize coding, non-coding and features composed of both, *e.g.,* genes
3. Assign VAAST score to variants and/or features
4. Can leverage pedigree information
5. Can train itself on the fly
6. Can use PG seqs, exomes, chip data, and datasets that are mixtures of all three

In the context of human genome sequences, it can be applied as:

1. a general probabilistic 'disease-gene' finder: Identify disease-causing genes & their variants in genomes and sets of genomes;
2. a search tool to rapidly search personal genome sequences for genes having significant differences in variant frequencies vs. controls;
3. a means to determine the statistical significance of 'hits';
4. a means to automatically relate hits to genetic pathways, network and ontologies.

These analyses can be carried out on sets of genomes, making possible both pairwise (single against single genome, single against set of background genomes) and case-control style studies (set(s) of target genomes against set of background genomes) of personal genome sequences. We present here several analyses of healthy and cancer genomes and show how VAAST can be used to identify variation hotspots both along the chromosome, and within gene ontologies, disease classes and metabolic pathways. Special emphasis is placed upon the impact of data quality and ethnicity, and their consequences for further downstream analyses. We also show how variant calling procedures, pseudogenes and gene families all combine to complicate clinically-orientated analyses of personal genome sequences in ways that only become apparent when cohorts of genomes are analyzed.

The FPT search method in VAAST can be applied to identify disease genes in novel genome sequences such as a whole human genome variant file. It can automatically prioritize features such as genes in a genome sequence based on the relevance of variants within the gene. A typical example is an individual with a rare genetic disease for which a researcher or doctor seeks to identify the causative variant(s)/mutation(s). The recently published Miller Syndrome analysis given an exome sequence provides a test case for VAAST: Nat Genet. 2010 Jan;42(1):30-5. Epub 2009 Nov 13; and whole genome sequencing: Nat Methods. 2010 May;7(5):350.). Given a variant file of 4 million single nucleotide variants (SNVs) the two disease causing genes where ranked 3rd and 11th given only the two affected personal genomes, and using dbSNP and data from the 1000 Genomes project as the set of background genomes. When pedigree information is included - in our example both parents - VAAST FPT assigns a statistically significant score to the two disease genes in question, and they are ranked on top (see Example 7).

The FPT search method in VAAST can be applied to case/control like studies (such as GWAS-like genome sequence studies). It can automatically rank which features/genes are most likely be disease relevant in the patient genomes versus the healthy genomes.

The FPT search method in VAAST can be applied to pairwise studies such as cancer analyses, where studies have sets of pairs of tumor and germline genomes from patients (often from skin). The task is to identify features that are relevant in tumor development (often somatic mutations) in the target (tumor) genomes versus background (germline) genomes.

The FPT search method in VAAST can be applied to progression studies in genomes such as different stages in tumor development (solid tumor, metastasis, recurrence, etc.)

The FPT search method in VAAST can be applied to genomes to remove noise of systematic errors in sequencing technologies. If a sequencing technology has systematic sequencing difficulties for a specific genome position or area, analyses of sets of genomes from the same technology can automatically filter out these errors during the VAAST analysis.

The FPT search method in VAAST can be applied to identify sites of purifying selection or rapid evolution in novel and agricultural genomes.

In addition to the likelihood ratio test as a scoring mode, VAAST can incorporate several alternative feature-ranking algorithms; for example, the Weighted Sum Statistic (wss) method (Madsen and Browning, PLoS Genet. 2009 February; 5(2): e1000384) for ranking features.

**Nucleotide Sequencing, Alignment, and Variant Identification**

We disclose methods of identifying and/or prioritizing phenotype causing variants utilizing nucleotide sequencing data. The methods can comprise comparing case and background sequencing information. Nucleotide sequencing information can be obtained using any known or future methodology or technology platform; for example, Sanger sequencing, dye-terminator sequencing, Massively Parallel Signature Sequencing (MPSS), Polony sequencing, 454 pyrosequencing, Illumina sequencing, SOLiD sequencing, ion semiconductor sequencing, DNA nanoball sequencing, sequencing by hybridization, or any combination thereof. Sequences from multiple different sequencing platforms can be used in the comparison. Non-limiting examples of types of sequence information that can be utilized in the methods disclosed herein are whole genome sequencing (WGS), exome sequencing, and exon-capture sequencing. The sequencing can be performed on paired-end sequencing libraries.

Sequencing data can be aligned to any known or future reference sequence. For example, if the sequencing data is from a human, the sequencing data can be aligned to a human genome sequence (*e.g.,* any current or future human sequence, *e.g.,* hg19 (GRCh37), hg18, hg17, hg16, hg15, hg13, hg12, hg11, hg8, hg7, hg6, hg5, hg4, *etc.*). (See hgdownload.cse.ucsc.edu/downloads.html). In one embodiment, the reference sequence is provided in a Fasta file. Fasta files can be used for providing a copy of the reference genome sequence. Each sequence (*e.g.*, chromosome or a contig) can begin with a header line, which can begin with the '>' character. The first contiguous set of non-whitespace characters after the '>' can be used as the ID of that sequence. In one embodiment, this ID must match the 'seqid' column described *supra* for the sequence feature and sequence variants. On the next and subsequent lines the sequence can be represented with the characters A, C, G, T, and N. In one embodiment, all other characters are disallowed. The sequence lines can be of any length. In one embodiment, all the lines must be the same length, except the final line of each sequence, which can terminate whenever necessary at the end of the sequence.

The GFF3 file format can be used to annotate genomic features in the reference sequence. The GFF3 (General Feature Format version 3) file format is a widely used format for annotating genomic features. Although various versions of GTF and GFF formats have been in use for many years, GFF3 was introduced by Lincoln Stein to standardize the various gene annotation formats to allow better interoperability between genome projects. The Sequence Ontology group currently maintains the GFF3 specification. (See www.sequenceontology.org/resources/gff3.html). Briefly, a GFF3 file can begin with one or more lines of pragma or meta-data information on lines that begin with '##'. In one embodiment, a required pragma is '## gff-version 3'. Header lines can be followed by one or more (usually many more) feature lines. In one embodiment, each feature line describes a single genomic feature. Each feature line can consist of nine tab-delimited columns. Each of the first eight columns can describe details about the feature and its location on the genome and the final line can be a set of tag value pairs that describe attributes of the feature.

A number of programs have been developed to perform sequence alignments and the choice of which particular program to use can depend upon the type of sequencing data and/or the type of alignment required; for example, programs have been developed to perform a database search, conduct a pairwise alignment, perform a multiple sequence alignment, perform a genomics analysis, find a motif, perform benchmarking, and conduct a short sequence alignment. Examples of programs that can be used to perform a database search include BLAST, FASTA, HMMER, IDF, Infernal, Sequilab, SAM, and SSEARCH. Examples of programs that can be used to perform a pairwise alignment include ACANA, Bioconductor Biostrings::pairwiseAlignment, BioPerl dpAlign, BLASTZ, LASTZ, DNADot, DOTLET, FEAST, JAligner, LALIGN, mAlign, matcher, MCALIGN2, MUMmer, needle, Ngila, PatternHunter, ProbA (also propA), REPuter, Satsuma, SEQALN, SIM, GAP, NAP, LAP, SIM, SPA: Super pairwise alignment, Sequences Studio, SWIFT suit, stretcher, tranalign, UGENE, water, wordmatch, and YASS. Examples of programs that can be used to perform a multiple sequence alignment include ALE, AMAP, anon., BAli-Phy, CHAOS/DIALIGN, ClustalW, CodonCode Aligner, DIALIGN-TX and DIALIGN-T, DNA Alignment, FSA, Geneious, Kalign, MAFFT, MARNA, MAVID, MSA, MULTALIN, Multi-LAGAN, MUSCLE, Opal, Pecan, Phylo, PSAlign, RevTrans, Se-Al, StatAlign, Stemloc, T-Coffee, and UGENE. Examples of programs that can be used for genomics analysis include ACT (Artemis Comparison Tool), AVID, BLAT, GMAP, Mauve, MGA, Mulan, Multiz, PLAST-ncRNA, Sequerome, Sequilab, Shuffle-LAGAN, SIBsim4 / Sim4, and SLAM. Examples of programs that can be used for finding motifs include BLOCKS, eMOTIF, Gibbs motif sampler, HMMTOP, I-sites, MEME/MAST, MERCI, PHI-Blast, Phyloscan, and TEIRESIAS. Examples of programs that can be used for benchmarking include BAliBASE, HOMSTRAD, Oxbench, PFAM, PREFAB, SABmark, and SMART. Examples of software that can be used to perform a short sequence alignment include BFAST, BLASTN, BLAT, Bowtie, BWA, CASHX, CUDA-EC, drFAST, ELAND, GNUMAP, GEM, GMAP and GSNAP, Geneious Assembler, LAST, MAQ, mrFAST and mrsFAST, MOM, MOSAIK, MPscan, Novoalign, NextGENe, PALMapper, PerM, QPalma, RazerS, RMAP, rNA, RTG Investigator, Segemehl, SeqMap, Shrec, SHRiMP, SLIDER, SOAP, SOCS, SSAHA and SSAHA2, Stampy, SToRM, Taipan, UGENE, XpressAlign, and ZOOM. In one embodiment, sequence data is aligned to a reference sequence using Burroughs Wheeler alignment (BWA). Sequence alignment data can be stored in a SAM file. SAM (Sequence Alignment/Map) is a flexible generic format for storing nucleotide sequence alignment (see samtools.sourceforge.net/SAM1.pdf). Sequence alignment data can be stored in a BAM file, which is a compressed binary version of the SAM format (see genome.ucsc.edu/FAQ/FAQformat.html#format5.1). In one embodiment, sequence alignment data in SAM format is converted to BAM format.

Variants can be identified in sequencing data that has been aligned to a reference sequence using any known methodology. A variant can be a coding variant or a non-coding variant. A variant can be a single nucleotide polymorphism (SNP), also called a single nucleotide variant (SNV). Examples of SNPs in a coding region are silent mutations, otherwise known as a synonymous mutation; missense mutations, and nonsense mutations. A SNP in a non-coding region can alter a splice-site. A SNP in a non-coding region can alter a regulator sequence (*e.g.*, a promoter sequence, an enhancer seqeunce, an inhibiter sequence, *etc.*). A variant can include an insertion or deletion (INDEL) of one or more nucleotides. Examples of INDELs include frame-shift mutations and splice-site mutations. A variant can be a large-scale mutation in a chromosome structure; for example, a copy-number variant caused by an amplification or duplication of one or more genes or chromosome regions or a deletion of one or more genes or chromosomal regions; or a translocation causing the interchange of genetic parts from non-homologous chromosomes, an interstitial deletion, or an inversion.

Variants can be identified using SamTools, which provides various utilities for manipulating alignments in the SAM format, including sorting, merging, indexing and generating alignments in a per-position format (see samtools.sourceforge.net). In one embodiment, variants are called using the mpileup command in SamTools. Variants can be identified using the Genome Analysis Toolkit (GATK) (see www.broadinstitute.org/gsa/wiki/index.php/The_Genome_Analysis_Toolkit). In one embodiment, regions surrounding potential indels can be realigned using the GATK IndelRealigner tool. In one embodiment, variants are called using the GATK UnifiedGenotypeCaller and IndelCaller. Variants can be identified using the Genomic Next-generation Universal MAPer (GNUMAP) program (see dna.cs.byu.edu/gnumap/). In one embodiment, GNUMAP is used to align and/or identify variants in next generation sequencing data.

**Variant Files**

We disclose methods of identifying and/or prioritizing phenotype causing variants, wherein the variants are provided in one or more variant files. The methods can comprise comparing a target cohort of variants to a background cohort of variants. The variants can be provided in one or more variant files. Non-limiting examples of variant file formats are genome variant file (GVF) format and variant call format (VCF). The GVF file format was introduced by the Sequence Ontology group for use in describing sequence variants. It is based on the GFF3 format and is fully compatible with GFF3 and tools built for parsing, analyzing and viewing GFF3. (See www.sequenceontology.org/gvf.html). GVF shares the same nine-column format for feature lines, but specifies additional pragmas for use at the top of the file and additional tag/value pairs to describe feature attributes in column nine that are specific to variant features (*e.g.*, variant effects). According to the methods disclosed herein, tools can be provided to convert a variant file provided in one format to another format. In one embodiment, variant files in VCF format are converted to GVF format using a tool called vaast_converter. In one embodiment, variant effect information is added to a GVF format file using a variant annotation tool (VAT). A variant file can comprise frequency information on the included variants.

**Target and Background Cohorts**

We disclose methods of identifying and/or prioritizing phenotype causing variants by comparing a target cohort of variants to a background cohort of variants. A cohort is defined as a grouping of one or more individuals. A cohort can contain any number of individuals; for example, about 1-10000, 1-5000, 1-2500, 1-1000, 1-500, 1-100, 1-50, 1-10, 10-10000, 10-5000, 10-2500, 10-1000, 10-500, 10-100, 10-50, 50-10000, 50-5000, 50-2500, 50-1000, 50-500, 50-100, 100-10000, 100-5000, 100-2500, 100-1000, 100-500, 500-10000, 500-5000, 500-2500, 500-1000, 1000-10000, 1000-5000, 1000-2500, 2500-10000, 2500-5000, or 5000-10000 individuals, or any included sub-range. A cohort can contain about 1, 2, 3, 4, 5, 6, 7, 8, 9, 10, 15, 20, 25, 30, 35, 40, 45, 50, 60, 70, 80, 90, 100, 150, 200, 250, 300, 350, 400, 450, 500, 600, 700, 800, 900, 1000, 1250, 1500, 1750, 2000, 2500, 3000, 3500, 4000, 4500, 5000, 6000, 7000, 8000, 9000, 10000, or more individuals, or any intervening integer. The target cohort can contain information from the individual(s) under study (e.g., individuals that exhibit the phenotype of interest). The background cohort contains information from the individual(s) serving as healthy controls.

Selection of variants within a cohort.

The target and/or background cohorts can contain a variant file corresponding to each of the individuals within the cohort. The variant file(s) can be derived from individual sequencing data aligned to a reference sequence. The variant files can be in any format; non limiting examples including the VCF and GVF formats. In one embodiment, a set of variants from the individual variant files in a target or background cohort are combined into a single, condensed variant file. A number of options for producing a set of variants in a condensed variant file can be used. The condensed variant file can contain the union of all of the individual variant files in a cohort, wherein the set of variant in the condensed variant file contains all the variants found in the individual files. The condensed variant file can contain the intersection of all individual variant files in a cohort, wherein set of variants in the condensed variant file contains only those variants that are common to all of the individual variant files. The condensed variant file can contain the compliment of the individual variant files, wherein set of variants in the condensed variant file contains the variants that are unique to a specified individual variant file within the cohort of individual variant files. The condensed variant file can contain the difference of the individual variant files, wherein the set of variants in the condensed variant file contains all of the variants that unique to any of the individual variant files. The condensed variant file can contain the variants that are shared between a specified number of individual files. For example, if the specified number is 2, then the set of variants in the condensed variant file would contain only those variants that are found in at least two individual variant files. The specified number of variant files can be between 2 and N, wherein N is the number of individual variant files in a cohort. In one embodiment, a subset of the individual variant files can be specified and combined into a condensed variant file using any of these described methods. More than one method of combining individual variant files can be used to produce a combined variant file. For example, a combined variant file can be produced that contains the set of variants found in one group of the cohort but not another group of the cohort. In one embodiment, a software tool is provided to combine variant files into a condensed variant file. In one embodiment, the software tool is the Variant Selection Tool (VST).

CDR files

In one embodiment, the condensed variant file is a CDR file. In one embodiment, the CDR file contains two types of lines: locus lines and meta-data lines. The locus lines can define the variants selected for analysis. A second type of line can contain meta-data that applies to the entire CDR file.

In one embodiment, the locus lines can contain the following columns:

1. seqid of the reference sequence used to identify the variants (*e.g.*, the chromosome);
2. start position in the reference sequence of the variant;
3. end position in the reference sequence of the variant;
4. variant type (*e.g.,* SNV, insert, deletion, inversion, *etc.*);
5. variant effect(s) (*e.g.,* a Sequence Ontology term, *e.g.,* synonymous, non-synonymous, *etc.*);
6. reference sequence between the start and end position; wherein the reference nucleotide(s) can be indicated and, if applicable, the reference amino acid(s) are also given, separated by a vertical bar '|'; if multiple reference amino acids are possible due to multiple transcripts translated in different frames, then only the most common (random if a tie) amino acid(s) are given; and

7+. column 7 and onward can be used to show all the genotypes that were selected from the cohort with a separate column for each genotype; the data in each of these columns can separated by vertical bars '|' into the following values:

a. a comma separated list of index numbers (or ranges, 5-8 represents 5,6,7,8) that represent the individual(s) in the cohort that have the given genotype;

b. the variant genotype (*e.g.,* A:C); and

c. (optional) if the given variant falls within a coding region, the variant amino acids corresponding to the genotype are given in the same order (*e.g.,* C:R).

In one embodiment, several lines of meta-data are given that apply to the entire file following the locus line(s). Each of these meta-data lines can begin with a double-hash '##'. The data following the double-hash can be tab separated. The following are exemplary meta-data lines that can be included in a CDR file with explanations below in italics:

1. ## W0C 6.25701499933934e-05
*Can be used to indicate an observed amino acid substitution frequency in the set; for example, W to O.*

   *a. The first column has two amino acids separated by a zero.*
   *b. The second column has the frequency that particular substitution occurs within the set, calculated as number of times that substitution is seen divided by the cumulative length of all nucleotide sequences in the set.*

2. ## PSUEDO-COUNT 3.2303107427267e-11
*The pseudo count can be used as a proxy for the frequency of any amino acid substitution not seen in a particular set and is 10X less than the minimum amino acid substitution frequency seen within the cohort.*
3 . ## GENOME-LENGTH 3095677412
*The length of the reference nucleotide sequence.*
4 . ## GENOME-COUNT 5
*The number of nucleotide sequences in the set.*
5. ## CUMULATIVE---GENOME---length 15478387060
*The cumulative length of all nucleotide sequences in the set.*
6. ## COMMAND-LINE /home/bmoore/VAAST_RC_1.0/bin/vaast_tools/vst -o U(0..4) file0.vat.gvf file 1.vat.gvf file2.vat.gvf file3.vat.gvf file4.vat.gvf
*The command line used.*
7. ## PROGRAM-VERSION RC1.0
*The program version*
8 . ## GENDER F:0-2 M:3-4
*The gender of the individuals in the file (0-based index to the files on the command line.*
9 . ## FILE-INDEX 0 file0.vat.gvf
*The 0-based index with the corresponding files given on the command line.*

An exemplary CDR file is shown below (some information is truncated (...) for display purposes).

```
chr1 879304 879304 SNV non_sy…_codon G|G 0,4-5|A:A|D:D
1|A:G|D:G chr1 879317 879317 SNV synonymous_codon C|Y 2|C:T|Y:Y
. . .
##      A0E   4.7243294612378e-06
##      COM   9.69093222818011e-10
##      R0Q   3.40591043470133e-05
. . .
##      PSUEDO-COUNT  3.2303107427267e-11
##        GENOME-LENGTH 3095677412
##      GENOME-COUNT   453
##      CUMULATIVE-GENOME-length      1402341867636
##        COMMAND-LINE file0.vat.gvf file1.vat.gvf file2.vat.gvf É
##        PROGRAM-VERSION rc_1.0
##        GENDER F:0-2 M:3-4
##      FILE-INDEX     0   file0.vat
##      FILE-INDEX     1   file1.vat
##      FILE-INDEX     2   file2.vat
##      FILE-INDEX     3   file3.vat
##      FILE-INDEX     4   file4.vat
##      FILE-INDEX     5   file5.vat
```

## Composite Likelihood Ratio Test With Optional Grouping

In one aspect, disclosed herein are methods of identifying and/or prioritizing phenotype causing variants that comprise calculating a composite likelihood ratio (CLR). The methods can comprise comparing a target cohort of variants to a background cohort of variants. The composite likelihood ratio test (CLRT) can evaluate whether a variant contributes to a phenotype. The first step can be to calculate the likelihood of the null and alternative models assuming independence between nucleotide sites and then to evaluate the significance of the likelihood ratio by permutation to control for linkage disequilibrium (LD). In one embodiment, the CLRT is a nested CLRT that depends only on differences in variant frequencies between target and background individuals. In some cases, the phenotype under investigation can be caused by many relatively rare but distinct variants within the same feature (*e.g.* gene). Each variant alone may have been rare enough to escape detection by the CRLT. Grouping the rare variants in a feature prior to the raw score calculation can enable the detection of features that are linked to the detected phenotype. Variants that are detected within the target cohort at a frequency less than a collapsing threshold can be collapsed, or grouped, into one or more categories. The total number of minor variant copies among all target and background individuals can be counted rather than just the presence or absence of minor variants within an individual. The collapsing, or grouping, threshold can be set to any number between 0 and N, wherein N is the number of individuals within the target cohort. Let *k* equal the number of uncollapsed variant sites among $n_i^U$ unaffected and $n_i^A$ affected individuals, with $n_i$ equal to $n_i^U+n_i^A$. Let $l_{k+1}..l_{k+m}$ equal the number of collapsed variant sites within *m* collapsing categories labeled *k*+1 to *m,* and let $l_1..l_k$ equal 1. Let $X_i$, $X_i^U$ and $X_i^A$ equal the number of copies of the minor allele(s) at variant site *i* or collapsing category *i* among all individuals, unaffected individuals, and affected individuals, respectively. Then the log-likelihood ratio is equal to:

$$\text{Equation 1.}\ \lambda = \ln\left(\frac{L_{Null}}{L_{Alt}}\right) = \sum_{i=1}^{k+m} \ln\left[\frac{\left(\hat{p}_i\right)^{X_i}\left(1-\hat{p}_i\right)^{2l_i n_i - X_i}}{\left(\hat{p}_i^U\right)^{X_i^U}\left(1-\hat{p}_i^U\right)^{2l_i n_i^U - X_i^U}\left(\hat{p}_i^A\right)^{X_i^A}\left(1-\hat{p}_i^A\right)^{2l_i n_i^A - X_i^A}}\right]$$

where $p_i$, $p_i^U$, and $p_i^A$ equal the maximum likelihood estimates for the frequency of minor allele(s) at variant site $i$ or collapsing category $i$ among all individuals, unaffected individuals, and affected individuals, respectively. When no constraints are placed on the frequency of phenotype-causing variants (*e.g.*, collapsing threshold = 0), the maximum likelihood estimates can be equal to the observed frequencies of the minor allele(s). Assuming that variant sites are unlinked, $-2\lambda$ can approximately follow a $\chi^2$ distribution with $k+m$ degrees of freedom. The $\log_e$ of the $\chi^2$ p-value can be reported to provide a statistic for rapid prioritization of phenotype-causing candidates. To evaluate the statistical significance of a genomic feature (*e.g.*, gene), a randomization test can be performed by permuting the affected/unaffected status of each individual (or each individual chromosome, when phased data are available). Because the degrees of freedom can vary between iterations of the permutation test, the $\chi^2$ p-value can be used as the test statistic for the randomization test.

**Amino Acid Substitution/Codon Bias**

We disclose methods of identifying and/or prioritizing phenotype causing variants by comparing a target cohort to a background cohort, wherein the comparing can incorporate Amino Acid Substitution (AAS) information. The AAS information can be obtained from one or more existing databases or substitution matrixes. The choice of a source for AAS information can depend upon the species of the target and background cohorts. The AAS information can be obtained from the Online Mendelian Inheritance in Man (OMIM) database. The AAS information can be obtained from the Online Mendelian Inheritance in Animals database (OMIA). The AAS information can be obtained from the Mouse Locus Catalogue (MLC). The AAS information can be obtained from a BLOSUM (Blocks of Amino Acid Substitution Matrix) substitution matrix. The AAS information can be obtained from a Point Accepted Mutation (PAM) substitution matrix.

Online Mendelian Inheritance in Man (OMIM)

Online Mendelian Inheritance in Man (OMIM) is a database that catalogues all the known diseases with a genetic component, and-when possible-links them to the relevant genes in the human genome and provides references for further research and tools for genomic analysis of a catalogued gene (see www.omim.org). OMIM is one of the databases housed in the U.S. National Center for Biotechnology Information (NCBI) and included in its search menus. Every disease and gene is assigned a six digit number of which the first number classifies the method of inheritance. If the initial digit is 1, the trait is deemed autosomal dominant; if 2, autosomal recessive; if 3, X-linked. Wherever a trait defined in this dictionary has a MIM number, the number from the 12th edition of MIM, is given in square brackets with or without an asterisk (asterisks indicate that the mode of inheritance is known; a number symbol (#) before an entry number means that the phenotype can be caused by mutation in any of two or more genes) as appropriate (*e.g.*, Pelizaeus-Merzbacher disease [MIM #312080] is an X-linked recessive disorder).

Range of MIM codes for method of inheritance: 100000-299999: autosomal loci or phenotypes (created before May 15, 1994); 300000-399999: X-linked loci or phenotypes; 400000-499999: Y-linked loci or phenotypes; 500000-599999: Mitochondrial loci or phenotypes; 600000-above : Autosomal loci or phenotypes (created after May 15, 1994). Allelic variants (mutations) are designated by the MIM number of the entry, followed by a decimal point and a unique 4-digit variant number. For example, allelic variants in the factor IX gene (300746) are numbered 300746.0001 through 300746.0101.

In one embodiment, the method of identifying and/or prioritizing phenotype causing variants by comparing a target cohort to a background cohort incorporates Amino Acid Substitution (AAS) information from the OMIM database.

BLOSUM Substitution Matrixes

BLOSUM substitution matrixes can be used to align protein sequences. They are based on local alignments. A BLOSUM substitution matrix contains the calculated log-odds (LOD) score for each of the 210 possible substitutions of the 20 standard amino acids. All BLOSUM matrices are based on observed alignments. Several sets of BLOSUM matrices exist using different alignment databases, named with numbers. BLOSUM matrices with high numbers are designed for comparing closely related sequences, while those with low numbers are designed for comparing distant related sequences. For example, BLOSUM80 is used for less divergent alignments, and BLOSUM45 is used for more divergent alignments. The matrices were created by merging (clustering) all sequences that were more similar than a given percentage into one single sequence and then comparing those sequences (that were all more divergent than the given percentage value) only; thus reducing the contribution of closely related sequences. The percentage used was appended to the name, giving BLOSUM80 for example where sequences that were more than 80% identical were clustered.

Scores within a BLOSUM are log-odds scores that measure, in an alignment, the logarithm for the ratio of the likelihood of two amino acids appearing with a biological sense and the likelihood of the same amino acids appearing by chance. The matrices are based on the minimum percentage identity of the aligned protein sequence used in calculating them. Every possible identity or substitution is assigned a score based on its observed frequencies in the alignment of related proteins. A positive score is given to the more likely substitutions while a negative score is given to the less likely substitutions.

To calculate a BLOSUM matrix, the following equation is used: $S_{ij} = (1/\lambda) \log [p_{ij}/(q_i * q_j)]$, where $p_{ij}$ is the probability of two amino acids i and j replacing each other in a homologous sequence, and $q_i$ and $q_j$ are the background probabilities of finding the amino acids i and j in any protein sequence at random. The factor $\lambda$ is a scaling factor, set such that the matrix contains easily computable integer values.

In one embodiment, the method of identifying and/or prioritizing phenotype causing variants by comparing a target cohort to a background cohort incorporates Amino Acid Substitution (AAS) information from a BLOSUM substitution matrix. The BLOSUM substitution matrix can be any BLOSUM substitution matrix; for example, BLOSUM30, BLOSUM31, BLOSUM32, BLOSUM33, BLOSUM34, BLOSUM35, BLOSUM36, BLOSUM37, BLOSUM38, BLOSUM39, BLOSUM40, BLOSUM41, BLOSUM42, BLOSUM43, BLOSUM44, BLOSUM45, BLOSUM46, BLOSUM47, BLOSUM48, BLOSUM49, BLOSUM50, BLOSUM51, BLOSUM52, BLOSUM53, BLOSUM54, BLOSUM55, BLOSUM56, BLOSUM57, BLOSUM58, BLOSUM59, BLOSUM60, BLOSUM61, BLOSUM62, BLOSUM63, BLOSUM64, BLOSUM65, BLOSUM66, BLOSUM67, BLOSUM68, BLOSUM69, BLOSUM70, BLOSUM71, BLOSUM72, BLOSUM73, BLOSUM74, BLOSUM75, BLOSUM76, BLOSUM77, BLOSUM78, BLOSUM79, BLOSUM80, BLOSUM81, BLOSUM82, BLOSUM83, BLOSUM84, BLOSUM85, BLOSUM86, BLOSUM87, BLOSUM88, BLOSUM89, BLOSUM90, BLOSUM91, BLOSUM92, BLOSUM93, BLOSUM94, BLOSUM95, BLOSUM96, BLOSUM97, BLOSUM98, BLOSUM99, or BLOSUM100. In one embodiment, the BLOSUM substitution matrix is BLOSUM 62.

To incorporate information about the potential consequences of amino acid changes, an additional parameter can be added in the null and alternative model for each variant site or collapsing category. The parameter $h_i$ in the null model is the likelihood that the amino acid change does not contribute to a phenotype. The parameter $h_i$ can be estimated by setting it equal to the proportion of this type of amino acid change in the population background. The parameter $a_i$ in the alternative model is the likelihood that the amino acid change contributes to the phenotype. The parameter $a_i$ can be estimated, for example, by setting it equal to the proportion of this type of amino acid change among all disease-causing mutations in OMIM. Incorporating information about amino acid severity, $\lambda$ is equal to:

$$\lambda = \ln\left(\frac{L_{Null}}{L_{Alt}}\right) = \sum_{i=1}^{k+m} \ln\left[\frac{h_i(\hat{p}_i)^{X_i}(1-\hat{p}_i)^{2l_i n_i - X_i}}{a_i(\hat{p}_i^U)^{X_i^U}(1-\hat{p}_i^U)^{2l_i n_i^U - X_i^U}(\hat{p}_i^A)^{X_i^A}(1-\hat{p}_i^A)^{2l_i n_i^A - X_i^A}}\right].$$

Equation 2.

To include the potential consequences of amino acid changes for collapsed rare variants, *m* collapsing categories can be created that are divided according to the severity of potential amino acid changes. To create the collapsing categories, all possible amino acid changes can be first ranked according to their severity. An equal number of potential changes can then assigned to each category, with the first category receiving the least severe changes and each subsequent category receiving progressively more severe changes. Each rare variant is then included in the category with its corresponding amino acid change. For each collapsing category, *i,* the parameters $h_i$ and $a_i$ can be set equal to their average values among all variants present in the category. The likelihood of the null and alternative models can then first be calculated assuming independence between nucleotide sites and then evaluate the significance of the likelihood ratio by permutation to control for LD.

**Scoring Non-Coding variants.**

We disclose methods of identifying and/or prioritizing phenotype causing variants by comparing a target cohort to a background cohort, wherein the CLRT scores non-coding variants and synonymous variants within coding regions. Scoring non-coding variants can employ an evolutionary approach to estimate the relative impacts of non-coding and synonymous variants. The evolutionary approach can involve multiple-species alignments, wherein the species are chosen because they are evolutionarily related to the species of the target and background cohorts (reference genome). For example, a multiple species alignment can be a vertebrate-to-human genome multiple alignment. In one embodiment, the vertebrate-to-human genome multiple species alignment can be downloaded from the UCSC Genome Browser (hgdownload.cse.ucsc.edu/goldenPath/hg18/multiz44way/maf/). For each codon in the reference genome, an alignment frequency can be calculated. For every codon alignment pair involving one or fewer nucleotide changes, a Normalized Mutational Proportion (NMP) can be determined, which can be defined as the proportion of occurrences of each such codon pair among all codon pairs with the identical reference codon and with 1 or fewer nucleotide changes. In a non-limiting example, suppose the human codon 'GCC' aligned to codons in primate genomes with the following frequencies: GCC => GCC: 1000 times; GCC => GCT: 200 times; GCC => GCG: 250 times; GCC => GGG: 50 times. The NMP value of GCC=>GCT would be 0.134 (*i.e.,* 200/(1000 + 200 + 250)). For every codon pair that involves a non-synonymous change, a severity parameter can be calculated using AAS information ($a_i/h_i$ in Eq. 2). Linear regression analysis indicates that $log(a_i/h_i)$ is significantly correlated with *log*(NMP) ($R^2$ =0.23, $p$ <0.001). This model can allow the estimation of the severity parameter of synonymous variants (again by linear regression). A similar approach can be used to derive an equivalent value for SNVs in non-coding regions. To do so, the analysis can be restricted to clustered DNAse hypersensitive sites and transcription factor binding regions. In one embodiement, an NMP is calculated for every conserved trinucleotide. These regions can be defined by ENCODE regulation tracks.

**Variant Frequency Estimates**

We disclose methods of identifying and/or prioritizing phenotype causing variants by comparing a target cohort to a background cohort, wherein an estimate of the phenotype causing variant frequency within the background cohort is available. In one embodiment, an estimated variant frequency is used to constrain the CLRT such that the variant frequency in the alternative model cannot exceed the specified value. A CLRT comprising an estimated variant frequency can included AAS information.

**Variant Masking**

We disclose methods of identifying and/or prioritizing phenotype causing variants by comparing a target cohort to a background cohort, wherein variants within certain regions of the reference sequence are excluded or masked from a CLRT. Variant calling can be error-prone in repetitive and/or homologous sequences. Variant calling errors in repetitive or homologous sequences can be caused by short sequence reads aligning to multiple sites within the reference sequence. Variant masking can decrease the effect of sequencing platform bias **(Fig. 3B).** A CLRT with variant masking can be performed with AAS information and/or variant frequency estimation.

**Inheritance and Penetrance models (Disease Models)**

We disclose herein are methods of identifying and/or prioritizing phenotype causing variants by comparing a target cohort to a background cohort, wherein variants are filtered according to an inheritance and penetrance model prior to the CLRT. The inheritance and penetrance model can also be referred to as a disease model. The inheritance and penetrance model can be enforced within a feature (*e.g.,* gene, chromosome, *etc.*). The inheritance and penetrance model can introduce interdependence between variants within a feature, such as a gene or a chromosome. The inheritance and penetrance model can be a recessive inheritance model. The recessive inheritance model can be recessive, recessive with complete penetrance, or monogenic recessive. In the recessive model, the CLRT can be constrained such that no more than two minor variants in each feature of each individual in the target cohort will be scored. In one embodiment, the two variants within a feature that maximize the likelihood of the alternative model will be scored. In the recessive with complete penetrance model, it can be assumed that all individuals in the background cohort do not exhibit the phenotype under investigation. In one embodiment, if any individual in the background cohort has the same genotype within a feature as a person in the target cohort, that feature can be removed from the analysis under a recessive model with complete penetrance. In one embodiment, the monogenic recessive model, wherein genomic features are only scored if all individuals in the target cohort possess two or more minor alleles at sites where the alternative (phenotype) model is more likely than the null (healthy) model. Within the monogenic recessive model, two alleles can be present at different nucleotide sites in each affected individual (i.e., allelic heterogeneity is permitted), but locus heterogeneity can be excluded. The inheritance and penetrance model can be a dominant inheritance model. The dominant inheritance model can be a dominant model, a dominant with complete penetrance model, or a monogenic dominance model. The dominant model can comprise scoring only one minor variant in each feature of each individual in the target cohort. The variant scored can be the variant that maximizes the likelihood of the alternative (phenotype) model. The complete penetrance dominant model can comprise scoring variants only if they are absent among all individuals in the background cohort. The monogenic dominant model can comprise scoring genomic features only if all individuals in the target cohort posses at least one minor variant at features where the alternative model is more likely than the null model. In some embodiments, inheritance and penetrance models can be combined; for example, a monogenic recessive with complete penetrance or a monogenic dominant with complete penetrance.

**Pedigree/Quartet Analysis**

We disclose methods of identifying and/or prioritizing phenotype causing variants wherein pedigree information can be used. The pedigree information can be used to select variants for CLRT. The methods can comprise comparing a target cohort to a background cohort. In one embodiment, variants within a target cohort can be selected based upon the principles of Mendelian inheritance. In one embodiment, variants identified in an individual in the case cohort that are not found in the parents of the individual are eliminated. This can be used to remove sequencing errors. Exclusion of variants using pedigree information can also cause *de novo* mutations to be missed. Although this procedure can exclude both de novo mutations and sequencing errors, for genomes with an error rate of approximately 1 in 100,000, approximately 99.9% of all Mendelian inheritance errors are genotyping errors.

**Statistical Power**

We disclose methods of identifying phenotype-causing variants wherein genetic variants are prioritized by combining variant frequency, one or more sequence characteristics, and a summing procedure. The sequence characteristics can comprise AAS, splice site(s), promoter(s), binding sites (s), regulatory site(s) (*e.g.,* enhancer(s), represser(s), *etc.*). The summing procedure can comprise calculating a log-likelihood ratio. The methods disclosed herein can have greater statistical power than the statistical power of a method that does not combine variant frequency and one or more sequence characteristics (*e.g.*, AAS). For example, the methods disclosed herein can have 1, 2, 3, 4, 5, 6, 7, 8, 9, 10, 20, 30, 40, 50, 60, 70, 80, 90, 100, 200, 300, 400, 500, 600, 700, 800, 900, 1000, 2000, 3000, 4000, 5000, 6000, 7000, 8000, 9000, or 10000 times the statistical power of a method that does not combine variant frequency and one or more sequence characteristics. Statistical power can be measured by comparing the average rank of phenotype-causing genes; for example, in a benchmarking study such as the study presented in Example 8. Statistical power can be measured by comparing the standard deviation of the average ranking of phenotype-causing genes. Statistical power can be measured by comparing the spread of the average ranking of phenotype-causing genes, wherein the spread encompasses 95% of the runs.

**Examples**

Examples illustrating the utility of the VAAST tool were published in Yandell M, et al., Genome Res. 2011 Jul 7.

VAAST (the Variant Annotation, Analysis & Search Tool) is a probabilistic search tool that can be used to identify phenotype-causing genes in a personal genome sequence. VAAST builds upon existing amino acid substitution (AAS) and aggregative approaches to variant prioritization, combining elements of both into a single unified likelihood-framework that can allow users to identify damaged genes and deleterious variants with greater accuracy, and in an easy-to-use fashion. VAAST can score both coding and non-coding variants, evaluating the cumulative impact of both types of variants simultaneously. VAAST can identify rare variants causing rare genetic diseases, and it can also use both rare and common variants to identify genes responsible for common diseases. VAAST can have a much greater scope of use than any existing methodology. In these examples, the ability of VAAST to identify damaged genes using small cohorts (n=3) of unrelated individuals, wherein no two share the same deleterious variants; and for common, multigenic diseases using as few as 150 cases.

The past three decades have witnessed major advances in technologies for identifying disease-causing genes. As genome-wide panels of polymorphic marker loci were developed, linkage analysis of human pedigrees identified the locations of many Mendelian disease-causing genes. With the advent of SNP microarrays, the principle of linkage disequilibrium was used to identify hundreds of SNPs associated with susceptibility to common diseases. However, the causes of many genetic disorders remain unidentified because of a lack of multiplex families, and most of the heritability that underlies common, complex diseases remains unexplained.

Recent developments in whole-genome sequencing technology could overcome these problems. Whole-genome (or exome) sequence data have indeed yielded some successes, but these data present significant new analytic challenges as well. As the volume of genomic data grows, the goals of genome analysis itself are changing. Broadly speaking, discovery of sequence dissimilarity (in the form of sequence variants), rather than similarity has become the goal of most human genome analyses. In addition, the human genome is no longer a frontier; sequence variants must be evaluated in the context of pre-existing gene annotations. This is not merely a matter of annotating non-synonymous variants, nor is it a matter of predicting the severity of individual variants in isolation. Rather, the challenge is to determine their aggregative impact on a gene's function, a challenge unmet by existing tools for genome-wide association studies (GWAS) and linkage analysis.

Much work is currently being done in this area. Recently, several heuristic search tools have been published for personal genome data. Useful as these tools are, the need for users to specify search criteria places hard-to-quantify limitations on their performance. More broadly applicable probabilistic approaches are thus desirable. Indeed, the development of such methods is currently an active area of research. Several aggregative approaches such as CAST (Morgenthaler and Thilly, 2007, Mutat Res 615, 28-56), CMC (Li and Leal, 2008, Am J Hum Genet 83, 311-321), WSS (Madsen and Browning, 2009, PLoS Genet 5) and KBAC (Liu and Leal, 2010, PLoS Genet 6, e1001156.), have recently been published, and all demonstrate greater statistical power than existing GWAS approaches. But promising as these approaches are, to date they have remained largely theoretical. And understandably so: creating a tool that can employ these methods on the very large and complex datasets associated with personal genomes data is a separate software engineering challenge. Nevertheless it is a significant one. To be truly practical, a disease-gene finder must be able to rapidly and simultaneously search hundreds of genomes and their annotations.

Also missing from published aggregative approaches is a general implementation that can make use of Amino Acid Substitution (AAS) data. In one aspect, disclosed herein are methods of combining elements of AAS and aggregative approaches into a single, unified likelihood framework (*e.g.,* a disease-gene finder). This can result in greater statistical power and accuracy compared to either method alone. It can also significantly widen the scope of potential applications. The methods disclosed can assay the impact of rare variants to identify rare diseases, and it can use both common and rare variants to identify genes involved in common diseases.

A disease-gene and disease variant finder tool (*e.g.*, VAAST) can contain many other practical features. Since many disease-associated variants are located in non-coding regions, it can be useful for the tool to assess the cumulative impact of variants in both coding and non-coding regions of the genome. The tool can be capable of dealing with low-complexity and repetitive genome sequences. These regions can complicate searches of personal genomes for damaged genes, as they can result in false-positive predictions. The tool can be capable of employing pedigree and phased genome data, as these can provide additional sources of information. The tool can have the same general utility that has made genomic search tools such as BLAST, GENSCAN, and GENIE so successful (*e.g.*, it can be portable, trainable, and/or easy to use). In one embodiment, the tool can be an *ab initio* tool, requiring only very limited user-specified search criteria. Here we show that VAAST is such a tool.

We demonstrate VAAST's ability to identify both common and rare disease-causing variants using several recently published personal genome datasets, benchmarking its performance on more than 100 Mendelian conditions including congenital chloride diarrhea and Miller syndrome. We also show that VAAST can identify genes responsible for two common, complex diseases: Crohn disease and hypertriglyceridemia.

Collectively, our results demonstrate that VAAST provides a highly accurate, statistically robust means to rapidly search personal genome data for damaged genes and disease-causing variants in an easy-to-use fashion.

**Example 1: Methods**

**Inputs and outputs.**

The VAAST search procedure is shown in **Fig. 7.** VAAST operates using two input files: a background and a target file. The background and target files contain the variants observed in control and case genomes, respectively. The same background file can be used again and again, obviating the need-and expense-of producing a new set of control data for each analysis. Background files prepared from whole-genome data can be used for whole-genome analyses, exome analyses and/or for individual gene analyses. These files can be in either VCF (www.1000genomes.org/wiki/Analysis/vcf4.0) or GVF (Reese et al., 2010, Genome Biol 11, R88.) format. VAAST also comes with a series of premade and annotated background condenser files for the 1000 genomes (Consortium, 2010, Nature 467, 1061-1073) data and the 10Gen dataset (Reese et al., 2010, Genome Biol 11, R88). Also needed is a third file in GFF3 (www.sequenceontology.org/resources/gff3.html) containing genome features to be searched.

**Basic CLR method.**

The composite likelihood ratio (CLR) test can evaluate whether a gene or other genomic feature contributes to disease risk. The first step is to calculate the likelihood of the null and alternative models assuming independence between nucleotide sites and then to evaluate the significance of the likelihood ratio by permutation to control for LD. The basic method is a nested CLR test that depends only on differences in allele frequencies between affected and unaffected individuals. Sites with rare minor alleles are collapsed into one or more categories, but the total number of minor allele copies among all affected and unaffected individuals is counted rather than just the presence or absence of minor alleles within an individual. For the analyses, the collapsing threshold is set at fewer than 5 copies of the minor allele among all affected individuals, but this parameter is adjustable. Let *k* equal the number of uncollapsed variant sites among $n_i^U$ unaffected and $n_i^A$ affected individuals, with $n_i$ equal to $n_i^U + n_i^A$. Let $l_{k+1}..l_{k+m}$ equal the number of collapsed variant sites within $m$ collapsing categories labeled $k$+1 to $m$, and let $l_1..l_k$ equal 1. Let $X_i$, $X_i^U$, and $X_i^A$ equal the number of copies of the minor allele(s) at variant site $i$ or collapsing category $i$ among all individuals, unaffected individuals, and affected individuals, respectively. Then the log-likelihood ratio is equal to:

$$\text{Equation 1. } \lambda = \ln\left(\frac{L_{Null}}{L_{Alt}}\right) = \sum_{i=1}^{k+m} \ln\left[\frac{(\hat{p}_i)^{X_i}(1-\hat{p}_i)^{2l_i n_i - X_i}}{(\hat{p}_i^U)^{X_i^U}(1-\hat{p}_i^U)^{2l_i n_i^U - X_i^U}(\hat{p}_i^A)^{X_i^A}(1-\hat{p}_i^A)^{2l_i n_i^A - X_i^A}}\right]$$

where $p_i$, $p_i^U$, and $p_i^A$ equal the maximum likelihood estimates for the frequency of minor allele(s) at variant site $i$ or collapsing category $i$ among all individuals, unaffected individuals, and affected individuals, respectively. When no constraints are placed on the frequency of disease-causing variants, the maximum likelihood estimates are equal to the observed frequencies of the minor allele(s). Assuming that variant sites are unlinked, -2$\lambda$ approximately follows a $\chi^2$ distribution with $k+m$ degrees of freedom. The $\log_e$ of the $\chi^2$ p-value are reported as the VAAST score to provide a statistic for rapid prioritization of disease-gene candidates. To evaluate the statistical significance of a genomic feature, a randomization test is performed by permuting the affected/unaffected status of each individual (or each individual chromosome, when phased data are available). Because the degrees of freedom can vary between iterations of the permutation test, the $\chi^2$ p-value is used as the test statistic for the randomization test.

**Extensions to the basic CLR method.**

In the basic CLR method, the null model is fully nested within the alternative model. Extensions to this method result in models that are no longer nested. Because the $\chi^2$ approximation is only appropriate for likelihood ratio tests of nested models, Vuong's closeness test is applied in extended CLR tests using the Akaike Information Criterion correction factor. Thus, the test statistic used in the permutation tests for these methods is -2$\lambda$-2($k$+$m$). To efficiently calculate the VAAST score for non-nested models, an importance sampling technique is employed in a randomization test that assumes independence between sites by permuting the affected/unaffected status of each allele at each site. To evaluate the statistical significance of genomic features for these models, the affected/unaffected status of each individual (or each individual chromosome) is permuted.

For rare diseases, the allele frequency of putative disease-causing alleles in the population background is constrained such that $p^{iU}$ cannot exceed a specified threshold, $t$, based on available information about the penetrance, inheritance mode, and prevalence of the disease. With this constraint, the maximum likelihood estimate for $p^{iU}$ is equal to the minimum of $t$ and $x_i/l_i n_i$.

The framework can incorporate various categories of indels, splice-site variants, synonymous variants, and non-coding variants. Methods incorporating amino acid severity and constraints on allele frequency can result in situations where the alternative model is less likely than the null model for a given variant. In these situations, the variant from the likelihood calculation is excluded, accounting for the bias introduced from this exclusion in the permutation test. For variants sufficiently rare to meet the collapsing criteria, the variant from the collapsing category is excluded if the alternative model is less likely than the null model prior to variant collapse.

**Severity of Amino Acid changes.**

To incorporate information about the potential severity of amino acid changes, we include one additional parameter in the null and alternative model for each variant site or collapsing category. The parameter $h_i$ in the null model is the likelihood that the amino acid change does not contribute to disease risk. We estimate $h_i$ by setting it equal to the proportion of this type of amino acid change in the population background. The parameter $a_i$ in the alternative model is the likelihood that the amino acid change contributes to disease risk. We estimate $a_i$ by setting it equal to the proportion of this type of amino acid change among all disease-causing mutations in OMIM (Yandell et al., 2008). Incorporating information about amino acid severity, $\lambda$ is equal to:

Equation 2.
$$\lambda = \ln\left(\frac{L_{Null}}{L_{Alt}}\right) = \sum_{i=1}^{k+m} \ln\left[\frac{h_i(\hat{p}_i)^{X_i}(1-\hat{p}_i)^{2l_i n_i - X_i}}{a_i(\hat{p}_i^U)^{X_i^U}(1-\hat{p}_i^U)^{2l_i n_i^U - X_i^U}(\hat{p}_i^A)^{X_i^A}(1-\hat{p}_i^A)^{2l_i n_i^A - X_i^A}}\right].$$

To include the severity of amino acid changes for collapsed rare variants, m collapsing categories are created that are divided according to the severity of potential amino acid changes. To create the collapsing categories, all possible amino acid changes are first ranked according to their severity. An equal number of potential changes are then assigned to each category, with the first category receiving the least severe changes and each subsequent category receiving progressively more severe changes. Each rare variant is then included in the category with its corresponding amino acid change. For each collapsing category, *i*, the parameters $h_i$ and $a_i$ are set equal to their average values among all variants present in the category. The likelihood of the null and alternative models is first calculated assuming independence between nucleotide sites and then the significance of the likelihood ratio is evaluated by permutation to control for LD.

**Scoring non-coding variants.**

The VAAST CLR framework can also score non-coding variants and synonymous variants within coding regions. Because ascertainment bias in OMIM can cause a bias against such variants, an evolutionary approach was taken to estimate the relative impacts of non-coding and synonymous variants using the vertebrate-to-human genome multiple alignments downloaded from UCSC Genome Browser (hgdownload.cse.ucsc.edu/goldenPath/hg18/multiz44way/maf/). For each codon in the human genome, the frequency in which it aligns to other codons in primate genomes (wherever an open reading frame (ORF) in the corresponding genomes is available) is calculated. Then, for every codon alignment pair involving one or fewer nucleotide changes, the Normalized Mutational Proportion (NMP) is calculated. The NMP is defined as the proportion of occurrences of each such codon pair among all codon pairs with the identical human codon and with 1 or fewer nucleotide changes. For example, if the human codon 'GCC' aligned to codons in primate genomes with the following frequencies: GCC => GCC: 1000 times; GCC => GCT: 200 times; GCC => GCG: 250 times; GCC => GGG: 50 times, then the NMP value of GCC=>GCT would be 0.134 (*i.e.,* 200/(1000 + 200 + 250)). For every codon pair that involves a non-synonymous change, the severity parameter is then calculated from the OMIM database and 180 healthy genomes from the 1000 Genomes Project ($a_i/h_i$ in Eq. 2). Linear regression analysis indicates that $log(a_i/h_i)$ is significantly correlated with *log*(NMP) ($R^2$ =0.23, $p$ <0.001). This model enables estimation of the severity parameter of synonymous variants (again by linear regression), which by this approach is 0.01 (100 times less severe than a typical non-synonymous variant). A similar approach is used to derive an equivalent value for SNVs in non-coding regions. To do so, the primate alignments from UCSC were again used, but here the analysis was restricted to primate clustered DNAse hypersensitive sites and transcription factor binding regions as defined by ENCODE regulation tracks, calculating NMP for every conserved trinucleotide. The resulting severity parameter for these regions of the genome is 0.03.

**Inheritance and penetrance patterns.**

VAAST includes several options to aid in the identification of disease-causing genes matching specific inheritance and penetrance patterns. These models enforce a particular disease model within a single gene or other genomic feature. Because the disease models introduce interdependence between sites, VAAST does not provide a site-based permutation p-value for these models.

For recessive diseases, VAAST includes three models: recessive, recessive with complete penetrance, and monogenic recessive. In the basic recessive model, the likelihood calculation is constrained such that no more than two minor alleles in each feature of each affected individual will be scored. The two alleles that receive a score are the alleles that maximize the likelihood of the alternative model. The complete penetrance model assumes that all of the individuals in the control dataset are unaffected. As the genotypes of each affected individual are evaluated within a genomic feature, if any individual in the control dataset has a genotype exactly matching an affected individual, the affected individual will be excluded from the likelihood calculation for that genomic feature. This process will frequently remove all affected individuals from the calculation, resulting in a genomic feature that receives no score. In the monogenic recessive model, genomic features are only scored if all affected individuals possess two or more minor alleles at sites where the alternative (disease) model is more likely than the null (healthy) model. The two alleles can be present at different nucleotide sites in each affected individual (i.e., allelic heterogeneity is permitted), but locus heterogeneity is excluded. The models can be combined, for example, in the case of a monogenic, completely penetrant disease.

The three dominant disease models parallel the recessive models: dominant, dominant with complete penetrance, and monogenic dominant. For the basic dominant model, only one minor allele in each feature of each affected individual will be scored (the allele that maximizes the likelihood of the alternative model). For the complete penetrance dominant model, alleles will only be scored if they are absent among all individuals in the control dataset. For the monogenic dominant model, genomic features are only scored if all affected individuals posses at least one minor allele at variant sites where the alternative model is more likely than the null model.

**Protective alleles.**

For non-nested models, the default behavior is to only score variants in which the minor allele is at higher frequency in cases than in controls, under the assumption that the disease-causing alleles are relatively rare. This assumption is problematic if protective alleles are also contributing to the difference between cases and controls. By enabling the "protective" option, VAAST will also score variants in which the minor allele frequency is higher in controls than in cases. This option also adds one additional collapsing category for rare protective alleles. Because there is no available AAS model for protective alleles, $h_i$ and $a_i$ are set equal to one for these variants.

**Variant masking.**

The variant masking option allows the user to exclude a list of nucleotide sites from the likelihood calculations based on information obtained prior to the genome analysis. The masking files used in these analyses excludes sites where short reads would map to more than one position in the reference genome. This procedure mitigates the effects introduced by cross-platform biases by excluding sites that are likely to produce spurious variant calls due to improper alignment of short reads to the reference sequence. The three masking schemes employed are a) 60-bp single-end reads, b) 35-bp single-end reads, and c) 35-bp paired-end reads separated by 400-bp. These three masking files are included with the VAAST distribution, although VAAST can mask any user-specified list of sites. Because variant masking depends only on information provided prior to the genome analysis, it is compatible with both nested and non-nested models CLR models.

**Trio option.**

By providing the genomes of the parents of one or more affected individuals, VAAST can identify and exclude Mendelian inheritance errors for variants that are present in the affected individual but absent in both parents. Although this procedure will exclude both *de novo* mutations and sequencing errors, for genomes with an error rate of approximately 1 in 100,000, approximately 99.9% of all Mendelian inheritance errors are genotyping errors. This option is compatible with both nested and non-nested models.

**Minor reference alleles.**

Most publicly available human genome and exome sequences do not distinguish between no-calls and reference alleles at any particular nucleotide site. For this reason, VAAST excludes reference alleles with frequencies of less than 50% from the likelihood calculation by default. This exclusion can be overridden with a command-line parameter.

**Benchmark Analyses.**

The ability of VAAST, SIFT and ANNOVAR to identify mutated genes and their disease-causing variants in genome-wide searches was assessed. To do so, a set of 100 genes was randomly selected, each having at least six SNVs that are annotated as deleterious by OMIM. For each run, the OMIM variants from one of the 100 genes were inserted into the genomes of healthy individuals sampled from the Complete Genomics Diversity Panel (www.completegenomics.com/sequence-data/download-data/). For the partial representation panel **(Fig. 5B),** the OMIM variants were inserted into only a partial set of the case genomes. For example, in the panel of 66% partial representation and dominant model, 4 OMIM variants were inserted into 4 of the 6 case genomes for each gene, so that 66% of the case genomes have deleterious variants; for 66% representation under the recessive model, 4 OMIM variants were inserted into 2 of the 3 case genomes.

VAAST was run using 443 background genomes (including 180 genomes from 1000 genomes project pilot phase, 63 Complete Genomics Diversity panel genomes, 9 published genomes and 191 Danish exomes) and with the inheritance model option (-iht). SIFT was run using its web-service (sift.jcvi.org/www/SIFT_chr_coords_submit.html, as of 5/3/2011). For ANNOVAR, version: 2011-02-11 00:07:48 was used with the 1000 Genomes Project 2010 July release as variant database. The automatic annotation pipeline (auto_annovar.pl) and default parameters for annotation was used, setting its -maf option to the upper 99% confidence interval of the expected minor allele frequency (MAF), such that the combined MAF for inserted alleles did not exceed 5%. dbSNP database was not used in this analysis because ANNOVAR's dbSNP130 database does not provide MAF information, and a portion of disease-causing OMIM alleles are collected by dbSNP130. Setting -maf and excluding dbSNP130 for this analysis greatly improved the accuracy of ANNOVAR in comparison to its default parameters; these more favorable parameters were used for the comparisons.

To compare the performance of the three algorithms with a sample size of 6 under a dominant model, for each of the 100 genes, the 6 different OMIM variants located in this gene were inserted into 6 different healthy genomes, making all of them heterozygous for a different disease-causing SNV at that locus. Under the recessive model, with a sample size of 2 for example, 4 different OMIM variants located in each gene were inserted into 2 healthy genomes, so that each case genome carries two different OMIM variants in this gene, *i.e.,* the individuals are compound heterozygotes.

**Example 2: VAAST Scores**

VAAST combines variant frequency data with AAS (Amino Acid Substitution) effect information on a feature-by-feature basis **(Fig. 1)** using the likelihood ratio ($\lambda$) shown in equations 1 and 2. Importantly, VAAST can make use of both coding and non-coding variants when doing so (see methods). The numerator and denominator in eq. 1 give the composite likelihoods of the observed genotypes for each feature under a healthy and disease model, respectively. For the healthy model, variant frequencies are drawn from the combined control (background) and case (target) genomes ($p_i$ in eq. 1); for the disease model variant frequencies are taken separately from the control genomes ($p_i^U$ in eq. 2) and the case genomes file ( $p_i^A$ in eq. 1), respectively. Similarly, genome-wide Amino Acid Substitution (AAS) frequencies are derived using the control (background) genome sets for the healthy model; for the disease model these are based either upon the frequencies of different AAS observed for OMIM alleles or from the BLOSUM matrix, depending upon user preference. **Fig. 2** shows the degree to which AAS frequencies among known disease-causing alleles in OMIM and AAS frequencies in healthy personal genomes differ from the BLOSUM model of amino acid substitution frequencies. As can be seen, the AAS frequency spectra of these datasets differ markedly from one another. The differences are most notable for stop codons, in part because stop gains and losses are never observed in the multiple protein alignments used by AAS methods and LOD-based scoring schemes such as BLOSUM.

VAAST aggregately scores variants within genomic features. In principle, a feature is simply one or more user-defined regions of the genome. The analyses reported here use protein-coding human gene models as features. Each feature's score is the one-tailed $\log_e$ probability of observing $\lambda$, which is estimated from a randomization test that permutes the case/control status of each individual. For the analyses reported below, the genome-wide statistical significance level (assuming 21,000 protein coding human genes) is 0.05/21,000 = $2.4 \times 10^{-6}$.

**Example 3: Comparison to AAS approaches.**

Our approach to determining a variant's impact on gene function allows VAAST to score a wider spectrum of variants than existing AAS methods (Lausch et al., 2008, Am J Hum Genet;83(5):649-55) (see Example 1, Eq. 2. for more details). SIFT (Kumar et al., 2009, Nat Protoc 4, 1073-1081), for example, examines non-synonymous changes in human proteins in the context of multiple alignments of homologous proteins from other organisms. Because not every human gene is conserved, and because conserved genes often contain un-conserved coding regions, an appreciable fraction of non-synonymous variants cannot be scored by this approach. For example, for the genomes shown in Table 2, about 10% of non-synonymous variants are not scored by SIFT due to a lack of conservation. VAAST, on the other hand, can score all non-synonymous variants. VAAST can also score synonymous variants and variants in non-coding regions of genes, which typically account for the great majority of SNVs (Single Nucleotide Variants) genome-wide. Because AAS approaches such as SIFT cannot score these variants, researchers typically either exclude them from the search entirely, or else impose a threshold on the variants' frequencies as observed in dbSNP or in the 1000 Genomes Project (Consortium, 2010 Nature 467, 1061-1073) dataset. VAAST takes a more rigorous, computationally tractable approach: the VAAST score assigned to a non-coding variant is not merely the reciprocal of the variant's frequency; rather the non-coding variant's score is a log likelihood ratio that incorporates an estimate of the severity of the substitution as well as the allele frequencies in the control and case genomes (see Example 1, subsection entitled *Scoring non-coding variants* for details).

**Table 1. Variant prioritization accuracy comparisons.**

| | Percent Judged Deleterious | | |
|---|---|---|---|
| | SIFT | Annovar | VAAST |
| **Diseased** | 58% | 71% | 99% |
| **Healthy** | 12% | 1% | 10% |

(continued)

| | Percent Judged Deleterious | | |
|---|---|---|---|
| | **SIFT** | **Annovar** | **VAAST** |
| **Accuracy** | 80% | 88% | 95% |

SIFT, ANNOVAR and VAAST were run on a collection of 1454 known disease casing variants (Diseased) and 1454 presumably healthy variants randomly chosen from 5 different CEU genomes (Healthy). The top portion of the table reports the percentage of variants in both sets judged deleterious by the three tools. The bottom row reports the accuracy of each tool. The filtering criteria used in ANNOVAR excluded all variants present in the 1000 Genomes Project data and dbSNP130 as well as any variant residing in a segmentally duplicated region of the genome. For the "Diseased" category, the VAAST control dataset contained 196 personal genomes drawn from the 1000 Genomes Project and 10Gen datasets and dbSNP130. For the "Healthy" category, the VAAST control dataset contained 55 other European-American genomes drawn from the 1000 Genomes Project dataset (to match the ethnicity of the 1454 CEU alleles).

**Table 2. Effect of background file size and stratification on accuracy.**

| Caucasian Only (65 Genomes) | | | | | |
|---|---|---|---|---|---|
| | **Genome-wide Significant Genes** | **DHODH** | | **DNAH5** | |
| | | **Rank** | **P-Value** | **Rank** | **P-Value** |
| **UMSK** | 32 | 25 | 7.92 E-07 | 32 | 1.98 E-06 |
| **MSK** | 17 | 14 | 9.93 E-07 | 19 | 5.79 E-05 |
| **Mixed Ethnicities (189 Genomes** | | | | | |
| | **Genome-wide Significant Genes** | **DHODH** | | **DNAH5** | |
| | | **Rank** | **P-Value** | **Rank** | **P-Value** |
| **UMSK** | 16 | 9 | 6.78 E-09 | 5 | 2.00 E-09 |
| **MSK** | 9 | 4 | 7.60 E-09 | 5 | 1.18 E-08 |

Results or searching the intersection or two Utah Miller Syndrome affected genomes against two different background files, with and without masking. Caucasians Only: 65 Caucasian genomes drawn from 6 different sequencing/alignment/variant calling platforms. Mixed Ethnicities: 189 genomes (62 YRI, 65 CAUC, 62 ASIAN), all from the 1000 Genomes Project. UMSK: unmasked; MSK: masked. Genome-wide Significant Genes: number of genes genome-wide attaining a significant non-LD corrected p-value. Rank: Gene rank of DHODH & DNAH5 among all scored genes; P-Value: non-LD corrected p-value; genome-wide significant alpha is 2.4E-6. Data was generated using a fully penetrant, monogenic recessive model. Causative allele incidence was set to 0.00035.

To illustrate the consequences of VAAST's novel approach to non-synonymous variant scoring, we compared it to two widely used tools for variant prioritization, SIFT and ANNOVAR. Using a previously published dataset of 1,454 high-confidence known disease-causing and predisposing coding variants from OMIM, we asked what fraction were identified as deleterious by each tool. SIFT correctly identified 57.5% of the disease-causing variants ($P < 0.05$), ANNOVAR identified 71%, and VAAST identified 98.0% (Table 1). We then carried out the same analysis using 1,454 non-synonymous variants, randomly drawn from 5 different European-American (CEU) genome sequences by the 1000 Genomes Project(Consortium, 2010). These variants are unlikely to be disease-causing given that the individuals are healthy adults. SIFT incorrectly identified 11.9% of the "healthy" variants as deleterious ($P < 0.05$), ANNOVAR identified 0.8%, and VAAST identified 8.12%. Under the assumption that there are 1,454 true positives, and an equal number of true negatives, these two analyses indicate that overall the accuracy (Sensitivity+Specificity/2) of SIFT was 79.8%, ANNOVAR 88.3%, and VAAST 94.9% (Table 1). **Fig. 5C** provides a comparison of the same three tools in the context of genome-wide disease-gene hunts.

We also used these data to investigate the relative contribution of AAS and variant frequency information to VAAST's allele prioritization accuracy. Running VAAST without using any AAS information, its accuracy decreased from 95% to 80%, demonstrating that the AAS information contributes significantly to VAAST's accuracy in identifying deleterious alleles.

**Example 4: Population Stratification.**

The impact of population stratification on VAAST's false- positive rate is shown in **Fig. 3A** (dark grey; "No Masking"). In this test we employed 30 European-American genomes as a background file and various mixtures of 30 European-American and Yoruban (African) genomes as targets. We then ran VAAST on these mixed datasets and observed the number of genes with VAAST scores that reached genome-wide significance, repeating the process after replacing one of the target or background genomes with a Yoruban genome from the 1000 Genomes dataset, until the target contained 30 Yoruban genomes and the background set contained 30 European-American genomes. The resulting curve shown in dark grey ("No Masking") in **Fig. 3A** thus reports the impact of differences in population stratification in cases and controls on VAAST's false positive prediction rate. With complete stratification (e.g., all genomes in the target are Yoruban and all background genomes are CEU) 1,087 genes have LD corrected genome-wide statistically significant scores (alpha = $2.4x10^{-6}$).

**Example 5: Platform errors.**

The impact of bias in sequencing platform and variant-calling procedures on false-positive rates was also investigated using a similar approach to example 4. Here we varied the number of case genomes drawn from different sequencing platforms and alignment/variant-calling pipelines. We began with 30 background genomes drawn from the CEU subset of the 1000 Genomes Project initial release. All of the selected genomes were sequenced to ~6X and called using the 1000 Genomes Projects variant-calling pipeline. The target file in this case consisted of 30 similar 1000 Genomes Project CEU Genomes that were not included in the background file. This was the starting point for these analyses. We then ran VAAST and recorded the number of genes with genome-wide statistically significant scores (alpha = $2.4x10^{-6}$), repeating the process after substituting one of the target genomes with a non-1000 Genomes Project European-American (CEU) genome. We repeated this process 30 times. These results are shown in **Fig. 3B** (dark grey; "No Masking"). Taken together, the results in **Fig. 3** quantify the impact of population stratification and the cumulative effects of platform differences, coverage and variant-calling procedures on false positive rates, and allow comparisons of relative magnitude of platform-related biases to population stratification effects. With all background genomes from the subset of 1000 Genomes Project data describe above, and all target genomes from datasets other than the 1000 Genomes dataset, 107 genes have genome-wide LD corrected statistically significant scores (alpha = $2.4x10^{-6}$), compared to the 1,087 observed in our population stratification experiments (alpha = $2.4x10^{-6}$).

**Example 6: Variant Masking.**

The limited number of personal genomes available today necessitates comparisons of genomes sequenced on different platforms, to different depths of coverage, and subjected to different variant-calling procedures. As shown in **Fig. 3B,** these factors can be a major source of false-positives in disease-gene searches. Based upon an analysis of these data, we found variant-calling errors to be over-represented in low-complexity and repetitive regions of the genome, which is not unexpected. We therefore developed a VAAST runtime option for masking variants within these regions of the genome. VAAST users specify a read length and paired or un-paired reads. VAAST then identifies all variants in non-unique regions of the genome meeting these criteria and excludes them from its calculations. The light grey lines ("Masking") in **Fig. 3** plot the numbers of genes attaining LD-corrected genome-wide significance after masking. As can be seen, whereas masking has negligible impact on false-positives due to population stratification, it has a much larger impact on sequencing platform and variant-calling bias. This is a desirable behavior. Population stratification introduces real, but confounding, signals into disease gene searches, and these real signals are unaffected by masking **(Fig. 3A).** In contrast, masking eliminates many false positives due to noise introduced by systematic errors in sequencing platform and variant-calling procedures **(Fig. 3B).**

**Example 7: Identification of Genes and Variants that Cause Rare Diseases.**

**Miller syndrome.**

Our targets in these analyses were the exome sequences of two siblings affected with Miller syndrome. Previous work has shown that the phenotypes of these individuals result from variants in two different genes. The affected siblings' craniofacial and limb malformations arise from compromised copies of *DHODH,* a gene involved in pyrimidine metabolism. Both affected siblings also suffer from primary ciliary dyskinesia as a result of mutations in another gene, *DNAH5,* which encodes a ciliary dynein motor protein. Both affected individuals are compound heterozygotes at both of these loci. Thus, this dataset allows us to test VAAST's ability to identify disease-causing loci when more than one locus is involved and the mutations at each locus are not identical by position or descent.

***Accuracy on the Miller syndrome data.***

We carried out a genome-wide search of 21,000 protein-coding genes using the two affected Miller syndrome exomes as targets and using two different healthy genome background files. The first background file consists of 65 European-American (CEU) genomes selected from the 1000 Genomes Project data and the 10Gen dataset. The second, larger background file consists of 189 genomes selected from the same data sources, but, in distinction to the first, is ethnically heterogeneous and contains a mixture of sequencing platforms, allowing us to assay the impact of these factors on VAAST's performance in disease-gene searches. In these experiments we ran VAAST using its recessive disease model option (see methods for a description of VAAST disease models), and with and without its variant-masking option. Depending upon whether or not its variant-masking option was used, VAAST identified a maximum of 32, and a minimum of 9, candidate genes. Variant masking, on average, halved the number of candidates (Table 2). The best accuracy was obtained using the larger background file together with the masking option. *DHODH* ranked 4$^{th}$ and *DNAH5* 5$^{th}$ among the 21,000 human genes searched. This result demonstrates that VAAST can identity both disease genes with great specificity using a cohort of only two related individuals, both compound heterozygotes for a rare recessive disease. Overall, accuracy was better using the second, larger background file, demonstrating that, for rare diseases, larger background datasets constructed from a diverse set of populations and sequencing platforms improve VAAST's accuracy, despite the stratification issues these datasets introduce.

We also took advantage of family quartet information to demonstrate the utility of pedigree information for VAAST searches. When run with the pedigree and variant masking options, only two genes are identified as candidates: *DNAH5* is ranked 1$^{st}$, *and DHODH* is ranked 2$^{nd}$, demonstrating that VAAST can achieve perfect accuracy using only a single family quartet of exomes **(Fig. 4).** Our previously published analysis identified four candidate genes, and further, expert post-hoc analyses were required to identify the two actual disease-causing genes. The results shown in **Fig. 4** thus demonstrate that VAAST can use pedigree data to improve its accuracy, which can be critical for clinical decision making and separates the disclosed methods from other computational tools. even in the face of confounding signals due to relatedness of target exomes, significant population stratification and platform-specific noise.

***Impact of non-coding SNVs.***

We used these same datasets to investigate the impact of using both coding and non-coding variants in our searches. To do so we extended our search to include all SNVs at synonymous codon positions and in conserved DNase hypersensitive sites and transcription factor-binding sites (see Example 1 for details). Doing so added an additional 36,883 synonymous and regulatory variants to the 19,249 non-synonymous changes we screened in the analyses reported above. Using only the two Utah siblings, 189 candidate genes are identified. DHODH is ranked 15$^{th}$ and DNAH5 is 6$^{th}$ among them. Repeating the analysis using family quartet information, 23 candidate genes are identified; DHODH is ranked 4$^{th}$ and *DNAH5* ranked 1$^{st}$. Thus, increasing the search space to include almost 37,000 additional non-coding variants had little negative impact on accuracy.

*Impact of cohort size.*

We also used the Miller syndrome data to assess the ability of VAAST to identify disease-causing genes in very small case cohorts wherein no two individuals in the target dataset are related or share the same disease-causing variants. We also wished to determine the extent to which the relatedness of the two siblings introduced spurious signals into the analyses reported in Table 2. We used information from additional Miller syndrome kindreds to test this scenario. To do so, we used a publically available set of Danish exome sequences. We added two different disease-causing variants in *DHODH* reported in individuals with Miller syndrome to 6 different Danish exomes to produce 6 unrelated Danish exomes, each carrying two different Miller syndrome causative alleles; no two of the genomes share any Miller syndrome alleles in common. The background file consisted of the same 189 genome-equivalents of mixed ethnicities and sequencing platforms used in Table 2. We then used VAAST to carry out a genome-wide screen using these six exomes as targets. We first used one exome as a target, then the union of two exomes as a target, and so on, in order to investigate VAAST's performance in a series of case cohorts containing pools of one to six exomes. The results are shown in Table 3.

**Table 3. Impact of cohort size on VAAST's ability to identify a rare-disease caused by compound heterozygous alleles.**

| Target Genome(s) | Genome Wide | | | DHODH Rank | | |
|---|---|---|---|---|---|---|
| | Genes Scored | Significant Genes | | Rank | P-Value | |
| | | Non LD Corrected | LD Corrected | | Non LD Corrected | LD Corrected |
| 1 Compound Heterozygote | 92 | 67 | 0 | 86 | 2.36 E-04 | 5.26 E-03 |
| 2 Compound Heterozygotes | 4 | 3 | 0 | 2 | 2.81 E-08 | 5.51 E-05 |
| 3 Compound Heterozygotes | 2 | 2 | 1 | 1 | 2.61 E-11 | 8.61 E-07 |
| 4 Compound Heterozygotes | 1 | 1 | 1 | 1 | 1.99 E-15 | 1.78 E-08 |
| 5 Compound Heterozygotes | 1 | 1 | 1 | 1 | 6.95 E-15 | 4.60 E-10 |
| 6 Compound Heterozygotes | 1 | 1 | 1 | 1 | 5.79 E-17 | 1.42 E-11 |
| Background file consisted of 189 genomes of mixed ethnicity from the 1000 Genomes Project combined with 9 additional genomes of mixed ethnicity and sequencing platforms drawn from the 10Gen genome set, Reese *et al*, 2010. Causative alleles reported in Ng *et al., 2010* were added to unrelated exomes from re-sequenced individuals from Denmark reported in Li *et al.,* 2010. Data were generated using a fully penetrant monogenic recessive model (see Table 6). Causative allele incidence was set to 0.00035 (see Table 6 for details), and amino acid substitution frequency was used along with and masking of repeats. Genes scored: number of genes in genome with variant distributions consistent with VAAST's fully penetrant monogenic recessive model and causative allele incidence threshold. Scoring was evaluated by permutation by gene and permutation by genome. | | | | | | |

*DHODH* is the highest ranked of 2 candidates for a cohort of three unrelated individuals and the only candidate to achieve LD-corrected genome-wide statistical significance (Table 3). In this dataset no two individuals share the same variants, nor are any homozygous for a variant. This dataset thus demonstrates VAAST's ability to identify a disease-causing gene in situations where the gene is enriched for rare variants, but no two individuals in the case dataset share the same variant, and the cohort size is as small as three unrelated individuals. This is an example where VAAST is used on a target cohort of severe phenotype children against a background set of genomes from healthy individuals. VAAST's probabilistic framework also makes it possible to assess the relative contribution of each variant to the overall VAAST score for that gene, allowing users to identify and prioritize for follow-up studies those variants predicted to have the greatest functional impact on a gene. Table 4 shows these scored variants for the Miller syndrome alleles of all six affected individuals.

**Table 4. Relative impacts of observed variants in DHODH.**

| Sequence Information | | | | VAAST Score | SIFT Scoring | |
|---|---|---|---|---|---|---|
| Genomic Position | Reference Sequence | Variant Genotype | Amino Acid Substitution | | Score | Impact |
| Chr16: 70599943 | T | C,T | Promoter | 0.00 | N/A | Unable to Score |
| Ch16: 70600183 | A | C,C | K→Q | 0.00 | 0.19 | TOLERATED (rs3213422:C) |
| Ch16: 70603484 | G | G,A | G→E | 4.87 | 0.05 | DAMAGING (novel) |

(continued)

| Sequence Information | | | | VAAST Score | SIFT Scoring | |
|---|---|---|---|---|---|---|
| Genomic Position | Reference Sequence | Variant Genotype | Amino Acid Substitution | | Score | Impact |
| Ch16: 70606041 | C | C,T | R→C | 6.21 | 0.00 | DAMAGING (novel) |
| Ch16: 70608443 | G | GA | G→R | 19.08 | 0.00 | DAMAGING (novel) |
| Ch16: 70612601 | C | C,T | R→C | 6.21 | 0.00 | DAMAGING (novel) |
| Ch16: 70612611 | G | G,C | G→A | 25.17 | 0.16 | Tolerated (novel) |
| Ch16: 70612617 | T | T,C | L→P | 5.19 | 0.02 | DAMAGING (novel) |
| Ch16: 70613786 | C | C,T | R→W | 6.66 | 0.02 | DAMAGING (novel) |
| Ch16: 70612596 | C | C,T | T→I | 3.52 | 0.00 | DAMAGING (novel) |
| Ch16: 70614936 | C | C,T | R→W | 13.27 | 0.00 | DAMAGING (novel) |
| Ch16: 70615586 | A | A,G | D→G | 5.16 | 0.06 | Tolerated (novel) |
| Score contribution column shows the magnitude of impact of each observed variant in *DHODH* to its final score. Red, most severe, green least severe. For comparison SIFT values are also shown. Note that SIFT judges two of the known disease causing alleles as tolerated, and is unable to score the non-coding SNV. Target file contained 6 unrelated individuals with compound heterozygous variants described in Table 3. Background file consisted of 189 genomes of mixed ethnicity from the 1000 Genomes Project combined with 9 additional genomes of mixed ethnicity and sequencing platforms drawn from the 10Gen set. Data were generated using VAAST's fully penetrant monogenic recessive model and masking. Causative allele incidence was set to 0.00035. | | | | | | |

**Congenital Chloride Diarrhea (CCD) dataset.**

We tested VAAST's ability to identify the genetic variant responsible for a rare recessive disease using the whole-exome sequence of a patient diagnosed with congenital chloride diarrhea (CCD) due to a homozygous D652N mutation in the *SLC26A3* gene. In this analysis the background dataset consisted of 189 European-American genomes (TABLE 5). Using the single affected exome as a target, *SLC26A3* is ranked 21$^{st}$ genome-wide. We also evaluated the impact of bias in platform and variant-calling procedures on this result. To do so, we added the CCD causative allele as a homozygote to an ethnically matched genome drawn from the 1000 Genomes dataset (TABLE 5), in the same manner that was used to generate the data in TABLE 3. Under the assumption that this rare recessive disease is due to variants at the same location in each affected genome (intersection by position), only a single pair of unrelated exomes is required to identify CCD with perfect specificity. Adding a third affected exome is sufficient to obtain LD-corrected genome-wide statistical significance, even when the selection criteria are relaxed to include the possibility of different disease-causing alleles at different positions in different individuals (union of variants by position).

**Table 5. Impact of cohort size on VAAST's ability to identify a rare recessive disease.**

| Target Genome(s) | Genome Wide | | | SLC26A3 | | |
|---|---|---|---|---|---|---|
| | Genes Scored | Significant Genes | | Rank | P-Value | |
| | | Non LD Corrected | LD Corrected | | Non LD Corrected | LD Corrected |
| **1 Homozygote** | 127 | 69 | 0 | 21 | 1.22 E-05 | 5.26 E-03 |

(continued)

| Target Genome(s) | Genome Wide | | | SLC26A3 | | |
|---|---|---|---|---|---|---|
| | Genes Scored | Significant Genes | | Rank | P-Value | |
| | | Non LD Corrected | LD Corrected | | Non LD Corrected | LD Corrected |
| Union 2-Unrelated Homozygotes | 7 | 7 | 0 | 3 | 4.74 E-10 | 5.51 E-05 |
| Intersection 2-Unrelated Homozygotes | 3 | 3 | 0 | 1 | 7.47 E-10 | 5.51 E-05 |
| Union 3-Unrelated Homozygotes | 2 | 2 | 2 | 1 | 2.83 E-13 | 8.61 E-07 |
| Intersection 3-Unrelated Homozygotes | 1 | 1 | 1 | 1 | 1.29 E-13 | 8.61 E-07 |

Background file consisted of 189 genomes of mixed ethnicity from the 1000 Genomes Project combined with 9 additional genomes of mixed ethnicity and sequencing platforms drawn from the 10Gen set. Targets: 1st Homozygote affected is the single CCD affected exome; remaining affecteds: unrelated exomes from re-sequenced individuals from Denmark with the causative allele added. Data were generated on either the union or intersection of affecteds using VAAST's fully penetrant monogenic recessive model. Causative allele incidence was set to 0.013; masking was also used. Scoring was evaluated by non-LD and LD-corrected permutation. Genes scored: number of genes in genome receiving a score > 0.

***Impact of recessive modeling on accuracy.***

We also investigated the impact of VAAST's recessive inheritance model on our rare disease analyses (Tables 6 and 7). In general, running VAAST with this option yielded improved specificity but had little impact on gene ranks. For a cohort of three unrelated Miller syndrome individuals, the recessive inheritance model had no impact on rank or specificity (Table 6). For CCD, using a cohort of three unrelated individuals, *SLC26A3* was ranked 1st in both cases, but the recessive model decreased the number of candidate genes from seven to two (Table 7). These results demonstrate VAAST's *ab initio* capabilities: it is capable of identifying disease-causing alleles with great accuracy, even without making assumptions regarding mode of inheritance. Our large-scale performance analyses, described below, support and clarify these conclusions.

**Table 6. Impact of recessive disease modeling on VAAST Miller syndrome accuracy.**

| Target Genome (s) | Recessive Modeling Employed | Genome Wide | | | DHODH Rank | | |
|---|---|---|---|---|---|---|---|
| | | Genes Scored | Significant Genes | | Rank | P-Value | |
| | | | Non LD Corrected | LD Corrected | | Non LD Corrected | LD Corrected |
| 1 Complex Heterozygote | NO | 305 | 72 | 0 | 87 | 7.35 E-05 | 5.26 E-03 |
| | YES | 92 | 67 | 0 | 86 | 2.36 E-04 | 5.26 E-03 |
| 2 Complex Heterozygotes | NO | 544 | 58 | 0 | 9 | 1.66 E-07 | 5.51 E-05 |
| | YES | 4 | 3 | 0 | 2 | 2.81 E-08 | 5.51 E-05 |
| 3 Complex Heterozygotes | NO | 849 | 111 | 1 | 1 | 6.65 E-11 | 8.61 E-07 |
| | YES | 2 | 2 | 1 | 1 | 2.61 E-11 | 8.61 E-07 |
| 4 Complex Heterozygotes | NO | 1069 | 144 | 6 | 1 | 1.55 E-16 | 1.78 E-08 |
| | YES | 1 | 1 | 1 | 1 | 1.99 E-15 | 1.78 E-08 |
| 5 Complex Heterozygotes | NO | 1277 | 170 | 8 | 1 | 2.42 E-18 | 4.60 E-10 |
| | YES | 1 | 1 | 1 | 1 | 6.95 E-15 | 4.60 E-10 |

(continued)

| Target Genome (s) | Recessive Modeling Employed | Genome Wide | | | DHODH Rank | | |
|---|---|---|---|---|---|---|---|
| | | Genes Scored | Significant Genes | | Rank | P-Value | |
| | | | Non LD Corrected | LD Corrected | | Non LD Corrected | LD Corrected |
| 6 Complex Heterozygotes | NO | 1481 | 215 | 11 | 1 | 2.85 E-19 | 1.42 E-11 |
| | YES | 1 | 1 | 1 | 1 | 5.79 E-17 | 1.42 E-11 |
| Background and target files are as described in Table 2. Gene scored refers to the number of genes in the genome with scores greater than zero and meeting the recessive model criteria, if applied. | | | | | | | |

**Table 7. Impact of recessive disease modeling on VAAST CCD Accuracy.**

| Target Genome (s) | Recessive Modeling Employed | Genome Wide | | | DHODH Rank | | |
|---|---|---|---|---|---|---|---|
| | | Genes Scored | Significant Genes | | Rank | P-Value | |
| | | | Non LD Corrected | LD Corrected | | Non LD Corrected | LD Corrected |
| Homozygous Affected | YES | 127 | 69 | 0 | 21 | 1.22 E-05 | 5.26 E-03 |
| | NO | 431 | 71 | 0 | 15 | 1.04 E-05 | 5.26 E-03 |
| Union 2-Unrelated Homozygotes | YES | 7 | 7 | 0 | 3 | 4.74 E-10 | 5.51 E-05 |
| | NO | 769 | 81 | 0 | 3 | 7.06 E-10 | 5.51 E-05 |
| Intersection 2-Unrelated Homozygotes | YES | 3 | 3 | 0 | 1 | 7.47 E-10 | 5.51 E-05 |
| | NO | 36 | 10 | 0 | 2 | 6.92 E-10 | 5.51 E-05 |
| Union 3-Unrelated Homozygotes | YES | 2 | 2 | 2 | 1 | 2.83 E-13 | 8.61 E-07 |
| | NO | 1067 | 122 | 7 | 1 | 3.52 E-13 | 8.61 E-07 |
| Intersection 3-Unrelated Homozygotes | YES | 1 | 1 | 1 | 1 | 1.29 E-13 | 8.61 E-07 |
| | NO | 32 | 11 | 2 | 1 | 3.17 E-13 | 8.61 E-07 |
| Background and target files are as described in Table 4. Genes scored refers to the number of genes in the genome with scores greater than zero and meeting the recessive model criteria, if applied. | | | | | | | |

**Example 8: Benchmark on 100 different known disease genes.**

To gain a better understanding of VAAST's performance characteristics, we also evaluated its ability to identify 100 different known disease-causing genes in genome-wide searches. For these analyses, we first randomly selected (without replacement) a known disease-causing gene from OMIM for which there existed at least six different published non-synonymous disease-causing alleles. Table 8 contains the complete list of the disease-causing variants included in this analysis. Next we randomly selected known disease-causing alleles at the selected locus (without replacement) and inserted them at their reported positions within the gene into different whole genome sequences drawn for the Complete Genomics Diversity Panel (www.completegenomics.com/sequence-data/download-data/). We then ran VAAST under a variety of scenarios (e.g., dominant, recessive, and various case cohort sizes) and recorded the rank of the disease gene, repeating the analyses for 100 different known disease genes. We also compared the performance of VAAST to SIFT and ANNOVAR using these same datasets. (Details of the experimental design can be found in Example 1.) The results of these analyses are shown in Fig. 5. In this figure the height of each box is proportional to the mean rank of the disease-causing gene for the 100 trials, and the number shown above each box is the mean rank from among 17,293 RefSeq genes. The error bars delimit the spread of the ranks, with 95% of the runs encompassed within the bars.

**Fig. 5A** summarizes VAAST's performance on this dataset under both dominant and recessive disease scenarios. For these experiments we assayed the average rank for three different cohort sizes: two, four, and six individuals for the dominant scenario, and one, two, and three individuals for the recessive analyses. For both scenarios, the mean and variance rapidly decrease as the cohort size increases. For the dominant scenario, using a case cohort of 6 unrelated individuals, each carrying a different disease-causing allele, VAAST ranked the disease-causing gene on average 9$^{th}$ out of 17,293 candidates with 95% of the runs having ranks between 5 and 40 in 100 different genome-wide searches. For the recessive scenario, using a case cohort of 3 unrelated individuals each carrying two different disease-causing variants at different positions (all compound heterozygotes), VAAST ranked the disease-causing gene on average 3$^{rd}$ out 17,293 candidates, with 95% of the runs having ranks between 2 and 10. None of the individuals had any disease-causing alleles in common.

**Fig. 5B** summarizes VAAST's performance when only a subset of the case cohort contains a disease-causing allele, which could result from: (1) no-calls at the disease-causing allele during variant calling; (2) the presence of phenocopies in the case cohort; and (3) locus heterogeneity. As can be seen in **Fig. 5B,** averages and variances decease monotonically as increasing numbers of individuals in the case cohort bear disease-causing alleles in the gene of interest. Moreover, for dominant diseases, even when 1/3 of the cases lack disease-causing alleles in the selected OMIM disease gene, VAAST achieves an average rank of 61 with 95% of the runs having ranks between 5 and 446. For recessive diseases the average was 21, with 95% of the disease genes ranking between 7 and 136 out of 17,293 genes, genome-wide.

**Fig. 5C** compares VAAST's accuracy to that of ANNOVAR and SIFT. For these analyses we used the same data used to produce **Fig. 5A,** running all three tools on a case cohort of six and three individuals for the dominant and recessive comparisons, respectively (see Example 1 for details). In these analyses, all members of the case cohort contain disease-causing alleles. For ANNOVAR, we set the expected combined disease-allele frequency at < 5% (see Example 1) as this improved ANNOVAR's performance (data not shown), but for VAAST no prior assumptions were made regarding the disease-causing alleles' frequencies in the population. VAAST outperforms both SIFT and ANNOVAR-both as regards to mean ranks and variances. VAAST, for example, achieves a mean rank of 3 for recessive diseases using 3 compound heterozygous individuals as a case cohort. SIFT achieves an average rank of 2,317, and ANNOVAR an average rank of 529. There is also much less variance in the VAAST ranks than in those of the other tools. For example, in the recessive scenario using 3 compound heterozygous individuals as a case cohort, in 95% of the VAAST runs the rank of the disease-causing gene was between ranks 2 and 10. By comparison ANNOVAR's ranks varied between 67 and 8,762 on the same data sets, and SIFT's varied between 66 and 9,107. Closer inspection of these data revealed the reasons for the high variances characteristic of SIFT and ANNOVAR. In SIFT's case the variance is due to failure to identify one or more of the disease-causing alleles as deleterious, a finding consistent with our accuracy analysis presented in Table 1. This, coupled with its inability to make use of variant frequencies, means that SIFT also identifies many very frequent alleles genome-wide as deleterious, increasing the rank of the actual disease-causing gene. ANNOVAR's performance, because it can filter candidate variants based on their allele frequencies, is thus better than SIFT's (average rank of 529 vs. 2,317). However, its variance from search to search remains high compared to VAAST, as the OMIM alleles in the analysis are distributed across a range of frequencies, and unlike VAAST, ANNOVAR is unable to leverage this information for greater accuracy.

**Table 8. Disease-causing variants.**

| Gene/ Locus | MIM Number | Ch | Position | Variant Genotype | Phenotype |
|---|---|---|---|---|---|
| RUNX2 | OMIM: 600211 | 6 | 45507660 | G:C:G; R0P,R0R | CLEIDOCRANIAL DYSPLASIA |
| RUNX2 | OMIM: 600211 | 6 | 45507726 | G:A:G; S0N,S0S | CLEIDOCRANIAL DYSPLASIA |
| RUNX2 | OMIM: 600211 | 6 | 45587992 | G:A:G; W0*,W0W | CLEIDOCRANIAL DYSPLASIA |
| RUNX2 | OMIM: 600211 | 6 | 45513755 | G:A:G; R0*,R0R | CLEIDOCRANIAL DYSPLASIA |
| RUNX2 | OMIM: 600211 | 6 | 45623019 | G:C:G; *0S,*0* | CLEIDOCRANIAL DYSPLASIA |
| RUNX2 | OMIM: 600211 | 6 | 45507678 | T:G:T; M0R,M0M | CLEIDOCRANIAL DYSPLASIA |

(continued)

| Gene/ Locus | MIM Number | Ch | Position | Variant Genotype | Phenotype |
|---|---|---|---|---|---|
| WT1 | OMIM: 607102 | 11 | 32370148 | A:G:A; F0L,F0F | FRASIER SYNDROME |
| WT1 | OMIM: 607102 | 11 | 32370839 | G:A:G; R0*,R0R | MESANGIAL SCLEROSIS, ISOLATED DIFFUSE, INCLUDED |
| WT1 | OMIM: 607102 | 11 | 32370142 | G:A:G; R0S,R0R | MEACHAM SYNDROME, INCLUDED |
| WT1 | OMIM: 607102 | 11 | 32370141 | C:G:C; R0S,R0R | DENYS-DRASH SYNDROME |
| WT1 | OMIM: 607102 | 11 | 32370826 | C:T:C; R0Y,R0R | DENYS-DRASH SYNDROME |
| WT1 | OMIM: 607102 | 11 | 32378147 | T:C:T; S0G,S0S | MESOTHELIOMA, SOMATIC |
| HBA1 | OMIM: 141800 | 16 | 166968 | C:G:C; H0G,H0H | HEMOGLOBIN POITIERS |
| HBA1 | OMIM: 141800 | 16 | 167015 | G:C:G; K0D,K0K | HEMOGLOBIN ZAMBIA |
| HBA1 | OMIM: 141800 | 16 | 167100 | C:T:C;H0*, H0H | HEMOGLOBIN VILLEURBANNE |
| HBA1 | OMIM: 141800 | 16 | 166739 | G:C:G; K0N,K0K | HEMOGLOBIN TATRAS |
| HBA1 | OMIM: 141800 | 16 | 167005 | G:A:G; G0H,G0G | HEMOGLOBIN NISHIK |
| HBA1 | OMIM: 141800 | 16 | 166766 | G:C:G; K0V,K0K | HEMOGLOBIN BEIJING |
| VHL | OMIM: 608537 | 3 | 10158649 | C:T:C; P0S,P0P | VON HIPPEL-LINDAU SYNDROME |
| VHL | OMIM: 608537 | 3 | 10166488 | C:T:C;R0*, R0R | VON HIPPEL-LINDAU SYNDROME |
| VHL | OMIM: 608537 | 3 | 10166503 | G:T:G; V0F,V0V | VON HIPPEL-LINDAU SYNDROME |
| VHL | OMIM: 608537 | 3 | 10158808 | G:A:G; G0S,G0G | PHEOCHROMOCYTOMA |
| VHL | OMIM: 608537 | 3 | 10166506 | C:T:C;R0*, R0R | PHEOCHROMOCYTOMA, INCLUDED |
| VHL | OMIM: 608537 | 3 | 10163233 | G:T:G; D0Y,D0D | ERYTHROCYTOSIS, FAMILIAL, 2 |
| EBP | OMIM: 300205 | X | 48267156 | T:C:T;L0P, L0L | CHONDRODYSPLASIA PUNCTATA 2, X-LINKED DOMINANT, ATYPICAL |
| EBP | OMIM: 300205 | X | 48267290 | C:T:C;R0*, R0R | CHONDRODYSPLASIA PUNCTATA 2, X-LINKED DOMINANT |
| EBP | OMIM: 300205 | X | 48271619 | C:T:C;Q0*, Q0Q | CHONDRODYSPLASIA PUNCTATA 2, X-LINKED DOMINANT |
| EBP | OMIM: 300205 | X | 48270534 | G:A:G; W0*,W0W | CHONDRODYSPLASIA PUNCTATA 2, X-LINKED DOMINANT |

(continued)

| Gene/ Locus | MIM Number | Ch | Position | Variant Genotype | Phenotype |
|---|---|---|---|---|---|
| EBP | OMIM: 300205 | X | 48267190 | G:A:G; W0*,W0W | CHONDRODYSPLASIA PUNCTATA 2, X-LINKED DOMINANT |
| EBP | OMIM: 300205 | X | 48267341 | G:A:G; E0K,E0E | CHONDRODYSPLASIA PUNCTATA 2, X-LINKED DOMINANT |
| F8 | OMIM: 306700 | X | 153742057 | C:A:C; W0C,W0W | HEMOPHILIA A |
| F8 | OMIM: 306700 | X | 153744611 | G:T:G; P0Q,P0P | HEMOPHILIA A |
| F8 | OMIM: 306700 | X | 153743228 | G:A:G; R0L,R0R | HEMOPHILIA A |
| F8 | OMIM: 306700 | X | 153744594 | G:A:G; R0F,R0R | HEMOPHILIA A |
| F8 | OMIM: 306700 | X | 153742007 | T:C:T; Q0R,Q0Q | HEMOPHILIA A |
| F8 | OMIM: 306700 | X | 153743227 | C:A:C; R0L,R0R | HEMOPHILIA A |
| GJB3 | OMIM: 603324 | 1 | 35023488 | C:T:C;R0*, R0R | DEAFNESS, AUTOSOMAL DOMINANT 2B |
| GJB3 | OMIM: 603324 | 1 | 35023075 | G:C:G; R0P,R0R | ERYTHROKERATODERMIA VARIABILIS |
| GJB3 | OMIM: 603324 | 1 | 35022984 | G:C:G; G0H,G0G | ERYTHROKERATODERMIA VARIABILIS |
| GJB3 | OMIM: 603324 | 1 | 35023497 | G:A:G; E0K,E0E | DEAFNESS, AUTOSOMAL DOMINANT 2B |
| GJB3 | OMIM: 603324 | 1 | 35023206 | T:A:T; C0S,C0C | ERYTHROKERATODERMIA VARIABILIS |
| GJB3 | OMIM: 603324 | 1 | 35022985 | G:A:G; G0H,G0G | ERYTHROKERATODERMIA VARIABILIS |
| EDA | OMIM: 300451 | X | 68753070 | C:G:C; R0G,R0R | TOOTH AGENESIS, SELECTIVE, X-LINKED, 1 |
| EDA | OMIM: 300451 | X | 68753060 | C:G:C; Y0Q,Y0Y | ECTODERMAL DYSPLASIA, X-LINKED |
| EDA | OMIM: 300451 | X | 68752944 | C:T:C;Q0*, Q0Q | ECTODERMAL DYSPLASIA, X-LINKED |
| EDA | OMIM: 300451 | X | 68753058 | T:C:T; Y0Q,Y0Y | ECTODERMAL DYSPLASIA, X-LINKED |
| EDA | OMIM: 300451 | X | 68753064 | G:A:G; E0K,E0E | ECTODERMAL DYSPLASIA, X-LINKED |
| EDA | OMIM: 300451 | X | 68753083 | G:T:G; R0L,R0R | ECTODERMAL DYSPLASIA, X-LINKED |
| BTK | OMIM: 300300 | X | 100494926 | G:T:G; A0D,A0A | AGAMMAGLOBULINEMIA, X-LINKED |
| BTK | OMIM: 300300 | X | 100495579 | C:G:C; R0S,R0R | AGAMMAGLOBULINEMIA, X-LINKED |

(continued)

| Gene/ Locus | MIM Number | Ch | Position | Variant Genotype | Phenotype |
|---|---|---|---|---|---|
| BTK | OMIM: 300300 | X | 100494908 | C:T:C; G0D,G0G | AGAMMAGLOBULINEMIA, X-LINKED |
| BTK | OMIM: 300300 | X | 100494857 | A:T:A; M0K,M0M | AGAMMAGLOBULINEMIA, X-LINKED |
| BTK | OMIM: 300300 | X | 100494973 | G:T:G;Y0*, Y0Y | AGAMMAGLOBULINEMIA, X-LINKED |
| BTK | OMIM: 300300 | X | 100494840 | C:A:C;E0*, E0E | AGAMMAGLOBULINEMIA, X-LINKED |
| DYSF | OMIM: 603009 | 2 | 71754880 | C:T:C;R0*, R0R | MUSCULAR DYSTROPHY, LIMB-GIRDLE, TYPE 2B, INCLUDED |
| DYSF | OMIM: 603009 | 2 | 71631711 | G:C:G; G0R,G0G | MIYOSHI MYOPATHY |
| DYSF | OMIM: 603009 | 2 | 71633769 | G:T:G; D0Y,D0D | MUSCULAR DYSTROPHY, LIMB-GIRDLE, TYPE 2B |
| DYSF | OMIM: 603009 | 2 | 71651342 | G:A:G; R0H,R0R | MIYOSHI MYOPATHY |
| DYSF | OMIM: 603009 | 2 | 71644712 | C:G:C; P0R,P0P | MIYOSHI MYOPATHY, INCLUDED |
| DYSF | OMIM: 603009 | 2 | 71633709 | C:T:C;Q0*, Q0Q | MIYOSHI MYOPATHY |
| SOX10 | OMIM: 602229 | 22 | 36699720 | G:A:G; Q0*,Q0Q | WAARDENBURG SYNDROME, TYPE 2E, WITH NEUROLOGIC INVOLVEMENT, INCLUDED |
| SOX10 | OMIM: 602229 | 22 | 36709489 | G:T:G;Y0*, Y0Y | WAARDENBURG SYNDROME, TYPE 4C |
| SOX10 | OMIM: 602229 | 22 | 36709334 | C:G:C; S0T,S0S | WAARDENBURG SYNDROME, TYPE 2E, WITHOUT NEUROLOGIC INVOLVEMENT |
| SOX10 | OMIM: 602229 | 22 | 36703952 | C:A:C;E0*, E0E | WAARDENBURG SYNDROME, TYPE 4C |
| SOX10 | OMIM: 602229 | 22 | 36700097 | G:T:G;S0*, S0S | SYNDROME, AND HIRSCHSPRUNG DISEASE |
| SOX10 | OMIM: 602229 | 22 | 36699910 | G:C:G; Y0*,Y0Y | SYNDROME, AND HIRSCHSPRUNG DISEASE |
| DSG2 | OMIM: 125671 | 18 | 27370259 | G:A:G; C0Y,C0C | ARRHYTHMOGENIC RIGHT VENTRICULAR DYSPLASIA, FAMILIAL, 10 |
| DSG2 | OMIM: 125671 | 18 | 27353819 | G:A:G; R0Q,R0R | ARRHYTHMOGENIC RIGHT VENTRICULAR DYSPLASIA, FAMILIAL, 10 |
| DSG2 | OMIM: 125671 | 18 | 27358826 | G:A:G; E0K,E0E | ARRHYTHMOGENIC RIGHT VENTRICULAR DYSPLASIA, FAMILIAL, 10 |
| DSG2 | OMIM: 25671 | 18 | 27358515 | A:G:A; N0S,N0N | ARRHYTHMOGENIC RIGHT VENTRICULAR DYSPLASIA, FAMILIAL, 10 |
| DSG2 | OMIM: 125671 | 18 | 27358753 | G:A:G; W0*,W0W | ARRHYTHMOGENIC RIGHT VENTRICULAR DYSPLASIA, FAMILIAL, 10 |
| DSG2 | OMIM: 125671 | 18 | 27353828 | G:A:G; R0H,R0R | ARRHYTHMOGENIC RIGHT VENTRICULAR DYSPLASIA, FAMILIAL, 10 |

(continued)

| Gene/ Locus | MIM Number | Ch | Position | Variant Genotype | Phenotype |
|---|---|---|---|---|---|
| PROC | OMIM: 176860 | 2 | 127902541 | C:T:C; S0L,S0S | THROMBOPHILIA, HEREDITARY, DUE TO PROTEIN C DEFICIENCY, AUTOSOMAL DOMINANT |
| PROC | OMIM: 176860 | 2 | 127902633 | G:A:G; G0S,G0G | THROMBOPHILIA, HEREDITARY, DUE TO PROTEIN C DEFICIENCY, AUTOSOMAL DOMINANT |
| PROC | OMIM: 176860 | 2 | 127902531 | G:A:G; A0T,A0A | THROMBOPHILIA, HEREDITARY, DUE TO PROTEIN C DEFICIENCY, AUTOSOMAL DOMINANT |
| PROC | OMIM: 176860 | 2 | 127901265 | C:T:C;L0F, L0L | THROMBOPHILIA, HEREDITARY, DUE TO PROTEIN C DEFICIENCY, AUTOSOMAL DOMINANT |
| PROC | OMIM: 176860 | 2 | 127895484 | G:A:G; V0M,V0V | THROMBOPHILIA, HEREDITARY, DUE TO PROTEIN C DEFICIENCY, AUTOSOMAL DOMINANT |
| PROC | OMIM: 176860 | 2 | 127902938 | G:C:G; W0C,W0W | THROMBOPHILIA, HEREDITARY, DUE TO PROTEIN C DEFICIENCY, AUTOSOMAL DOMINANT |
| LAMA2 | OMIM: 156225 | 6 | 129827282 | C:T:C;R0*, R0R | MUSCULAR DYSTROPHY, CONGENITAL, DUE TO PARTIAL LAMA2 DEFICIENCY |
| LAMA2 | OMIM: 156225 | 6 | 129879069 | C:T:C;R0*, R0R | MUSCULAR DYSTROPHY, CONGENITAL MEROSIN-DEFICIENT |
| LAMA2 | OMIM: 156225 | 6 | 129553155 | G:A:G; C0Y,C0C | MUSCULAR DYSTROPHY, CONGENITAL, DUE TO PARTIAL LAMA2 DEFICIENCY |
| LAMA2 | OMIM: 156225 | 6 | 129844219 | T:C:T;L0P, L0L | MUSCULAR DYSTROPHY, CONGENITAL MEROSIN-DEFICIENT |
| LAMA2 | OMIM: 156225 | 6 | 129660567 | C:A:C;C0*, C0C | MUSCULAR DYSTROPHY, CONGENITAL MEROSIN-DEFICIENT |
| LAMA2 | OMIM: 156225 | 6 | 129844260 | C:T:C;R0*, R0R | MUSCULAR DYSTROPHY, CONGENITAL MEROSIN-DEFICIENT |
| TERT | OMIM: 187270 | 5 | 1332456 | C:T:C; V0M,V0V | APLASTIC ANEMIA, SUSCEPTIBILITY TO |
| TERT | OMIM: 187270 | 5 | 1307510 | C:T:C; V0M,V0V | APLASTIC ANEMIA, SUSCEPTIBILITY TO |
| TERT | OMIM: 187270 | 5 | 1347433 | C:T:C; A0T,A0A | APLASTIC ANEMIA, SUSCEPTIBILITY TO |
| TERT | OMIM: 187270 | 5 | 1347397 | C:T:C; A0T,A0A | APLASTIC ANEMIA, SUSCEPTIBILITY TO |
| TERT | OMIM: 187270 | 5 | 1346767 | G:A:G; H0Y,H0H | APLASTIC ANEMIA, SUSCEPTIBILITY TO |
| TERT | OMIM: 187270 | 5 | 1346803 | G:A:G; H0Y,H0H | APLASTIC ANEMIA, SUSCEPTIBILITY TO |
| PHF6 | OMIM: 300414 | X | 133375633 | A:G:A; K0E,K0K | BORJESON-FORSSMAN-LEHMANN SYNDROME |

(continued)

| Gene/ Locus | MIM Number | Ch | Position | Variant Genotype | Phenotype |
|---|---|---|---|---|---|
| PHF6 | OMIM: 300414 | X | 133375619 | A:G:A; H0R,H0H | BORJESON-FORSSMAN-LEHMANN SYNDROME |
| PHF6 | OMIM: 300414 | X | 133339447 | G:A:G; C0Y,C0C | BORJESON-FORSSMAN-LEHMANN SYNDROME |
| PHF6 | OMIM: 300414 | X | 133376751 | A:G:A; R0G,R0R | BORJESON-FORSSMAN-LEHMANN SYNDROME |
| PHF6 | OMIM: 300414 | X | 133339335 | A:T:A;K0*, K0K | BORJESON-FORSSMAN-LEHMANN SYNDROME |
| PHF6 | OMIM: 300414 | X | 133355252 | G:T:G; C0F,C0C | BORJESON-FORSSMAN-LEHMANN SYNDROME |
| LAMB3 | OMIM: 150310 | 1 | 207873070 | C:G:C; G0A,G0G | REVERTANT |
| LAMB3 | OMIM: 150310 | 1 | 207873047 | T:G:T; K0Q,K0K | REVERTANT |
| LAMB3 | OMIM: 150310 | 1 | 207874483 | G:A:G; Q0*,Q0Q | EPIDERMOLYSIS BULLOSA, JUNCTIONAL, HERLITZ TYPE |
| LAMB3 | OMIM: 150310 | 1 | 207865762 | C:T:C; W0*,W0W | EPIDERMOLYSIS BULLOSA, JUNCTIONAL, HERLITZ TYPE |
| LAMB3 | OMIM: 150310 | 1 | 207889991 | G:A:G; R0*,R0R | EPIDERMOLYSIS BULLOSA, JUNCTIONAL, NON-HERLITZ TYPE, INCLUDED |
| LAMB3 | OMIM: 150310 | 1 | 207858523 | G:A:G; Q0*,Q0Q | EPIDERMOLYSIS BULLOSA, JUNCTIONAL, HERLITZ TYPE |
| ABCA12 | OMIM: 607800 | 2 | 215553577 | C:T:C; E0K,E0E | ICHTHYOSIS, LAMELLAR, 2 |
| ABCA12 | OMIM: 607800 | 2 | 215555194 | C:T:C; R0H,R0R | ICHTHYOSIS, LAMELLAR, 2 |
| ABCA12 | OMIM: 607800 | 2 | 215559532 | C:T:C; G0E,G0G | ICHTHYOSIS, LAMELLAR, 2 |
| ABCA12 | OMIM: 607800 | 2 | 215551799 | C:T:C; G0S,G0G | ICHTHYOSIS, LAMELLAR, 2 |
| ABCA12 | OMIM: 607800 | 2 | 215563760 | C:T:C; G0R,G0G | HARLEQUIN ICHTHYOSIS |
| ABCA12 | OMIM: 607800 | 2 | 215559535 | T:C:T; N0S,N0N | ICHTHYOSIS, LAMELLAR, 2 |
| FBLN5 | OMIM: 604580 | 14 | 91473211 | C:T:C; R0Q,R0R | MACULAR DEGENERATION, AGE-RELATED, 3 |
| FBLN5 | OMIM: 604580 | 14 | 91406433 | C:T:C; G0E,G0G | MACULAR DEGENERATION, AGE-RELATED, 3 |
| FBLN5 | OMIM: 604580 | 14 | 91473245 | C:G:C; V0L,V0V | MACULAR DEGENERATION, AGE-RELATED, 3 |
| FBLN5 | OMIM: 604580 | 14 | 91427431 | A:G:A;I0T, I0I | MACULAR DEGENERATION, AGE-RELATED, 3 |
| FBLN5 | OMIM: 604580 | 14 | 91413718 | G:A:G; R0W,R0R | MACULAR DEGENERATION, AGE-RELATED, 3 |

(continued)

| Gene/ Locus | MIM Number | Ch | Position | Variant Genotype | Phenotype |
|---|---|---|---|---|---|
| FBLN5 | OMIM: 604580 | 14 | 91413682 | C:T:C; A0T,A0A | MACULAR DEGENERATION, AGE-RELATED, 3 |
| LMX1B | OMIM: 602575 | 9 | 128492962 | G:T:G; C0F,C0C | NAIL-PATELLA SYNDROME |
| LMX1B | OMIM: 602575 | 9 | 128495627 | C:T:C;R0*, R0R | NAIL-PATELLA SYNDROME |
| LMX1B | OMIM: 602575 | 9 | 128495343 | C:T:C;R0*, R0R | NAIL-PATELLA SYNDROME |
| LMX1B | OMIM: 602575 | 9 | 128495373 | C:T:C;R0*, R0R | NAIL-PATELLA SYNDROME |
| LMX1B | OMIM: 602575 | 9 | 128495350 | G:A:G; R0Q,R0R | NAIL-PATELLA SYNDROME |
| LMX1B | OMIM: 602575 | 9 | 128417587 | C:T:C;Q0*, Q0Q | NAIL-PATELLA SYNDROME |
| TH | OMIM: 191290 | 11 | 2144801 | T:G:T; T0P,T0T | SEGAWA SYNDROME, AUTOSOMAL RECESSIVE |
| TH | OMIM: 191290 | 11 | 2145711 | C:T:C; R0H,R0R | SEGAWA SYNDROME, AUTOSOMAL RECESSIVE |
| TH | OMIM: 191290 | 11 | 2144350 | C:A:C; C0F,C0C | SEGAWA SYNDROME, AUTOSOMAL RECESSIVE |
| TH | OMIM: 191290 | 11 | 2144499 | C:T:C; R0H,R0R | SEGAWA SYNDROME, AUTOSOMAL RECESSIVE |
| TH | OMIM: 191290 | 11 | 2142145 | G:A:G; T0M,T0T | SEGAWA SYNDROME, AUTOSOMAL RECESSIVE |
| TH | OMIM: 191290 | 11 | 2143533 | G:T:G; Q0K,Q0Q | SEGAWA SYNDROME, AUTOSOMAL RECESSIVE |
| HLCS | OMIM: 609018 | 21 | 37059341 | G:A:G; R0W,R0R | HOLOCARBOXYLASE SYNTHETASE DEFICIENCY |
| HLCS | OMIM: 609018 | 21 | 37230905 | A:G:A; L0P,L0L | HOLOCARBOXYLASE SYNTHETASE DEFICIENCY |
| HLCS | OMIM: 609018 | 21 | 37053952 | C:T:C; G0S,G0G | HOLOCARBOXYLASE SYNTHETASE DEFICIENCY |
| HLCS | OMIM: 609018 | 21 | 37059215 | C:T:C; V0M,V0V | HOLOCARBOXYLASE SYNTHETASE DEFICIENCY |
| HLCS | OMIM: 609018 | 21 | 37053982 | C:T:C; D0N,D0D | HOLOCARBOXYLASE SYNTHETASE DEFICIENCY |
| HLCS | OMIM: 609018 | 21 | 37230968 | A:C:A; L0R,L0L | HOLOCARBOXYLASE SYNTHETASE DEFICIENCY |
| PSEN2 | OMIM: 600759 | 1 | 225149845 | C:T:C; T0M,T0T | ALZHEIMER DISEASE, FAMILIAL, 4 |
| PSEN2 | OMIM: 600759 | 1 | 225139894 | C:T:C; S0L,S0S | ALZHEIMER DISEASE, FAMILIAL, 4, INCLUDED |
| PSEN2 | OMIM: 600759 | 1 | 225149872 | A:C:A; D0A,D0D | ALZHEIMER DISEASE, FAMILIAL, 4 |

(continued)

| Gene/ Locus | MIM Number | Ch | Position | Variant Genotype | Phenotype |
|---|---|---|---|---|---|
| PSEN2 | OMIM: 600759 | 1 | 225139870 | C:G:C; T0R,T0T | ALZHEIMER DISEASE, FAMILIAL, 4 |
| PSEN2 | OMIM: 600759 | 1 | 225139869 | A:C:A; T0R,T0T | ALZHEIMER DISEASE, FAMILIAL, 4 |
| PSEN2 | OMIM: 600759 | 1 | 225143301 | A:G:A; M0V,M0M | ALZHEIMER DISEASE, FAMILIAL, 4 |
| SCN4A | OMIM: 603967 | 17 | 59373049 | A:T:A; V0E,V0V | MYASTHENIC SYNDROME |
| SCN4A | OMIM: 603967 | 17 | 59395679 | C:T:C; V0M,V0V | MYOTONIA CONGENITA, ATYPICAL |
| SCN4A | OMIM: 603967 | 17 | 59375800 | C:T:C;V0I, V0V | PARAMYOTONIA CONGENITA |
| SCN4A | OMIM: 603967 | 17 | 59375786 | G:T:G; N0K,N0N | PARAMYOTONIA CONGENITA/HYPERKALEMIC PERIODIC PARALYSIS |
| SCN4A | OMIM: 603967 | 17 | 59374917 | G:A:G; T0M,T0T | PARAMYOTONIA CONGENITA |
| SCN4A | OMIM: 603967 | 17 | 59373031 | C:T:C; R0Y,R0R | PARAMYOTONIA CONGENITA |
| PMP22 | OMIM: 601097 | 17 | 15104705 | G:A:G; S0F,S0S | CHARCOT-MARIE-TOOTH DISEASE, TYPE 1A, INCLUDED |
| PMP22 | OMIM: 601097 | 17 | 15104723 | A:G:A; L0P,L0L | CHARCOT-MARIE-TOOTH DISEASE, TYPE 1A |
| PMP22 | OMIM: 601097 | 17 | 15074973 | G:A:G; R0W,R0R | DEJERINE-SOTTAS SYNDROME, AUTOSOMAL RECESSIVE |
| PMP22 | OMIM: 601097 | 17 | 15083626 | A:T:A; M0K,M0M | DEJERINE-SOTTAS SYNDROME, AUTOSOMAL DOMINANT |
| PMP22 | OMIM: 601097 | 17 | 15083633 | C:G:C; A0P,A0A | CHARCOT-MARIE-TOOTH DISEASE AND DEAFNESS |
| PMP22 | OMIM: 601097 | 17 | 15104734 | G:T:G; H0Q,H0H | DEJERINE-SOTTAS SYNDROME, AUTOSOMAL DOMINANT |
| CYP27A1 | OMIM: 606530 | 2 | 219387376 | G:A:G; R0Q,R0R | CEREBROTENDINOUS XANTHOMATOSIS |
| CYP27A1 | OMIM: 606530 | 2 | 219387683 | C:T:C; R0C,R0R | CEREBROTENDINOUS XANTHOMATOSIS |
| CYP27A1 | OMIM: 606530 | 2 | 219386062 | C:T:C; TOM,TOT | CEREBROTENDINOUS XANTHOMATOSIS |
| CYP27A1 | OMIM: 606530 | 2 | 219382722 | G:A:G; G0E,G0G | CEREBROTENDINOUS XANTHOMATOSIS |
| CYP27A1 | OMIM: 606530 | 2 | 219387668 | C:T:C;R0*, R0R | CEREBROTENDINOUS XANTHOMATOSIS |
| CYP27A1 | OMIM: 606530 | 2 | 219387669 | G:A:G; R0*,R0R | CEREBROTENDINOUS XANTHOMATOSIS |
| HMBS | OMIM: 609806 | 11 | 118467333 | C:T:C; R0L,R0R | PORPHYRIA, ACUTE INTERMITTENT |

(continued)

| Gene/ Locus | MIM Number | Ch | Position | Variant Genotype | Phenotype |
|---|---|---|---|---|---|
| HMBS | OMIM: 609806 | 11 | 118466133 | G:A:G; R0Q,R0R | PORPHYRIA, ACUTE INTERMITTENT |
| HMBS | OMIM: 609806 | 11 | 118467435 | C:T:C; R0W,R0R | PORPHYRIA, ACUTE INTERMITTENT |
| HMBS | OMIM: 609806 | 11 | 118468878 | G:A:G; W0*,W0W | PORPHYRIA, ACUTE INTERMITTENT |
| HMBS | OMIM: 609806 | 11 | 118468877 | G:A:G; W0*,W0W | PORPHYRIA, ACUTE INTERMITTENT |
| HMBS | OMIM: 609806 | 11 | 118467352 | G:A:G; R0Q,R0R | PORPHYRIA, ACUTE INTERMITTENT |
| EGR2 | OMIM: 129010 | 10 | 64243260 | T:G:T; S0R,S0S | NEUROPATHY, CONGENITAL HYPOMYELINATING, AUTOSOMAL DOMINANT |
| EGR2 | OMIM: 129010 | 10 | 64243257 | C:A:C; D0Y,D0D | NEUROPATHY, CONGENITAL HYPOMYELINATING, AUTOSOMAL DOMINANT |
| EGR2 | OMIM: 129010 | 10 | 64243179 | G:A:G; ROW,ROR | CHARCOT-MARIE-TOOTH DISEASE, TYPE 1D |
| EGR2 | OMIM: 129010 | 10 | 64243601 | A:T:A;I0N, I0I | NEUROPATHY, CONGENITAL HYPOMYELINATING, AUTOSOMAL RECESSIVE |
| EGR2 | OMIM: 129010 | 10 | 64243170 | C:T:C; E0K,E0E | DEJERINE-SOTTAS NEUROPATHY |
| EGR2 | OMIM: 129010 | 10 | 64243329 | G:A:G; R0W,R0R | CHARCOT-MARIE-TOOTH DISEASE, TYPE 1D, INCLUDED |
| THRB | OMIM: 190160 | 3 | 24139463 | G:T:G; C0*,C0C | THYROID HORMONE RESISTANCE, GENERALIZED, AUTOSOMAL DOMINANT |
| THRB | OMIM: 190160 | 3 | 24144174 | C:G:C; D0H,D0D | THYROID HORMONE RESISTANCE, GENERALIZED |
| THRB | OMIM: 190160 | 3 | 24139438 | T:C:T; K0E,K0K | THYROID HORMONE RESISTANCE, GENERALIZED, AUTOSOMAL DOMINANT |
| THRB | OMIM: 190160 | 3 | 24144098 | C:T:C; G0E,G0G | THYROID HORMONE RESISTANCE, GENERALIZED, AUTOSOMAL DOMINANT |
| THRB | OMIM: 190160 | 3 | 24139617 | C:T:C; R0H,R0R | THYROID HORMONE RESISTANCE, GENERALIZED, AUTOSOMAL DOMINANT |
| THRB | OMIM: 190160 | 3 | 24144189 | C:T:C; A0T,A0A | THYROID HORMONE RESISTANCE, GENERALIZED, AUTOSOMAL DOMINANT |
| TGFBI | OMIM: 601692 | 5 | 135420324 | T:C:T;F0S, F0F | CORNEAL DYSTROPHY, LATTICE TYPE IIIA |
| TGFBI | OMIM: 601692 | 5 | 135420369 | G:A:G; R0*,R0R | CORNEAL DYSTROPHY, THIEL-BEHNKE TYPE |
| TGFBI | OMIM: 601692 | 5 | 135419383 | T:G:T; L0R,L0L | CORNEAL DYSTROPHY, EPITHELIAL BASEMENT MEMBRANE |
| TGFBI | OMIM: 601692 | 5 | 135420368 | C:T:C;R0*, R0R | CORNEAL DYSTROPHY, GROENOUW TYPE I |
| TGFBI | OMIM: 601692 | 5 | 135424486 | G:A:G; G0D,G0G | CORNEAL DYSTROPHY, REIS-BUCKLERS TYPE |

(continued)

| Gene/ Locus | MIM Number | Ch | Position | Variant Genotype | Phenotype |
|---|---|---|---|---|---|
| TGFBI | OMIM: 601692 | 5 | 135426262 | G:C:G; R0S,R0R | CORNEAL DYSTROPHY, EPITHELIAL BASEMENT MEMBRANE |
| DSP | OMIM: 125647 | 6 | 7511660 | C:T:C;Q0*, Q0Q | KERATOSIS PALMOPLANTARIS STRIATA II |
| DSP | OMIM: 125647 | 6 | 7487235 | G:A:G; V0M,V0V | ARRHYTHMOGENIC RIGHT VENTRICULAR DYSPLASIA, FAMILIAL, 8 |
| DSP | OMIM: 125647 | 6 | 7528294 | C:T:C;R0*, R0R | EPIDERMOLYSIS BULLOSA, LETHAL ACANTHOLYTIC |
| DSP | OMIM: 125647 | 6 | 7520018 | T:A:T;C0*, C0C | SKIN FRAGILITY-WOOLLY HAIR SYNDROME |
| DSP | OMIM: 125647 | 6 | 7510710 | C:G:C; S0R,S0S | ARRHYTHMOGENIC RIGHT VENTRICULAR DYSPLASIA, FAMILIAL, 8 |
| DSP | OMIM: 125647 | 6 | 7517160 | C:T:C;Q0*, Q0Q | SKIN FRAGILITY-WOOLLY HAIR SYNDROME |
| FAH | OMIM: 276700 | 15 | 78241679 | C:A:C; A0D,A0A | TYROSINEMIA, TYPE I |
| FAH | OMIM: 276700 | 15 | 78260517 | A:G:A; R0G,R0R | TYROSINEMIA, TYPE I |
| FAH | OMIM: 276700 | 15 | 78260466 | G:T:G;E0*, E0E | TYROSINEMIA, TYPE I |
| FAH | OMIM: 276700 | 15 | 78252490 | G:A:G; W0*,W0W | TYROSINEMIA, TYPE I |
| FAH | OMIM: 276700 | 15 | 78260445 | G:T:G;E0*, E0E | TYROSINEMIA, TYPE I |
| FAH | OMIM: 276700 | 15 | 78252540 | A:G:A; Q0R,Q0Q | TYROSINEMIA, TYPE I |
| PRKCG | OMIM: 176980 | 19 | 59084773 | T:C:T; S0P,S0S | SPINOCEREBELLAR ATAXIA 14 |
| PRKCG | OMIM: 176980 | 19 | 59084771 | G:A:G; G0D,G0G | SPINOCEREBELLAR ATAXIA 14 |
| PRKCG | OMIM: 176980 | 19 | 59084719 | C:T:C;H0*, H0H | SPINOCEREBELLAR ATAXIA 14 |
| PRKCG | OMIM: 176980 | 19 | 59084801 | G:A:G; G0D,G0G | SPINOCEREBELLAR ATAXIA 14 |
| PRKCG | OMIM: 176980 | 19 | 59084798 | A:G:A; Q0R,Q0Q | SPINOCEREBELLAR ATAXIA 14 |
| PRKCG | OMIM: 176980 | 19 | 59084721 | C:G:C; H0*,H0H | SPINOCEREBELLAR ATAXIA 14 |
| EFNB1 | OMIM: 300035 | X | 67975252 | C:T:C;R0*, R0R | CRANIOFRONTONASAL SYNDROME |
| EFNB1 | OMIM: 300035 | X | 67975388 | C:T:C;T0I, T0T | CRANIOFRONTONASAL SYNDROME |
| EFNB1 | OMIM: 300035 | X | 67966454 | G:A:G; W0E,W0W | CRANIOFRONTONASAL SYNDROME |

(continued)

| Gene/ Locus | MIM Number | Ch | Position | Variant Genotype | Phenotype |
|---|---|---|---|---|---|
| EFNB1 | OMIM: 300035 | X | 67976299 | G:T:G; M0V,M0M | CRANIOFRONTONASAL SYNDROME |
| EFNB1 | OMIM: 300035 | X | 67966453 | T:G:T; W0E,W0W | CRANIOFRONTONASAL SYNDROME |
| EFNB1 | OMIM: 300035 | X | 67975217 | C:T:C; P0L,P0P | CRANIOFRONTONASAL SYNDROME |
| CYP21A2 | OMIM: 201910 | 6 | 32115866 | G:C:G; V0L,V0V | ADRENAL HYPERPLASIA, CONGENITAL, DUE TO 21-HYDROXYLASE DEFICIENCY |
| CYP21A2 | OMIM: 201910 | 6 | 32114874 | C:T:C; P0L,P0P | LATE-ONSET FORM |
| CYP21A2 | OMIM: 201910 | 6 | 32115182 | T:A:T;I0N, I0I | CLASSIC TYPE |
| CYP21A2 | OMIM: 201910 | 6 | 32115935 | G:A:G; V0M,V0V | HYPERANDROGENISM, NONCLASSIC TYPE, DUE TO 21-HYDROXYLASE DEFICIENCY |
| CYP21A2 | OMIM: 201910 | 6 | 32116431 | G:A:G; G0S,G0G | HYPERANDROGENISM, NONCLASSIC TYPE, DUE TO 21-HYDROXYLASE DEFICIENCY |
| CYP21A2 | OMIM: 201910 | 6 | 32116675 | G:A:G; G0S,G0G | ADRENAL HYPERPLASIA, CONGENITAL, DUE TO 21-HYDROXYLASE DEFICIENCY |
| IKBKG | OMIM: 300248 | X | 153444313 | C:G:C; A0G,A0A | ECTODERMAL DYSPLASIA, ANHIDROTIC, WITH IMMUNE DEFICIENCY |
| IKBKG | OMIM: 300248 | X | 153445859 | T:C:T;C0L, C0C | INCLUDED |
| IKBKG | OMIM: 300248 | X | 153444999 | A:C:A; E0A,E0E | MYCOBACTERIAL DISEASE, SUSCEPTIBILITY TO, X-LINKED, 1 |
| IKBKG | OMIM: 300248 | X | 153445817 | C:T:C;Q0*, Q0Q | ECTODERMAL DYSPLASIA, HYPOHIDROTIC, WITH IMMUNE DEFICIENCY |
| IKBKG | OMIM: 300248 | X | 153445829 | A:G:A; M0V,M0M | INCONTINENTIA PIGMENTI, TYPE II |
| IKBKG | OMIM: 300248 | X | 153445860 | G:T:G; C0L,C0C | ECTODERMAL DYSPLASIA, HYPOHIDROTIC, WITH IMMUNE DEFICIENCY |
| NROB1 | OMIM: 300473 | X | 30236256 | C:A:C; K0N,K0K | ADRENAL HYPOPLASIA, CONGENITAL |
| NROB1 | OMIM: 300473 | X | 30236698 | C:T:C; W0*,W0W | ADRENAL HYPOPLASIA, CONGENITAL |
| NROB1 | OMIM: 300473 | X | 30232847 | G:A:G; Q0*,Q0Q | ADRENAL HYPOPLASIA, CONGENITAL |
| NROB1 | OMIM: 300473 | X | 30232711 | T:A:T;N0I, N0N | ADRENAL HYPOPLASIA, CONGENITAL |
| NROB1 | OMIM: 300473 | X | 30236889 | C:T:C; W0*,W0W | ADRENAL HYPOPLASIA, CONGENITAL |
| NROB1 | OMIM: 300473 | X | 30236555 | G:A:G; Q0*,Q0Q | ADRENAL HYPOPLASIA, CONGENITAL |
| KCNJ1 | OMIM: 600359 | 11 | 128214906 | C:T:C; G0E,G0G | BARTTER SYNDROME, ANTENATAL, TYPE 2 |

(continued)

| Gene/ Locus | MIM Number | Ch | Position | Variant Genotype | Phenotype |
|---|---|---|---|---|---|
| KCNJ1 | OMIM: 600359 | 11 | 128215084 | C:G:C; D0H,D0D | BARTTER SYNDROME, ANTENATAL, TYPE 2 |
| KCNJ1 | OMIM: 600359 | 11 | 128215034 | A:T:A; N0K,N0N | BARTTER SYNDROME, ANTENATAL, TYPE 2 |
| KCNJ1 | OMIM: 600359 | 11 | 128215169 | G:C:G; Y0*,Y0Y | BARTTER SYNDROME, ANTENATAL, TYPE 2 |
| KCNJ1 | OMIM: 600359 | 11 | 128214336 | A:G:A; M0T,M0M | BARTTER SYNDROME, ANTENATAL, TYPE 2 |
| KCNJ1 | OMIM: 600359 | 11 | 128214765 | G:A:G; A0V,A0A | BARTTER SYNDROME, ANTENATAL, TYPE 2 |
| CFTR | OMIM: 602421 | 7 | 116936330 | G:A:G; W0*,W0W | CYSTIC FIBROSIS |
| CFTR | OMIM: 602421 | 7 | 116936430 | G:A:G; G0R,G0G | CYSTIC FIBROSIS |
| CFTR | OMIM: 602421 | 7 | 117019585 | A:T:A;K0*, K0K | CYSTIC FIBROSIS |
| CFTR | OMIM: 602421 | 7 | 117069856 | G:A:G; W0*,W0W | CYSTIC FIBROSIS |
| CFTR | OMIM: 602421 | 7 | 117038927 | C:T:C; R0Y,R0R | CYSTIC FIBROSIS |
| CFTR | OMIM: 602421 | 7 | 116976094 | G:T:G; G0V,G0G | CYSTIC FIBROSIS |
| SLC2A2 | OMIM: 138160 | 3 | 172205872 | G:A:G; Q0*,Q0Q | FANCONI-BICKEL SYNDROME |
| SLC2A2 | OMIM: 138160 | 3 | 172207654 | C:T:C;V0I, V0V | DIABETES MELLITUS, NONINSULIN-DEPENDENT |
| SLC2A2 | OMIM: 138160 | 3 | 172205830 | G:A:G; R0*,R0R | FANCONI-BICKEL SYNDROME |
| SLC2A2 | OMIM: 138160 | 3 | 172198782 | A:T:A; V0E,V0V | FANCONI-BICKEL SYNDROME |
| SLC2A2 | OMIM: 138160 | 3 | 172199625 | G:A:G; R0*,R0R | FANCONI-BICKEL SYNDROME |
| SLC2A2 | OMIM: 138160 | 3 | 172198800 | G:A:G; P0L,P0P | FANCONI-BICKEL SYNDROME |
| NF1 | OMIM: 162200 | 17 | 26681603 | G:T:G;E0*, E0E | NEUROFIBROMATOSIS, TYPE I |
| NF1 | OMIM: 162200 | 17 | 26586774 | T:C:T;L0P, L0L | NEUROFIBROMATOSIS, TYPE I |
| NF1 | OMIM: 162200 | 17 | 26612954 | G:A:G; W0*,W0W | NEUROFIBROMATOSIS, TYPE I |
| NF1 | OMIM: 162200 | 17 | 26581517 | T:G:T; M0R,M0M | NEUROFIBROMATOSIS, TYPE I |
| NF1 | OMIM: 162200 | 17 | 26609550 | A:T:A; R0S,R0R | NEUROFIBROMATOSIS, TYPE I |

(continued)

| Gene/ Locus | MIM Number | Ch | Position | Variant Genotype | Phenotype |
|---|---|---|---|---|---|
| NF1 | OMIM: 162200 | 17 | 26600174 | C:T:C;Q0*, Q0Q | NEUROFIBROMATOSIS, TYPE I |
| MCCC2 | OMIM: 609014 | 5 | 70934169 | G:A:G; R0Q,R0R | 3-@METHYLCROTONYL-CoA CARBOXYLASE 2 DEFICIENCY |
| MCCC2 | OMIM: 609014 | 5 | 70966560 | G:T:G; D0Y,D0D | 3-@METHYLCROTONYL-CoA CARBOXYLASE 2 DEFICIENCY |
| MCCC2 | OMIM: 609014 | 5 | 70935996 | A:G:A; H0R,H0H | 3-@METHYLCROTONYL-CoA CARBOXYLASE 2 DEFICIENCY |
| MCCC2 | OMIM: 609014 | 5 | 70931255 | G:C:G; E0Q,E0E | 3-@METHYLCROTONYL-CoA CARBOXYLASE 2 DEFICIENCY |
| MCCC2 | OMIM: 609014 | 5 | 70966759 | C:G:C; P0R,P0P | 3-@METHYLCROTONYL-CoA CARBOXYLASE 2 DEFICIENCY |
| MCCC2 | OMIM: 609014 | 5 | 70934204 | T:C:T; C0R,C0C | 3-@METHYLCROTONYL-CoA CARBOXYLASE 2 DEFICIENCY |
| RPS6KA3 | OMIM: 300075 | X | 20137346 | C:A:C; G0V,G0G | COFFIN-LOWRY SYNDROME |
| RPS6KA3 | OMIM: 300075 | X | 20121553 | A:T:A;I0K, I0I | COFFIN-LOWRY SYNDROME |
| RPS6KA3 | OMIM: 300075 | X | 20114377 | A:G:A; F0S,F0F | COFFIN-LOWRY SYNDROME |
| RPS6KA3 | OMIM: 300075 | X | 20115962 | A:C:A; S0A,S0S | COFFIN-LOWRY SYNDROME |
| RPS6KA3 | OMIM: 300075 | X | 20103283 | G:A:G; R0W,R0R | MENTAL RETARDATION, X-LINKED 19 |
| RPS6KA3 | OMIM: 300075 | X | 20123170 | G:A:G; R0W,R0R | COFFIN-LOWRY SYNDROME |
| PRODH | OMIM: 606810 | 22 | 17285893 | C:A:C; A0S,A0A | SCHIZOPHRENIA, SUSCEPTIBILITY TO, 4, INCLUDED |
| PRODH | OMIM: 606810 | 22 | 17281004 | C:T:C; R0Q,R0R | SCHIZOPHRENIA, SUSCEPTIBILITY TO, 4, INCLUDED |
| PRODH | OMIM: 606810 | 22 | 17289902 | A:T:A; L0M,L0L | SCHIZOPHRENIA, SUSCEPTIBILITY TO, 4, INCLUDED |
| PRODH | OMIM: 606810 | 22 | 17285934 | A:G:A; L0P,L0L | SCHIZOPHRENIA, SUSCEPTIBILITY TO, 4, INCLUDED |
| PRODH | OMIM: 606810 | 22 | 17285859 | G:A:G; T0M,T0T | SCHIZOPHRENIA, SUSCEPTIBILITY TO, 4, INCLUDED |
| PRODH | OMIM: 606810 | 22 | 17285899 | G:A:G; R0C,R0R | SCHIZOPHRENIA, SUSCEPTIBILITY TO, 4, INCLUDED |
| COL4A5 | OMIM: 303630 | X | 107795465 | G:A:G; G0D,G0G | ALPORT SYNDROME, X-LINKED |
| COL4A5 | OMIM: 303630 | X | 107726928 | G:T:G; G0C,G0G | ALPORT SYNDROME, X-LINKED |
| COL4A5 | OMIM: 303630 | X | 107712815 | G:C:G; G0R,G0G | ALPORT SYNDROME, X-LINKED |

(continued)

| Gene/ Locus | MIM Number | Ch | Position | Variant Genotype | Phenotype |
|---|---|---|---|---|---|
| COL4A5 | OMIM: 303630 | X | 107825295 | T:G:T; L0R,L0L | ALPORT SYNDROME, X-LINKED |
| COL4A5 | OMIM: 303630 | X | 107826236 | G:A:G; R0Q,R0R | ALPORT SYNDROME, X-LINKED |
| COL4A5 | OMIM: 303630 | X | 107710608 | G:T:G; G0V,G0G | ALPORT SYNDROME, X-LINKED |
| TRIOBP | OMIM: 609761 | 22 | 36485161 | C:T:C;Q0*, Q0Q | DEAFNESS, AUTOSOMAL RECESSIVE 28 |
| TRIOBP | OMIM: 609761 | 22 | 36497633 | C:T:C;Q0*, Q0Q | DEAFNESS, AUTOSOMAL RECESSIVE 28 |
| TRIOBP | OMIM: 609761 | 22 | 36449398 | C:T:C;Q0*, Q0Q | DEAFNESS, AUTOSOMAL RECESSIVE 28 |
| TRIOBP | OMIM: 609761 | 22 | 36451858 | C:T:C;R0*, R0R | DEAFNESS, AUTOSOMAL RECESSIVE 28 |
| TRIOBP | OMIM: 609761 | 22 | 36450871 | C:T:C;R0*, R0R | DEAFNESS, AUTOSOMAL RECESSIVE 28 |
| TRIOBP | OMIM: 609761 | 22 | 36451711 | C:T:C;R0*, R0R | DEAFNESS, AUTOSOMAL RECESSIVE 28 |
| ANG | OMIM: 105850 | 14 | 20231752 | C:G:C; C0W,C0C | AMYOTROPHIC LATERAL SCLEROSIS 9 |
| ANG | OMIM: 105850 | 14 | 20231972 | G:A:G;V0I, V0V | AMYOTROPHIC LATERAL SCLEROSIS 9 |
| ANG | OMIM: 105850 | 14 | 20231685 | A:T:A; K0V,K0K | AMYOTROPHIC LATERAL SCLEROSIS 9 |
| ANG | OMIM: 105850 | 14 | 20231727 | G:A:G; R0K,R0R | AMYOTROPHIC LATERAL SCLEROSIS 9 |
| ANG | OMIM: 105850 | 14 | 20231718 | G:A:G; S0N,S0S | AMYOTROPHIC LATERAL SCLEROSIS 9 |
| ANG | OMIM: 105850 | 14 | 20231771 | A:G:A;I0V, I0I | AMYOTROPHIC LATERAL SCLEROSIS 9 |
| DKC1 | OMIM: 300126 | X | 153646934 | T:G:T; F0V,F0F | DYSKERATOSIS CONGENITA, X-LINKED |
| DKC1 | OMIM: 300126 | X | 153656120 | G:A:G; G0E,G0G | DYSKERATOSIS CONGENITA, X-LINKED |
| DKC1 | OMIM: 300126 | X | 153646919 | C:A:C; Q0K,Q0Q | DYSKERATOSIS CONGENITA, X-LINKED |
| DKC1 | OMIM: 300126 | X | 153647782 | A:G:A; SOG,SOS | HOYERAAL-HREIDARSSON SYNDROME |
| DKC1 | OMIM: 300126 | X | 153646974 | C:T:C; T0M,T0T | DYSKERATOSIS CONGENITA, X-LINKED, INCLUDED |
| DKC1 | OMIM: 300126 | X | 153654621 | C:T:C; A0V,A0A | HOYERAAL-HREIDARSSON SYNDROME, INCLUDED |
| FSHR | OMIM: 136435 | 2 | 49043425 | C:T:C; S0N,S0S | OVARIAN HYPERSTIMULATION SYNDROME, MODIFIER OF SEVERITY OF, INCLUDED |

(continued)

| Gene/ Locus | MIM Number | Ch | Position | Variant Genotype | Phenotype |
|---|---|---|---|---|---|
| FSHR | OMIM: 136435 | 2 | 49071272 | G:T:G; S0Y,S0S | OVARIAN HYPERSTIMULATION SYNDROME |
| FSHR | OMIM: 136435 | 2 | 49044118 | G:A:G; T0V,T0T | OVARIAN HYPERSTIMULATION SYNDROME |
| FSHR | OMIM: 136435 | 2 | 49044119 | T:C:T;T0V, T0T | OVARIAN HYPERSTIMULATION SYNDROME |
| FSHR | OMIM: 136435 | 2 | 49043747 | G:A:G; R0C,R0R | OVARIAN DYSGENESIS 1 |
| FSHR | OMIM: 136435 | 2 | 49044545 | C:T:C; A0T,A0A | OVARIAN RESPONSE TO FSH STIMULATION |
| BCS1L | OMIM: 603647 | 2 | 219234057 | G:C:G; G0R,G0G | BJORNSTAD SYNDROME WITH MILD MITOCHONDRIAL COMPLEX III DEFICIENCY |
| BCS1L | OMIM: 603647 | 2 | 219234815 | C:T:C; R0C,R0R | MITOCHONDRIAL COMPLEX III DEFICIENCY, INCLUDED |
| BCS1L | OMIM: 603647 | 2 | 219234729 | G:C:G; R0P,R0R | MITOCHONDRIAL COMPLEX III DEFICIENCY |
| BCS1L | OMIM: 603647 | 2 | 219234186 | A:G:A; S0G,S0S | GRACILE SYNDROME |
| BCS1L | OMIM: 603647 | 2 | 219234250 | C:T:C; P0L,P0P | LEIGH SYNDROME DUE TO MITOCHONDRIAL COMPLEX III DEFICIENCY |
| BCS1L | OMIM: 603647 | 2 | 219234813 | G:A:G; R0H,R0R | BJORNSTAD SYNDROME |
| SMPD1 | OMIM: 607608 | 11 | 6371473 | C:A:C; S0R,S0S | NIEMANN-PICK DISEASE, TYPE B |
| SMPD1 | OMIM: 607608 | 11 | 6371486 | C:T:C;R0*, R0R | NIEMANN-PICK DISEASE, TYPE B |
| SMPD1 | OMIM: 607608 | 11 | 6371107 | T:G:T; W0G, W0W | NIEMANN-PICK DISEASE, INTERMEDIATE, PROTRACTED NEUROVISCERAL |
| SMPD1 | OMIM: 607608 | 11 | 6369659 | T:A:T;L0*, L0L | NIEMANN-PICK DISEASE, TYPE A |
| SMPD1 | OMIM: 607608 | 11 | 6369782 | T:C:T;L0P, L0L | NIEMANN-PICK DISEASE, TYPE A |
| SMPD1 | OMIM: 607608 | 11 | 6371426 | C:T:C; H0Y,H0H | NIEMANN-PICK DISEASE, TYPE B |
| PI | OMIM: 107400 | 14 | 93914596 | T:G:T; E0D,E0E | PI M3 |
| PI | OMIM: 107400 | 14 | 93917139 | G:A:G; R0C,R0R | PI F |
| PI | OMIM: 107400 | 14 | 93919002 | G:A:G; T0M,T0T | PI Z(BRISTOL) |
| PI | OMIM: 107400 | 14 | 93914703 | C:T:C; D0N,D0D | PI P(ST. ALBANS) |

(continued)

| Gene/ Locus | MIM Number | Ch | Position | Variant Genotype | Phenotype |
|---|---|---|---|---|---|
| PI | OMIM: 107400 | 14 | 93919141 | G:A:G; R0C,R0R | PI I |
| PI | OMIM: 107400 | 14 | 93914618 | G:A:G; P0L,P0P | PI M(HEERLEN) |
| CNGA3 | OMIM: 600053 | 2 | 98379650 | G:A:G; V0M,V0V | ROD MONOCHROMACY |
| CNGA3 | OMIM: 600053 | 2 | 98378894 | C:T:C; R0C,R0R | ROD MONOCHROMACY |
| CNGA3 | OMIM: 600053 | 2 | 98378937 | C:G:C; T0R,T0T | ROD MONOCHROMACY |
| CNGA3 | OMIM: 600053 | 2 | 98372591 | C:T:C; P0L,P0P | ROD MONOCHROMACY |
| CNGA3 | OMIM: 600053 | 2 | 98378913 | G:A:G; R0*,R0R | ROD MONOCHROMACY |
| CNGA3 | OMIM: 600053 | 2 | 98378912 | C:T:C;R0*, R0R | ROD MONOCHROMACY |
| PRNP | OMIM: 176640 | 20 | 4628404 | G:A:G;V0I, V0V | CREUTZFELDT-JAKOB DISEASE |
| PRNP | OMIM: 176640 | 20 | 4628179 | C:A:C;P0I, P0P | SPONGIFORM ENCEPHALOPATHY WITH NEUROPSYCHIATRIC FEATURES |
| PRNP | OMIM: 176640 | 20 | 4628516 | A:G:A; Q0R,Q0Q | GERSTMANN-STRAUSSLER DISEASE |
| | OMIM: 176640 | 20 | 4628494 | G:A:G;V0I, V0V | |
| PRNP | OMIM: 176640 | 20 | 4628378 | A:G:A; N0S,N0N | INCLUDED |
| PRNP | OMIM: 176640 | 20 | 4628180 | C:T:C;P0I, P0P | GERSTMANN-STRAUSSLER DISEASE |
| FGB | OMIM: 134830 | 4 | 155706428 | G:T:G; G0C,G0G | FIBRINOGEN ISE |
| FGB | OMIM: 134830 | 4 | 155708309 | T:A:T;L0Q, L0L | HYPOFIBRINOGENEMIA, CONGENITAL |
| FGB | OMIM: 134830 | 4 | 155706434 | C:T:C;R0*, R0R | HYPOFIBRINOGENEMIA, CONGENITAL |
| FGB | OMIM: 134830 | 4 | 155706425 | C:T:C; R0C,R0R | FIBRINOGEN NIJMEGEN |
| FGB | OMIM: 134830 | 4 | 155710250 | G:A:G; A0T,A0A | FIBRINOGEN PONTOISE 2 |
| FGB | OMIM: 134830 | 4 | 155711209 | G:A:G; R0K,R0R | FIBRINOGEN BALTIMORE 2 |
| GNAS | OMIM: 139320 | 20 | 56913893 | C:T:C; R0C,R0R | PSEUDOHYPOPARATHYROIDISM, TYPE IA |
| GNAS | OMIM: 139320 | 20 | 56917853 | G:T:G; Q0R,Q0Q | PITUITARY ADENOMA, ACTH-SECRETING, SOMATIC |

(continued)

| Gene/ Locus | MIM Number | Ch | Position | Variant Genotype | Phenotype |
|---|---|---|---|---|---|
| GNAS | OMIM: 139320 | 20 | 56917852 | A:G:A; Q0R,Q0Q | PITUITARY ADENOMA, ACTH-SECRETING, SOMATIC, INCLUDED |
| GNAS | OMIM: 139320 | 20 | 56912019 | T:C:T;L0P, L0L | PSEUDOHYPOPARATHYROIDISM, TYPE IA |
| GNAS | OMIM: 139320 | 20 | 56918187 | C:T:C; R0W,R0R | PSEUDOPSEUDOHYPOPARATHYROIDIS M |
| GNAS | OMIM: 139320 | 20 | 56917815 | C:A:C; R0N,R0R | ACTH-INDEPENDENT MACRONODULAR ADRENAL HYPERPLASIA, SOMATIC, INCLUDED |
| CTNS | OMIM: 606272 | 17 | 3510017 | C:G:C; N0K,N0N | CYSTINOSIS, LATE-ONSET JUVENILE OR ADOLESCENT NEPHROPATHIC TYPE |
| CTNS | OMIM: 606272 | 17 | 3506574 | G:A:G; G0D,G0G | CYSTINOSIS, NEPHROPATHIC |
| CTNS | OMIM: 606272 | 17 | 3505098 | G:T:G; G0*,G0G | CYSTINOSIS, NEPHROPATHIC |
| CTNS | OMIM: 606272 | 17 | 3497549 | G:A:G;V0I, V0V | CYSTINOSIS, ATYPICAL NEPHROPATHIC |
| CTNS | OMIM: 606272 | 17 | 3506746 | G:A:G; G0R,G0G | CYSTINOSIS, OCULAR NONNEPHROPATHIC |
| CTNS | OMIM: 606272 | 17 | 3510323 | G:A:G; G0R,G0G | CYSTINOSIS, NEPHROPATHIC |
| FGFR3 | OMIM: 134934 | 4 | 1777689 | G:T:G; K0V,K0K | HYPOCHONDROPLASIA |
| FGFR3 | OMIM: 134934 | 4 | 1775897 | A:G:A; Y0C,Y0Y | THANATOPHORIC DYSPLASIA, TYPE I |
| FGFR3 | OMIM: 134934 | 4 | 1777168 | A:C:A; N0T,N0N | HYPOCHONDROPLASIA |
| FGFR3 | OMIM: 134934 | 4 | 1778787 | A:C:A; K0Q,K0K | THANATOPHORIC DYSPLASIA, TYPE I |
| FGFR3 | OMIM: 134934 | 4 | 1773362 | C:T:C; R0C,R0R | NEVUS, EPIDERMAL, INCLUDED |
| FGFR3 | OMIM: 134934 | 4 | 1773366 | C:G:C; S0C,S0S | BLADDER CANCER, SOMATIC, INCLUDED |
| FOXL2 | OMIM: 605597 | 3 | 140147600 | G:A:G; Q0*,Q0Q | BLEPHAROPHIMOSIS, PTOSIS, AND EPICANTHUS INVERSUS, TYPE I |
| FOXL2 | OMIM: 605597 | 3 | 140147669 | G:A:G; Q0*,Q0Q | BLEPHAROPHIMOSIS, PTOSIS, AND EPICANTHUS INVERSUS |
| FOXL2 | OMIM: 605597 | 3 | 140147483 | A:T:A; Y0N,Y0Y | PREMATURE OVARIAN FAILURE 3 |
| FOXL2 | OMIM: 605597 | 3 | 140147433 | G:C:G; Y0*,Y0Y | BLEPHAROPHIMOSIS, PTOSIS, AND EPICANTHUS INVERSUS, TYPE II, INCLUDED |
| FOXL2 | OMIM: 605597 | 3 | 140148098 | G:A:G; Q0*,Q0Q | BLEPHAROPHIMOSIS, PTOSIS, AND EPICANTHUS INVERSUS, TYPE I |
| FOXL2 | OMIM: 605597 | 3 | 140148004 | A:C:A;I0S, I0I | BLEPHAROPHIMOSIS, PTOSIS, AND EPICANTHUS INVERSUS, TYPE I |

(continued)

| Gene/ Locus | MIM Number | Ch | Position | Variant Genotype | Phenotype |
|---|---|---|---|---|---|
| RYR1 | OMIM: 180901 | 19 | 43762920 | G:A:G; R0H,R0R | NEUROMUSCULAR DISEASE, CONGENITAL, WITH UNIFORM TYPE 1 FIBER, INCLUDED |
| RYR1 | OMIM: 180901 | 19 | 43767469 | T:C:T;I0T, I0I | MALIGNANT HYPERTHERMIA, SUSCEPTIBILITY TO, 1, INCLUDED |
| RYR1 | OMIM: 180901 | 19 | 43626691 | C:T:C; R0C,R0R | CENTRAL CORE DISEASE, INCLUDED |
| RYR1 | OMIM: 180901 | 19 | 43762883 | G:A:G;V0I, V0V | MINICORE MYOPATHY WITH EXTERNAL OPHTHALMOPLEGIA, INCLUDED |
| RYR1 | OMIM: 180901 | 19 | 43631192 | G:A:G; G0R,G0G | MALIGNANT HYPERTHERMIA, SUSCEPTIBILITY TO, 1 |
| RYR1 | OMIM: 180901 | 19 | 43629190 | G:A:G; G0R,G0G | MALIGNANT HYPERTHERMIA, SUSCEPTIBILITY TO, 1 |
| DNM2 | OMIM: 602378 | 19 | 10765505 | G:A:G; E0K,E0E | MYOPATHY, CENTRONUCLEAR, AUTOSOMAL DOMINANT |
| DNM2 | OMIM: 602378 | 19 | 10791668 | A:G:A; K0E,K0K | CHARCOT-MARIE-TOOTH DISEASE, DOMINANT INTERMEDIATE B, WITH NEUTROPENIA |
| DNM2 | OMIM: 602378 | 19 | 10770219 | C:T:C; R0W,R0R | MYOPATHY, CENTRONUCLEAR, AUTOSOMAL DOMINANT |
| DNM2 | OMIM: 602378 | 19 | 10791693 | T:A:T;L0H, L0L | CHARCOT-MARIE-TOOTH DISEASE, AXONAL, TYPE 2M |
| DNM2 | OMIM: 602378 | 19 | 10783991 | G:T:G; G0C,G0G | CHARCOT-MARIE-TOOTH DISEASE, AXONAL, TYPE 2M |
| DNM2 | OMIM: 602378 | 19 | 10795538 | C:G:C; S0W,S0S | MYOPATHY, CENTRONUCLEAR |
| UGT1A1 | OMIM: 191740 | 2 | 234340546 | A:G:A; Q0W,Q0Q | CRIGLER-NAJJAR SYNDROME, TYPE II |
| UGT1A1 | OMIM: 191740 | 2 | 234341258 | C:T:C;R0*, R0R | GILBERT SYNDROME, INCLUDED |
| UGT1A1 | OMIM: 191740 | 2 | 234341307 | A:G:A; Q0R,Q0Q | CRIGLER-NAJJAR SYNDROME, TYPE I |
| UGT1A1 | OMIM: 191740 | 2 | 234341718 | A:G:A; N0D,N0N | GILBERT SYNDROME, INCLUDED |
| UGT1A1 | OMIM: 191740 | 2 | 234345798 | T:G:T; Y0D,Y0Y | CRIGLER-NAJJAR SYNDROME, TYPE II, INCLUDED |
| UGT1A1 | OMIM: 191740 | 2 | 234340545 | C:T:C; Q0W,Q0Q | CRIGLER-NAJJAR SYNDROME, TYPE I |
| ING1 | OMIM: 601566 | 13 | 110170084 | G:C:G; C0T,C0C | SQUAMOUS CELL CARCINOMA, HEAD AND NECK |
| ING1 | OMIM: 601566 | 13 | 110170087 | A:G:A; N0S,N0N | SQUAMOUS CELL CARCINOMA, HEAD AND NECK |
| ING1 | OMIM: 601566 | 13 | 110170083 | T:A:T;C0T, C0C | SQUAMOUS CELL CARCINOMA, HEAD AND NECK |
| ING1 | OMIM: 601566 | 13 | 110170159 | G:C:G; C0T,C0C | SQUAMOUS CELL CARCINOMA, HEAD AND NECK |

(continued)

| Gene/ Locus | MIM Number | Ch | Position | Variant Genotype | Phenotype |
|---|---|---|---|---|---|
| ING1 | OMIM: 601566 | 13 | 110170158 | T:A:T;C0T, C0C | SQUAMOUS CELL CARCINOMA, HEAD AND NECK |
| ING1 | OMIM: 601566 | 13 | 110170015 | C:A:C; A0D,A0A | SQUAMOUS CELL CARCINOMA, HEAD AND NECK |
| PDHA1 | OMIM: 300502 | X | 19285703 | A:C:A; M0L,M0M | PYRUVATE DEHYDROGENASE EI-ALPHA DEFICIENCY |
| PDHA1 | OMIM: 300502 | X | 19283738 | A:C:A; DOA,DOD | LEIGH SYNDROME, X-LINKED |
| PDHA1 | OMIM: 300502 | X | 19283752 | C:G:C; R0G,R0R | LEIGH SYNDROME, X-LINKED |
| PDHA1 | OMIM: 300502 | X | 19285722 | G:A:G; R0H,R0R | PYRUVATE DEHYDROGENASE EI-ALPHA DEFICIENCY |
| PDHA1 | OMIM: 300502 | X | 19283432 | A:C:A;L0F, L0L | PYRUVATE DEHYDROGENASE EI-ALPHA DEFICIENCY |
| PDHA1 | OMIM: 300502 | X | 19283511 | T:A:T; Y0N,Y0Y | PYRUVATE DEHYDROGENASE EI-ALPHA DEFICIENCY |
| PARK2 | OMIM: 602544 | 6 | 162314339 | G:A:G; TOM,TOT | PARKINSON DISEASE 2, AUTOSOMAL RECESSIVE JUVENILE |
| PARK2 | OMIM: 602544 | 6 | 162314423 | C:T:C; C0Y,C0C | PARKINSON DISEASE 2, AUTOSOMAL RECESSIVE JUVENILE |
| PARK2 | OMIM: 602544 | 6 | 161691161 | C:T:C; W0*,W0W | PARKINSON DISEASE 2, AUTOSOMAL RECESSIVE JUVENILE |
| PARK2 | OMIM: 602544 | 6 | 162314425 | T:A:T; K0N,K0K | PARKINSON DISEASE 2, AUTOSOMAL RECESSIVE JUVENILE |
| PARK2 | OMIM: 602544 | 6 | 162126842 | G:A:G; ROW,ROR | PARKINSON DISEASE 2, AUTOSOMAL RECESSIVE JUVENILE |
| PARK2 | OMIM: 602544 | 6 | 161910379 | G:A:G; Q0*,Q0Q | PARKINSON DISEASE 2, AUTOSOMAL RECESSIVE JUVENILE |
| ATP1A2 | OMIM: 182340 | 1 | 158366697 | G:A:G; R0H,R0R | MIGRAINE, FAMILIAL BASILAR |
| ATP1A2 | OMIM: 182340 | 1 | 158365181 | C:A:C; T0N,T0T | ALTERNATING HEMIPLEGIA OF CHILDHOOD |
| ATP1A2 | OMIM: 182340 | 1 | 158365421 | C:T:C; T0M,T0T | MIGRAINE, FAMILIAL HEMIPLEGIC, 2 |
| ATP1A2 | OMIM: 182340 | 1 | 158373079 | T:C:T; W0R,W0W | MIGRAINE, FAMILIAL HEMIPLEGIC, 2 |
| ATP1A2 | OMIM: 182340 | 1 | 158365175 | C:T:C; T0M,T0T | MIGRAINE, FAMILIAL HEMIPLEGIC, 2 |
| ATP1A2 | OMIM: 182340 | 1 | 158371884 | G:A:G; D0N,D0D | MIGRAINE, FAMILIAL HEMIPLEGIC, 2 |
| FOXC1 | OMIM: 601090 | 6 | 1556014 | T:C:T;F0S, F0F | PETERS ANOMALY, INCLUDED |
| FOXC1 | OMIM: 601090 | 6 | 1555746 | C:T:C;Q0*, Q0Q | AXENFELD-RIEGER SYNDROME, TYPE 3 |

(continued)

| Gene/ Locus | MIM Number | Ch | Position | Variant Genotype | Phenotype |
|---|---|---|---|---|---|
| FOXC1 | OMIM: 601090 | 6 | 1555924 | G:C:G; S0T,S0S | AXENFELD-RIEGER SYNDROME, TYPE 3 |
| FOXC1 | OMIM: 601090 | 6 | 1555940 | C:G:C; I0M,I0I | AXENFELD-RIEGER ANOMALY |
| FOXC1 | OMIM: 601090 | 6 | 1556071 | C:T:C; S0L,S0S | RIEGER ANOMALY |
| FOXC1 | OMIM: 601090 | 6 | 1556057 | C:G:C; I0M,I0I | AXENFELD ANOMALY |
| ALB | OMIM: 103600 | 4 | 74498190 | G:A:G; E0K,E0E | ALBUMIN ROMA |
| ALB | OMIM: 103600 | 4 | 74499722 | G:C:G; D0L,D0D | ALBUMIN PARKLANDS |
| ALB | OMIM: 103600 | 4 | 74504215 | G:A:G; E0K,E0E | ALBUMIN VERONA B |
| ALB | OMIM: 103600 | 4 | 74504113 | A:G:A; K0E,K0K | ALBUMIN CASTEL DI SANGRO |
| ALB | OMIM: 103600 | 4 | 74498168 | G:T:G; K0N,K0K | ALBUMIN COOPERSTOWN |
| ALB | OMIM: 103600 | 4 | 74499723 | A:T:A;D0L, D0D | ALBUMIN IOWA CITY 1 |
| CYB5R3 | OMIM: 250800 | 22 | 41349753 | T:C:T; D0G,D0D | METHEMOGLOBINEMIA DUE TO DEFICIENCY OF METHEMOGLOBIN REDUCTASE |
| CYB5R3 | OMIM: 250800 | 22 | 41354119 | A:G:A; L0P,L0L | METHEMOGLOBINEMIA DUE TO DEFICIENCY OF METHEMOGLOBIN REDUCTASE |
| CYB5R3 | OMIM: 250800 | 22 | 41357336 | A:G:A; L0P,L0L | METHEMOGLOBINEMIA DUE TO DEFICIENCY OF METHEMOGLOBIN REDUCTASE |
| CYB5R3 | OMIM: 250800 | 22 | 41354183 | A:G:A; S0P,S0S | METHEMOGLOBINEMIA DUE TO DEFICIENCY OF METHEMOGLOBIN REDUCTASE |
| CYB5R3 | OMIM: 250800 | 22 | 41353277 | A:G:A; C0H,C0C | METHEMOGLOBINEMIA DUE TO DEFICIENCY OF METHEMOGLOBIN REDUCTASE |
| CYB5R3 | OMIM: 250800 | 22 | 41353276 | C:T:C; C0H,C0C | METHEMOGLOBINEMIA DUE TO DEFICIENCY OF METHEMOGLOBIN REDUCTASE |
| CTSC | OMIM: 602365 | 11 | 87666927 | C:G:C; W0C,W0W | PAPILLON-LEFEVRE SYNDROME |
| CTSC | OMIM: 602365 | 11 | 87673348 | T:A:T;Q0L, Q0Q | PAPILLON-LEFEVRE SYNDROME |
| CTSC | OMIM: 602365 | 11 | 87666979 | T:C:T; Y0C,Y0Y | PERIODONTITIS, AGGRESSIVE, 1 |
| CTSC | OMIM: 602365 | 11 | 87667174 | T:C:T; Y0C,Y0Y | PERIODONTITIS, AGGRESSIVE, 1 |
| CTSC | OMIM: 602365 | 11 | 87668981 | T:C:T; Q0W,Q0Q | HAIM-MUNK SYNDROME |
| CTSC | OMIM: 602365 | 11 | 87667313 | C:T:C; G0S,G0G | PAPILLON-LEFEVRE SYNDROME |

(continued)

| Gene/ Locus | MIM Number | Ch | Position | Variant Genotype | Phenotype |
|---|---|---|---|---|---|
| CPOX | OMIM: 121300 | 3 | 99787156 | G:A:G; R0W,R0R | COPROPORPHYRIA, HEREDITARY;HCP |
| CPOX | OMIM: 121300 | 3 | 99794954 | G:A:G; Q0*,Q0Q | COPROPORPHYRIA, HEREDITARY;HCP |
| CPOX | OMIM: 121300 | 3 | 99782243 | G:A:G; R0C,R0R | COPROPORPHYRIA, HEREDITARY;HCP |
| CPOX | OMIM: 121300 | 3 | 99790365 | C:G:C; G0R,G0G | COPROPORPHYRIA, HEREDITARY;HCP |
| CPOX | OMIM: 121300 | 3 | 99792623 | G:A:G; S0F,S0S | COPROPORPHYRIA, HEREDITARY;HCP |
| CPOX | OMIM: 121300 | 3 | 99790317 | G:C:G; H0D,H0H | COPROPORPHYRIA, HEREDITARY;HCP |
| SPTA1 | OMIM: 182860 | 1 | 156921704 | G:A:G; R0F,R0R | PYROPOIKILOCYTOSIS, HEREDITARY, INCLUDED |
| SPTA1 | OMIM: 182860 | 1 | 156914846 | A:G:A; S0P,S0S | ELLIPTOCYTOSIS 2 |
| SPTA1 | OMIM: 182860 | 1 | 156921665 | G:A:G; R0W,R0R | ELLIPTOCYTOSIS 2 |
| SPTA1 | OMIM: 182860 | 1 | 156899207 | G:T:G; D0E,D0D | ELLIPTOCYTOSIS 2 |
| SPTA1 | OMIM: 182860 | 1 | 156921703 | C:A:C; R0F,R0R | ELLIPTOCYTOSIS 2 |
| SPTA1 | OMIM: 182860 | 1 | 156917055 | A:G:A; L0P,L0L | ELLIPTOCYTOSIS 2, INCLUDED |
| PAX8 | OMIM: 167415 | 2 | 113720832 | T:C:T; S0G,S0S | HYPOTHYROIDISM, CONGENITAL, NONGOITROUS, 2 |
| PAX8 | OMIM: 167415 | 2 | 113718541 | G:A:G; R0*,R0R | HYPOTHYROIDISM, CONGENITAL, NONGOITROUS, 2 |
| PAX8 | OMIM: 167415 | 2 | 113720849 | G:A:G; S0F,S0S | HYPOTHYROIDISM, CONGENITAL, NONGOITROUS, 2 |
| PAX8 | OMIM: 167415 | 2 | 113720900 | C:T:C; R0H,R0R | HYPOTHYROIDISM, CONGENITAL, NONGOITROUS, 2 |
| PAX8 | OMIM: 167415 | 2 | 113709541 | C:T:C; L0M,F0L , L0L | PAX8 POLYMORPHISM |
| PAX8 | OMIM: 167415 | 2 | 113720807 | A:C:A; L0R,L0L | HYPOTHYROIDISM, CONGENITAL, NONGOITROUS, 2 |
| TYMP | OMIM: 131222 | 22 | 49314874 | C:T:C; R0Q,R0R | MITOCHONDRIAL NEUROGASTROINTESTINAL ENCEPHALOMYOPATHY SYNDROME |
| TYMP | OMIM: 131222 | 22 | 49313866 | C:T:C; G0S,G0G | MITOCHONDRIAL NEUROGASTROINTESTINAL ENCEPHALOMYOPATHY SYNDROME |
| TYMP | OMIM: 131222 | 22 | 49312907 | C:T:C; V0M,V0V | MITOCHONDRIAL NEUROGASTROINTESTINAL ENCEPHALOMYOPATHY SYNDROME |

(continued)

| Gene/ Locus | MIM Number | Ch | Position | Variant Genotype | Phenotype |
|---|---|---|---|---|---|
| TYMP | OMIM: 131222 | 22 | 49312924 | C:G:C; R0T,R0R | MITOCHONDRIAL NEUROGASTROINTESTINAL ENCEPHALOMYOPATHY SYNDROME |
| TYMP | OMIM: 131222 | 22 | 49311945 | A:G:A; L0P,L0L | MITOCHONDRIAL NEUROGASTROINTESTINAL ENCEPHALOMYOPATHY SYNDROME |
| TYMP | OMIM: 131222 | 22 | 49311769 | C:G:C; G0R,G0G | MITOCHONDRIAL NEUROGASTROINTESTINAL ENCEPHALOMYOPATHY SYNDROME |
| FLNB | OMIM: 603381 | 3 | 58109538 | C:T:C;R0*, R0R | SPONDYLOCARPOTARSAL SYNOSTOSIS SYNDROME |
| FLNB | OMIM: 603381 | 3 | 58039546 | A:G:A; M0V,M0M | ATELOSTEOGENESIS, TYPE III, INCLUDED |
| FLNB | OMIM: 603381 | 3 | 58099258 | G:A:G; G0S,G0G | LARSEN SYNDROME, AUTOSOMAL DOMINANT |
| FLNB | OMIM: 603381 | 3 | 58096830 | G:A:G; G0R,G0G | LARSEN SYNDROME, AUTOSOMAL DOMINANT |
| FLNB | OMIM: 603381 | 3 | 58042435 | G:A:G; E0K,E0E | LARSEN SYNDROME, AUTOSOMAL DOMINANT |
| FLNB | OMIM: 603381 | 3 | 58038038 | C:T:C; A0V,A0A | ATELOSTEOGENESIS, TYPE I |
| IDS | OMIM: 309900 | X | 148372433 | G:A:G; R0L,R0R | MUCOPOLYSACCHARIDOSIS TYPE II, MILD FORM |
| IDS | OMIM: 309900 | X | 148372410 | C:T:C; W0*,W0W | MUCOPOLYSACCHARIDOSIS TYPE II |
| IDS | OMIM: 309900 | X | 148372432 | C:A:C; R0L,R0R | MUCOPOLYSACCHARIDOSIS TYPE II, SEVERE FORM |
| IDS | OMIM: 309900 | X | 148372371 | C:A:C;M0I, M0M | MUCOPOLYSACCHARIDOSIS TYPE II |
| IDS | OMIM: 309900 | X | 148372369 | C:G:C; G0A,G0G | MUCOPOLYSACCHARIDOSIS TYPE II |
| IDS | OMIM: 309900 | X | 148379758 | G:A:G; S0L,S0S | MUCOPOLYSACCHARIDOSIS TYPE II |
| TP53 | OMIM: 191170 | 17 | 7518251 | A:G:A; L0P,L0L | LI-FRAUMENI SYNDROME 1 |
| TP53 | OMIM: 191170 | 17 | 7519131 | C:T:C; R0H,R0R | LI-FRAUMENI SYNDROME 1 |
| TP53 | OMIM: 191170 | 17 | 7518263 | C:T:C;R0*, R0R | LI-FRAUMENI SYNDROME 1 |
| TP53 | OMIM: 191170 | 17 | 7518273 | C:T:C; G0N,G0G | OSTEOSARCOMA |
| TP53 | OMIM: 191170 | 17 | 7518236 | A:T:A; L0Q,L0L | OSTEOSARCOMA |
| TP53 | OMIM: 191170 | 17 | 7519243 | C:G:C; A0P,A0A | LI-FRAUMENI SYNDROME 1 |
| HSD17B3 | OMIM: 605573 | 9 | 98042880 | C:T:C; C0Y,C0C | 17-@BETA HYDROXYSTEROID DEHYDROGENASE III DEFICIENCY |

(continued)

| Gene/ Locus | MIM Number | Ch | Position | Variant Genotype | Phenotype |
|---|---|---|---|---|---|
| HSD17B3 | OMIM: 605573 | 9 | 98100554 | C:T:C; A0T,A0A | 17-@BETA HYDROXYSTEROID DEHYDROGENASE III DEFICIENCY |
| HSD17B3 | OMIM: 605573 | 9 | 98053585 | T:C:T; N0S,N0N | 17-@BETA HYDROXYSTEROID DEHYDROGENASE III DEFICIENCY |
| HSD17B3 | OMIM: 605573 | 9 | 98042980 | T:C:T; M0V,M0M | 17-@BETA HYDROXYSTEROID DEHYDROGENASE III DEFICIENCY |
| HSD17B3 | OMIM: 605573 | 9 | 98057009 | C:T:C;R0*, R0R | 17-@BETA HYDROXYSTEROID DEHYDROGENASE III DEFICIENCY |
| HSD17B3 | OMIM: 605573 | 9 | 98057010 | G:A:G; R0*,R0R | 17-@BETA HYDROXYSTEROID DEHYDROGENASE III DEFICIENCY |
| ATM | OMIM: 607585 | 11 | 107603743 | C:T:C;R0*, R0R | ATAXIA-TELANGIECTASIA |
| ATM | OMIM: 607585 | 11 | 107648509 | A:G:A; M0V,M0M | B-CELL NON-HODGKIN LYMPHOMA, SOMATIC |
| ATM | OMIM: 607585 | 11 | 107603786 | C:G:C; S0C,S0S | BREAST CANCER, SUSCEPTIBILITY TO |
| ATM | OMIM: 607585 | 11 | 107706170 | C:T:C;R0*, R0R | ATAXIA-TELANGIECTASIA |
| ATM | OMIM: 607585 | 11 | 107705136 | A:G:A; E0G,E0E | MANTLE CELL LYMPHOMA |
| ATM | OMIM: 607585 | 11 | 107710925 | A:G:A; Y0C,Y0Y | ATAXIA-TELANGIECTASIA VARIANT |
| FGFR2 | OMIM: 176943 | 10 | 123269556 | T:G:T; Q0P,Q0Q | JACKSON-WEISS SYNDROME, INCLUDED |
| FGFR2 | OMIM: 176943 | 10 | 123269623 | A:G:A; S0P,S0S | GASTRIC CANCER, SOMATIC, INCLUDED |
| FGFR2 | OMIM: 176943 | 10 | 123269552 | C:A:C; W0G, W0W | CRANIOFACIAL-SKELETAL-DERMATOLOGIC DYSPLASIA, INCLUDED |
| FGFR2 | OMIM: 176943 | 10 | 123269664 | G:C:G; P0R,P0P | APERT SYNDROME |
| FGFR2 | OMIM: 176943 | 10 | 123269554 | A:C:A; W0G, W0W | CROUZON SYNDROME |
| FGFR2 | OMIM: 176943 | 10 | 123269667 | G:C:G; S0W,S0S | ENDOMETRIAL CANCER, SOMATIC, INCLUDED |
| MLC1 | OMIM: 605908 | 22 | 48865283 | C:T:C; G0E,G0G | MEGALENCEPHALIC LEUKOENCEPHALOPATHY WITH SUBCORTICAL CYSTS |
| MLC1 | OMIM: 605908 | 22 | 48849044 | G:A:G; S0L,S0S | MEGALENCEPHALIC LEUKOENCEPHALOPATHY WITH SUBCORTICAL CYSTS |
| MLC1 | OMIM: 605908 | 22 | 48860474 | G:T:G; N0R,N0N | MEGALENCEPHALIC LEUKOENCEPHALOPATHY WITH SUBCORTICAL CYSTS |
| MLC1 | OMIM: 605908 | 22 | 48860947 | G:A:G; P0S,P0P | MEGALENCEPHALIC LEUKOENCEPHALOPATHY WITH SUBCORTICAL CYSTS |

(continued)

| Gene/ Locus | MIM Number | Ch | Position | Variant Genotype | Phenotype |
|---|---|---|---|---|---|
| MLC1 | OMIM: 605908 | 22 | 48860943 | G:A:G; S0L,S0S | MEGALENCEPHALIC LEUKOENCEPHALOPATHY WITH SUBCORTICAL CYSTS |
| MLC1 | OMIM: 605908 | 22 | 48860475 | T:C:T; N0R,N0N | MEGALENCEPHALIC LEUKOENCEPHALOPATHY WITH SUBCORTICAL CYSTS |
| LDLR | OMIM: 606945 | 19 | 11087829 | G:A:G; G0D,G0G | FH GENOA |
| LDLR | OMIM: 606945 | 19 | 11092058 | G:A:G; C0Y,C0C | FH FRENCH CANADIAN 2 |
| LDLR | OMIM: 606945 | 19 | 11071962 | G:A:G; W0*,W0W | HYPERCHOLESTEROLEMIA, FAMILIAL |
| LDLR | OMIM: 606945 | 19 | 11071968 | G:C:G; C0S,C0C | HYPERCHOLESTEROLEMIA, FAMILIAL |
| LDLR | OMIM: 606945 | 19 | 11087820 | G:A:G; G0D,G0G | FH SAINT OMER |
| LDLR | OMIM: 606945 | 19 | 11085419 | G:A:G; V0M,V0V | FH KUWAIT |
| TF | OMIM: 190000 | 3 | 134959388 | A:G:A; H0R,H0H | TRANSFERRIN VARIANT CHI |
| TF | OMIM: 190000 | 3 | 134959319 | A:G:A; D0G,D0D | TRANSFERRIN VARIANT D1 |
| TF | OMIM: 190000 | 3 | 134960840 | G:A:G; E0K,E0E | ATRANSFERRINEMIA |
| TF | OMIM: 190000 | 3 | 134967910 | G:C:G; A0P,A0A | ATRANSFERRINEMIA |
| TF | OMIM: 190000 | 3 | 134978646 | A:G:A; K0E,K0K | TRANSFERRIN VARIANT Bv |
| TF | OMIM: 190000 | 3 | 134955141 | G:A:G; D0N,D0D | ATRANSFERRINEMIA |
| PAX6 | OMIM: 607108 | 11 | 31779735 | G:A:G; R0*,R0R | ANIRIDIA |
| PAX6 | OMIM: 607108 | 11 | 31772203 | G:A:G; R0*,R0R | ANIRIDIA |
| PAX6 | OMIM: 607108 | 11 | 31779851 | C:A:C; G0V,G0G | FOVEAL HYPOPLASIA AND PRESENILE CATARACT SYNDROME |
| PAX6 | OMIM: 607108 | 11 | 31780893 | G:C:G; R0G,R0R | ANIRIDIA, INCLUDED |
| PAX6 | OMIM: 607108 | 11 | 31772829 | G:A:G; R0*,R0R | ANIRIDIA |
| PAX6 | OMIM: 607108 | 11 | 31779840 | G:A:G; P0S,P0P | MORNING GLORY DISC ANOMALY |
| RAG1 | OMIM: 179615 | 11 | 36553111 | C:T:C; R0Y,R0R | SEVERE COMBINED IMMUNODEFICIENCY, B CELL-NEGATIVE, INCLUDED |
| RAG1 | OMIM: 179615 | 11 | 36554165 | A:G:A; Y0C,Y0Y | OMENN SYNDROME |

(continued)

| Gene/ Locus | MIM Number | Ch | Position | Variant Genotype | Phenotype |
|---|---|---|---|---|---|
| RAG1 | OMIM: 179615 | 11 | 36554372 | A:C:A; Q0P,Q0Q | CYTOMEGALOVIRUS INFECTION, AND AUTOIMMUNITY |
| RAG1 | OMIM: 179615 | 11 | 36553750 | G:T:G;E0*, E0E | SEVERE COMBINED IMMUNODEFICIENCY, B CELL-NEGATIVE |
| RAG1 | OMIM: 179615 | 11 | 36553640 | G:A:G; R0H,R0R | COMBINED CELLULAR AND HUMORAL IMMUNE DEFECTS WITH GRANULOMAS |
| RAG1 | OMIM: 179615 | 11 | 36553951 | C:T:C; R0W,R0R | CYTOMEGALOVIRUS INFECTION, AND AUTOIMMUNITY |
| PTEN | OMIM: 601728 | 10 | 89682858 | C:G:C; A0G,A0A | SQUAMOUS CELL CARCINOMA, HEAD AND NECK, SOMATIC |
| PTEN | OMIM: 601728 | 10 | 89707604 | G:A:G;V0I, V0V | MALIGNANT MELANOMA, SOMATIC |
| PTEN | OMIM: 601728 | 10 | 89707656 | G:A:G; R0Q,R0R | MENINGIOMA, INCLUDED |
| PTEN | OMIM: 601728 | 10 | 89710832 | C:T:C;R0*, R0R | PROTEUS-LIKE SYNDROME, INCLUDED |
| PTEN | OMIM: 601728 | 10 | 89707677 | T:C:T;F0S, F0F | MACROCEPHALY/AUTISM SYNDROME |
| PTEN | OMIM: 601728 | 10 | 89682882 | G:A:G; G0E,G0G | COWDEN DISEASE |
| TPM1 | OMIM: 191010 | 15 | 61136240 | G:A:G; E0K,E0E | CARDIOMYOPATHY, DILATED, 1Y |
| TPM1 | OMIM: 191010 | 15 | 61140151 | G:A:G; D0N,D0D | CARDIOMYOPATHY, FAMILIAL HYPERTROPHIC, 3 |
| TPM1 | OMIM: 191010 | 15 | 61141492 | A:G:A; E0G,E0E | CARDIOMYOPATHY, FAMILIAL HYPERTROPHIC, 3 |
| TPM1 | OMIM: 191010 | 15 | 61123282 | G:A:G; E0K,E0E | CARDIOMYOPATHY, DILATED, 1Y |
| TPM1 | OMIM: 191010 | 15 | 61136280 | T:C:T; V0A,V0V | CARDIOMYOPATHY, FAMILIAL HYPERTROPHIC, 3 |
| TPM1 | OMIM: 191010 | 15 | 61123324 | G:A:G; E0K,E0E | CARDIOMYOPATHY, DILATED, 1Y |
| POLG | OMIM: 174763 | 15 | 87663288 | C:G:C; G0R,G0G | SENSORY ATAXIC NEUROPATHY, DYSARTHRIA, AND OPHTHALMOPARESIS |
| POLG | OMIM: 174763 | 15 | 87671241 | C:G:C; Q0H,Q0Q | SPINOCEREBELLAR ATAXIA WITH EPILEPSY |
| POLG | OMIM: 174763 | 15 | 87669874 | G:A:G; P0L,P0P | PROGRESSIVE EXTERNAL OPHTHALMOPLEGIA WITH HYPOGONADISM, INCLUDED |
| POLG | OMIM: 174763 | 15 | 87666012 | G:A:G; R0W,R0R | AUTOSOMAL RECESSIVE |
| POLG | OMIM: 174763 | 15 | 87666027 | C:T:C; G0S,G0G | LEUKOENCEPHALOPATHY, INCLUDED |
| POLG | OMIM: 174763 | 15 | 87665477 | C:A:C;E0*, E0E | ALPERS SYNDROME |

(continued)

| Gene/ Locus | MIM Number | Ch | Position | Variant Genotype | Phenotype |
|---|---|---|---|---|---|
| PLP1 | OMIM: 300401 | X | 102930949 | C:T:C; A0V,A0A | PELIZAEUS-MERZBACHER DISEASE, CONNATAL |
| PLP1 | OMIM: 300401 | X | 102929396 | C:T:C;T0I, T0T | PELIZAEUS-MERZBACHER DISEASE |
| PLP1 | OMIM: 300401 | X | 102929489 | T:C:T;I0T, I0I | SPASTIC PARAPLEGIA 2 |
| PLP1 | OMIM: 300401 | X | 102929438 | C:T:C; S0F,S0S | SPASTIC PARAPLEGIA 2 |
| PLP1 | OMIM: 300401 | X | 102928276 | C:T:C; H0Y,H0H | SPASTIC PARAPLEGIA 2 |
| PLP1 | OMIM: 300401 | X | 102930045 | C:T:C; P0S,P0P | PELIZAEUS-MERZBACHER DISEASE |
| RAPSN | OMIM: 601592 | 11 | 47426960 | C:T:C; V0M,V0V | DEFICIENCY |
| RAPSN | OMIM: 601592 | 11 | 47426207 | G:T:G; N0K,N0N | DEFICIENCY |
| RAPSN | OMIM: 601592 | 11 | 47427052 | A:G:A; L0P,L0L | DEFICIENCY |
| RAPSN | OMIM: 601592 | 11 | 47420908 | G:A:G; A0V,A0A | FETAL AKINESIA DEFORMATION SEQUENCE |
| RAPSN | OMIM: 601592 | 11 | 47425981 | G:A:G; R0C,R0R | DEFICIENCY |
| RAPSN | OMIM: 601592 | 11 | 47425987 | C:T:C; E0K,E0E | DEFICIENCY |
| CBS | OMIM: 236200 | 21 | 43359443 | C:T:C; E0K,E0E | HOMOCYSTINURIA DUE TO CYSTATHIONINE BETA-SYNTHASE DEFICIENCY |
| CBS | OMIM: 236200 | 21 | 43347099 | A:G:A; L0S,L0L | HOMOCYSTINURIA DUE TO CYSTATHIONINE BETA-SYNTHASE DEFICIENCY |
| CBS | OMIM: 236200 | 21 | 43359532 | G:A:G; A0V,A0A | HOMOCYSTINURIA DUE TO CYSTATHIONINE BETA-SYNTHASE DEFICIENCY |
| CBS | OMIM: 236200 | 21 | 43358660 | G:A:G; T0M,T0T | HOMOCYSTINURIA DUE TO CYSTATHIONINE BETA-SYNTHASE DEFICIENCY |
| CBS | OMIM: 236200 | 21 | 43353707 | G:A:G; T0M,T0T | HOMOCYSTINURIA DUE TO CYSTATHIONINE BETA-SYNTHASE DEFICIENCY |
| CBS | OMIM: 236200 | 21 | 43352041 | C:T:C; D0N,D0D | HOMOCYSTINURIA DUE TO CYSTATHIONINE BETA-SYNTHASE DEFICIENCY |
| UMOD | OMIM: 191845 | 16 | 20267360 | C:T:C; C0Y,C0C | HYPERURICEMIC NEPHROPATHY, FAMILIAL JUVENILE 1 |
| UMOD | OMIM: 191845 | 16 | 20267681 | C:T:C; C0Y,C0C | HYPERURICEMIC NEPHROPATHY, FAMILIAL JUVENILE 1 |
| UMOD | OMIM: 191845 | 16 | 20267741 | T:C:T; N0S,N0N | HYPERURICEMIC NEPHROPATHY, FAMILIAL JUVENILE 1 |
| UMOD | OMIM: 191845 | 16 | 20267121 | A:C:A; C0G,C0C | HYPERURICEMIC NEPHROPATHY, FAMILIAL JUVENILE 1 |

(continued)

| Gene/ Locus | MIM Number | Ch | Position | Variant Genotype | Phenotype |
|---|---|---|---|---|---|
| UMOD | OMIM: 191845 | 16 | 20267076 | A:G:A; C0R,C0C | GLOMERULOCYSTIC KIDNEY DISEASE WITH HYPERURICEMIA AND ISOSTHENURIA |
| UMOD | OMIM: 191845 | 16 | 20267475 | A:G:A; C0R,C0C | HYPERURICEMIC NEPHROPATHY, FAMILIAL JUVENILE 1 |
| HEXA | OMIM: 606869 | 15 | 70432524 | C:T:C;R0*, R0R | TAY-SACHS DISEASE |
| HEXA | OMIM: 606869 | 15 | 70430610 | T:G:T; K0T,K0K | GM2-GANGLIOSIDOSIS, LATE ONSET |
| HEXA | OMIM: 606869 | 15 | 70428474 | C:T:C; W0*,W0W | GM2-GANGLIOSIDOSIS, B1 VARIANT |
| HEXA | OMIM: 606869 | 15 | 70428558 | A:C:A; M0R,M0M | TAY-SACHS DISEASE |
| HEXA | OMIM: 606869 | 15 | 70432500 | C:A:C; R0F,R0R | TAY-SACHS DISEASE |
| HEXA | OMIM: 606869 | 15 | 70430589 | T:C:T; H0R,H0H | TAY-SACHS DISEASE |
| COCH | OMIM: 603196 | 14 | 30416643 | T:G:T; V0G,V0V | DEAFNESS, AUTOSOMAL DOMINANT 9 |
| COCH | OMIM: 603196 | 14 | 30417791 | G:A:G; G0E,G0G | DEAFNESS, AUTOSOMAL DOMINANT 9 |
| COCH | OMIM: 603196 | 14 | 30417854 | T:A:T;I0N, I0I | DEAFNESS, AUTOSOMAL DOMINANT 9 |
| COCH | OMIM: 603196 | 14 | 30417883 | G:A:G; A0T,A0A | DEAFNESS, AUTOSOMAL DOMINANT 9 |
| COCH | OMIM: 603196 | 14 | 30417877 | T:C:T; W0R,W0W | DEAFNESS, AUTOSOMAL DOMINANT 9 |
| COCH | OMIM: 603196 | 14 | 30416597 | C:T:C; P0S,P0P | DEAFNESS, AUTOSOMAL DOMINANT 9 |
| DMD | OMIM: 300377 | X | 31406352 | T:A:T;E0V, E0E | DUCHENNE MUSCULAR DYSTROPHY |
| DMD | OMIM: 300377 | X | 31110822 | G:A:G; Q0*,Q0Q | DUCHENNE MUSCULAR DYSTROPHY |
| DMD | OMIM: 300377 | X | 31251663 | G:T:G;S0*, S0S | DUCHENNE MUSCULAR DYSTROPHY |
| DMD | OMIM: 300377 | X | 31702138 | C:A:C;E0*, E0E | DUCHENNE MUSCULAR DYSTROPHY |
| DMD | OMIM: 300377 | X | 31424957 | G:A:G; Q0*,Q0Q | DUCHENNE MUSCULAR DYSTROPHY |
| DMD | OMIM: 300377 | X | 31406319 | T:C:T; H0R,H0H | BECKER MUSCULAR DYSTROPHY |
| NR3C2 | OMIM: 600983 | 4 | 149335426 | G:T:G; C0*,C0C | PSEUDOHYPOALDOSTERONISM, TYPE I, AUTOSOMAL DOMINANT |
| NR3C2 | OMIM: 600983 | 4 | 149222061 | G:A:G; R0*,R0R | PSEUDOHYPOALDOSTERONISM, TYPE I, AUTOSOMAL DOMINANT |

(continued)

| Gene/ Locus | MIM Number | Ch | Position | Variant Genotype | Phenotype |
|---|---|---|---|---|---|
| NR3C1 | OMIM: 600983 | 4 | 149295493 | G:C:G; S0*,S0S | PSEUDOHYPOALDOSTERONISM, TYPE I, AUTOSOMAL DOMINANT |
| NR3C2 | OMIM: 600983 | 4 | 149254733 | A:G:A; L0P,L0L | PSEUDOHYPOALDOSTERONISM, TYPE I, AUTOSOMAL DOMINANT |
| NR3C2 | OMIM: 600983 | 4 | 149295190 | T:C:T; Q0R,Q0Q | PSEUDOHYPOALDOSTERONISM, TYPE I, AUTOSOMAL DOMINANT |
| NR3C2 | OMIM: 600983 | 4 | 149575854 | G:A:G; R0*,R0R | PSEUDOHYPOALDOSTERONISM, TYPE I, AUTOSOMAL DOMINANT |
| MPL | OMIM: 159530 | 1 | 43578354 | C:A:C; P0T,P0P | AMEGAKARYOCYTIC THROMBOCYTOPENIA, CONGENITAL |
| MPL | OMIM: 159530 | 1 | 43591026 | C:T:C; P0L,P0P | AMEGAKARYOCYTIC THROMBOCYTOPENIA, CONGENITAL |
| MPL | OMIM: 159530 | 1 | 43577693 | C:T:C;Q0*, Q0Q | AMEGAKARYOCYTIC THROMBOCYTOPENIA, CONGENITAL |
| MPL | OMIM: 159530 | 1 | 43587525 | G:A:G; W0*,W0W | AMEGAKARYOCYTIC THROMBOCYTOPENIA, CONGENITAL |
| MPL | OMIM: 159530 | 1 | 43587566 | G:A:G; S0N,S0S | THROMBOCYTHEMIA, ESSENTIAL, AUTOSOMAL DOMINANT |
| MPL | OMIM: 159530 | 1 | 43576394 | G:T:G; K0N,K0K | THROMBOCYTHEMIA, SUSCEPTIBILITY TO |
| F10 | OMIM: 227600 | 13 | 112831836 | A:G:A; E0G,E0E | FACTOR X ST. LOUIS-2 |
| F10 | OMIM: 227600 | 13 | 112831856 | G:A:G; E0R,E0E | FACTOR X VORARLBERG |
| F10 | OMIM: 227600 | 13 | 112851485 | T:C:T; S0P,S0S | FACTOR X DEFICIENCY |
| F10 | OMIM: 227600 | 13 | 112851461 | C:T:C; R0C,R0R | FACTOR X SAN ANTONIO-1 |
| F10 | OMIM: 227600 | 13 | 112843287 | G:A:G; E0K,E0E | FACTOR X DEFICIENCY |
| F10 | OMIM: 227600 | 13 | 112831857 | A:G:A; E0R,E0E | FACTOR X KETCHIKAN |
| GLRA1 | OMIM: 138491 | 5 | 151188814 | T:C:T; Y0C,Y0Y | HYPEREKPLEXIA, AUTOSOMAL DOMINANT |
| GLRA1 | OMIM: 138491 | 5 | 151211146 | T:C:T; K0E,K0K | HYPEREKPLEXIA AND SPASTIC PARAPARESIS |
| GLRA1 | OMIM: 138491 | 5 | 151188763 | G:T:G;S0*, S0S | HYPEREKPLEXIA, AUTOSOMAL DOMINANT |
| GLRA1 | OMIM: 138491 | 5 | 151211160 | C:A:C; R0L,R0R | HYPEREKPLEXIA, AUTOSOMAL DOMINANT |
| GLRA1 | OMIM: 138491 | 5 | 151211279 | G:C:G; S0R,S0S | HYPEREKPLEXIA, AUTOSOMAL RECESSIVE |
| GLRA1 | OMIM: 138491 | 5 | 151211194 | C:T:C; V0M,V0V | HYPEREKPLEXIA, AUTOSOMAL DOMINANT |

(continued)

| Gene/ Locus | MIM Number | Ch | Position | Variant Genotype | Phenotype |
|---|---|---|---|---|---|
| FMO3 | OMIM: 136132 | 1 | 169346704 | G:A:G; V0M,V0V | TRIMETHYLAMINURIA |
| FMO3 | OMIM: 136132 | 1 | 169328517 | G:A:G; E0K,E0E | TRIMETHYLAMINURIA |
| FMO3 | OMIM: 136132 | 1 | 169343576 | C:T:C; P0L,P0P | TRIMETHYLAMINURIA |
| FMO3 | OMIM: 136132 | 1 | 169343560 | G:T:G; G0*,G0G | FMO3 ACTIVITY, DECREASED |
| FMO3 | OMIM: 136132 | 1 | 169353081 | C:T:C; R0W,R0R | TRIMETHYLAMINURIA |
| FMO3 | OMIM: 136132 | 1 | 169349883 | G:T:G;E0*, E0E | TRIMETHYLAMINURIA |

**Example 9: Identification of genes and variants causing common multigenic diseases.**

**Power analyses.**

Our goal in these analyses was twofold: first to benchmark the statistical power of VAAST compared to the standard single nucleotide variation (SNV) GWAS approach, and second, to determine the relative contributions of variant frequencies and amino acid substitution frequencies to VAAST's statistical power. We also compared the statistical power of VAAST's default scoring algorithm to that of WSS, one of the most accurate aggregative methods to date for identifying common disease genes using rare variants. **Fig. 6A** shows the results for the *NOD2* gene, implicated in Crohn disease (CD). This dataset contains both rare (minor allele frequency (MAF) <5%) and common variants. **Fib. 6B** shows the same power analysis using *LPL,* a gene implicated in hypertriglyceridemia (HTG). This analysis uses a dataset of 438 re-sequenced subjects. For the *LPL* gene, only rare variants (MAF<5%) were available; therefore, this analysis tests VAAST's ability to detect disease genes for common diseases in which only rare variants contribute to disease risk. To control for Type I error in this analysis, we applied a Bonferroni correction, with the number of tests approximately equal to the number of genes that would be included in a genome-wide analysis (alpha = 0.05/21,000 = $2.4 \times 10^{-6}$).

VAAST rapidly obtains good statistical power even with modest sample sizes; its estimated power is 89% *for* *NOD2* using as few as 150 individuals (alpha= $2.4 \times 10^{-6}$). By comparison, the power of GWAS is less than 4% at the same sample size. Notably, for *NOD2,* nearly 100% power is obtained with VAAST when a GWAS would still have less than 10% power. Also shown is VAAST's power as a function of sample size without the use of amino acid substitution data. The solid line in **Fig. 6A** show the power curves for VAAST using OMIM for its AAS disease models. As can be seen, power is improved when AAS information is used.

In general, the *LPL* results mirror those of *NOD2.* Although VAAST obtained less power using the *LPL* dataset compared to *NOD2,* this was true for every approach. Interestingly, for *NOD2,* BLOSUM attains higher power using smaller sample sizes compared to OMIM. The fact that the trend is reversed for *LPL,* however, suggests that the two AAS models are roughly equivalent. We also compared VAAST's performance to that of WSS another aggregative prioritization method. VAAST achieves greater statistical power than WSS on both datasets, even when VAAST is run without use of AAS information.

**Example 10: Discussion**

VAAST employs a generalized feature-based prioritization approach, aggregating variants to achieve greater statistical search power. VAAST can score both coding and non-coding variants, evaluating the aggregative impact of both types of SNVs simultaneously. Here we have focused on genes, but in principle the tool can be used to search for disease-causing variants in other classes of features as well; for example, regulatory elements, sets of genes belonging to a particular genetic pathway, or genes belonging to a common functional category, e.g. transcription factors.

In contrast to GWAS approaches, which evaluate the statistical significance of frequency differences for individual variants in cases vs. controls, VAAST evaluates the likelihood of observing the aggregate genotype of a feature given a background dataset of control genomes. As our results demonstrate, this approach greatly improves statistical power, in part because it bypasses the need for large statistical corrections for multiple tests.

Much additional statistical power and accuracy are also gained from other components of the VAAST architecture, such as its ability to employ pedigrees, phased datasets, and disease inheritance models. The power of VAAST's pedigree approach is made clear in the quartet-based Miller syndrome analysis shown in **Fig. 4,** where genome-wide only the two disease-causing genes are identified in a genome-wide screen of 19,249 non-synonymous variants. Another important feature of VAAST is its ability to identify and mask variants in repetitive regions of the genome. As our results show, this provides a valuable method for mitigating platform-specific sequencing errors in situations where it is cost-prohibitive to obtain a sufficiently large control set of genomes matched with regard to sequencing and variant calling pipeline. VAAST also differs in important ways from published heuristic search tools such as ANNOVAR. Unlike these tools, VAAST is not designed specifically to identify rare variants responsible for rare diseases. Instead, VAAST can search any collection of variants, regardless of their frequency distributions, to identify genes involved in both rare and common diseases.

Collectively our results make clear the synergy that exists between these various components of the VAAST architecture. For example, they grant VAAST several unique features that distinguish it from commonly used AAS methods such as SIFT. Unlike AAS approaches, VAAST can score all variants, coding and non-coding, and in non-conserved regions of the genome. In addition, VAAST can obtain greater accuracy in judging which variants are deleterious. Comparison of the two Utah Miller syndrome exomes serves to highlight these differences. The two Miller syndrome exomes, for example, share 337 SNVs that are judged deleterious by SIFT; these 337 shared SNVs are distributed among 277 different genes. Thus, although AAS tools such as SIFT are useful for prioritizing the variants within a single known-disease gene for follow-up studies, they are of limited use when carrying out genome-wide disease gene searches, especially when the affected individuals are compound heterozygotes, as in the Miller syndrome examples.

In comparison to SIFT, VAAST scores 20% more non-synonymous SNVs, but identifies only 9 candidate genes (Table 2), with the two disease-causing genes ranked 4th and 5$^{th}$. When run in its pedigree mode, only the four disease-causing variants in the two disease genes are judged deleterious by VAAST genome-wide. The original analysis of the family of four required three months and identified eight potential disease-causing variants in four genes. An exome analysis required four affected individuals in three families to identify *DHODH* as the sole candidate for Miller syndrome. In contrast, using only the data from the family of four, VAAST identified the two disease genes in about 11 minutes using a 24 CPU compute server, and with perfect accuracy. Even when an additional 36,883 synonymous and non-coding regulatory variants are included in this genome-wide screen, only 23 candidate genes are identified, with *DHODH* still ranked 4$^{th}$ and *DNAH5* ranked 1$^{st}$.

Our benchmark analyses using 100 different known diseases and 600 different known disease-causing alleles make it clear that our Miller syndrome and CCD analyses are representative results, and that VAAST is both a very accurate and a very reliable tool. VAAST consistently ranked the disease gene in the top 3 candidates genome-wide for recessive diseases and in the top 9 gene candidates for dominant diseases. Equally important is reliability. VAAST has a much lower variance than either SIFT or ANNOVAR. In the recessive scenario, using 3 compound heterozygous individuals as a case cohort, for 95% of the VAAST runs the disease-causing gene was ranked between 2$^{nd}$ & 10$^{th}$ genome-wide; by comparison ANNOVAR's ranks varied between 67 & 8,762 on the same data sets, and SIFT's varied between 66 & 9,107. Thus VAAST can not only be more accurate, it can also be a more reliable tool. These same analyses also demonstrate that VAAST remains a reliable tool even when confronted with missing data due to phenomena such as missed variants, locus heterogeneity, and phenocopies in the case cohorts. Even when 1/3 of the cohort lacked disease-causing alleles at the locus, the average rank was still 61 for dominant diseases and 21 for recessive diseases **(Fig. 5B).**

VAAST can also be used to search for genes that cause common diseases and to estimate the impact of common alleles on gene function, something tools like ANNOVAR are not able to do. For example, when run over a published collection of 1,454 high-confidence disease-causing and predisposing SNVs from OMIM, VAAST identifies all but 29 (2%) of these SNVs as damaging. ANNOVAR, by comparison, excludes 427 (29%) of these SNVs from further analysis because they are present in the 1000 Genomes Project data, dbSNP130 or in segmentally duplicated regions. These results underscore the advantages of VAAST's probabilistic approach. VAAST can assay the impact of rare variants to identify rare diseases, and both common and rare variants to identify the alleles responsible for common diseases, and it operates equally well on datasets (e.g. *NOD2)* wherein both rare and common variants are contributing to disease. Our common-disease analyses serve to illustrate these points. These results demonstrate that VAAST can achieve close to 100% statistical power on common-disease datasets where a traditional GWAS test has almost no power. We also demonstrate that VAAST's own feature-based scoring significantly outperforms WSS, which like all published aggregative scoring methods does not use AAS information. These analyses also demonstrate another key feature of VAAST: while the controls in the Crohn disease dataset were fully sequenced at *NOD2,* only a small subset of the cases were sequenced, and the rest were genotyped at sites that were polymorphic in the sample. VAAST does well with this mixed dataset. It is likely that VAAST would do even better using a dataset of the same size consisting only of sequence data, as such a cohort would likely contain additional rare variants not detectable with chip-based technologies. Consistent with this hypothesis, VAAST also attains high statistical power compared to traditional GWAS methods on the LPL dataset, which only contains alleles with a frequency of less than 5%. This demonstrates that VAAST can also identify common-disease genes even when they contain no common variants that contribute to disease risk.

These results suggest that VAAST can prove useful for re-analyses of existing GWAS and linkage studies. Targeted VAAST analyses combined with region-specific resequencing around GWAS hits will allow smaller Bonferroni corrections than the genome-wide analyses presented here, which can result in still greater statistical power, especially in light of VAAST's feature-based approach. The same can be true for linkage studies. In addition, because much of the power of VAAST is derived from rare variants and amino acid substitutions, the likelihood of false positives due to linkage disequilibrium with causal variants can be low. Thus, it is likely that VAAST can allow identification of disease genes and causative variants in GWAS datasets in which the relationships of hits to actual disease genes and the causative variants are unclear, and for linkage studies, where only broad spans of statistically significant linkage peaks have been detected to date.

VAAST is compatible with current genomic data standards. Given the size and complexity of personal genomes data, this is not a trivial hurdle for software applications. VAAST uses GFF3 (www.sequenceontology.org/resources/gff3.html) and GVF & VCF (www.1000genomes.org/wiki/Analysis/vcf4.0), standardized file formats for genome annotations and personal genomes data, respectively. The size and heterogeneity of the datasets employed in our analyses make clear VAAST's ability to mine hundreds of genomes and their annotations at a time. We also point out that VAAST has a modular software architecture that makes it easy to add additional scoring methods. Indeed, we have already done so for WSS. This is an important point, as aggregative scoring methods are a rapidly developing area of personal genomics. VAAST thus can provide an easy means to incorporate and compare new scoring methods, lending them its many other functionalities.

To our knowledge, VAAST is the first generalized, probabilistic *ab initio* tool for identifying both rare and common disease-causing variants using personal exomes and genomes. VAAST is a practical, portable, self-contained piece of software that substantially improves upon existing methods with regard to statistical power, flexibility, and scope of use. It is resistant to no-calls, automated, fast, works across all variant frequencies and deals with platform-specific noise.

**Example 11: The use of VAAST to identify an X-linked disorder resulting in lethality in male infants due to N-terminal acetyltransferase deficiency.**

Examples of the clinical power of the VAAST application were published in Rope, A.F., et al. American Journal of Human Genetics (2011), doi: 10.1016/j.ajhg.2011.05.017.

A previously unrecognized syndrome, with the boys having an aged appearance, craniofacial anomalies, hypotonia, global developmental delays, cryptorchidism, and cardiac arrhythmias, was characterized. X-chromosome exon capture and sequencing and a recently developed probabilistic disease-variant discovery algorithm were used to determine its genetic basis.

**Subjects and Methods**

The sample collection and analyses for Families 1 and 2 were approved by the Institutional Review Boards at the University of Utah and the National Human Genome Research Institute, respectively. Blood samples were collected and genomic DNA extracted using alkaline lysis and ethanol precipitation (Gentra Puregene, Qiagen Corp, USA). Two DNA samples from family 1 were extracted from stored formalin-fixed-paraffin-embedded (FFPE) tissues from the prior autopsies of two of the deceased boys. Slices from FFPE tissue blocks were digested overnight in proteinase K, and then boiled for 10 min to inactivate the enzyme. The crude lysates were diluted 1:10 for PCR.

***X-chromosome Exon Capture and Sequencing.*** Exon capture for Family 1 was carried out using a commercially available Agilent in-solution method (SureSelect Human X chromosome kit, Agilent) as per the manufacturer guidelines with minor modifications. The pure and high molecular weight genomic DNA samples were randomly fragmented using a Covaris S series Adaptive Focused Acoustics machine, resulting in DNA fragments with a base pair peak of 150 to 200 bps. Adaptors were then ligated to both ends of the resulting fragments. The adaptor-ligated templates were purified by the Agencourt AMPure SPRI beads. Adapter-ligated products were PCR amplified with Phusion polymerase (6 cycles) and subsequently purified using a Qiagen QIAquick PCR purification kit. The quality and size distribution of the amplified product was assessed on an Agilent Bioanalyzer DNA 1000 chip. Amplified library (500 ng) with a size range of 200-400 bp was hybridized to SureSelect Biotinylated RNA Library (BAITS) for enrichment. Hybridized fragments were bound to the strepavidin beads whereas non-hybridized fragments were washed out after a 24 hour hybridization. The captured library was enriched by PCR amplification (12 cycles) and the amplified product was subsequently qualified on an Agilent High Sensitivity DNA Bioanalyzer chip to verify the size range and quantity of the enriched library. Each captured library was sequenced with 76 bp single-end reads on one lane each of the Illumina GAIIx platform. Raw image files were processed by Illumina Pipeline vl.6 for base-calling with default parameters. All coordinates are based on human genome build hg18. The pseudoautosomal regions were defined based on annotations within the UCSC genome browser. Specifically, these are the regions: chrX: 1-2709520, chrX: 154584238-154913754, chrY: 1-2709520, and chrY:57443438-57772954. For family 2, solution hybridization selection of the X exome (Sure Select, Agilent, Santa Clara, CA) was used to produce a paired-end sequencing library (Illumina, San Diego, CA). Paired-end 75 base reads were generated from the target-selected DNA library in one lane of an Illumina GAIIx. Coding changes were predicted using custom-designed software.

***Sanger Sequence Confirmation Analysis:*** PCR amplification and Sanger sequencing to confirm the mutations and co-segregation were performed. Mutation numbering was performed according to Human Gene Variation Society nomenclature using reference NM_003491.2.

***Sequence Alignment.*** For family 1, sequence reads were converted from Illumina fastq format to fastq files that conform to the Sanger specification for base-quality encoding using perl scripts. BWA version 0.5.8 was used to align the sequencing reads, with default parameters for fragment reads, to the human genome sequence build 36 downloaded from the UCSC Genome Browser or the 1000 Genomes Project website. Alignments were converted from SAM format to sorted, indexed BAM files with SamTools. The picard tool (see sourceforge.net/projects/picard/) was used to remove invalid alignments and remove duplicate reads from the BAM files. Regions surrounding potential indels were re-aligned with the GATK IndelRealigner tool. Variants were called with SamTools pileup command using default parameters except that no upper limit was placed on the depth of coverage for calling variants. In addition, haploid chromosomes were called with the -N 1 options SamTools pileup. Variants were annotated with respect to their impact on coding features using perl scripts and the knownGenes and RefGenes tracks from UCSC Genome Browser.

***Genotype Calling.*** Regions surrounding potential indels were re-aligned with the GATK IndelRealigner tool. Genotypes were called with both SamTools (pileup command) and the GATK UnifiedGenotypeCaller and IndelCaller. The union of single nucleotide variant (SNV) and indel variant calls from GATK and SamTools were then analyzed by the ANNOVAR program to identify exonic variants, and to identify variants not previously reported in the 1000 Genomes Project and the dbSNP version 130. All variant calls not present on chromosome X were removed given the X-linked nature of the disease. The location and genotype of variants for each individual were compared to locate the subset of variants on chromosome X that were heterozygous in female carriers, hemizygous in III-4, and hemizygous reference in the unaffected males. Each candidate variant was also screened for presence/absence in dbSNP 130, the 10Gen Dataset and variant data from the 1000 genomes project.

The read mapping and SNV calling algorithm, GNUMAP, was also independently used to align the reads from the Illumina.qseq files to the X chromosome (human sequence build 36) and to simultaneously call SNVs. GNUMAP utilizes a probabilistic Pair-Hidden Markov Model (PHMM) for base calling and SNV detection that incorporates base uncertainty based on the quality scores from the sequencing run, as well as mapping uncertainty from multiple optimal and sub-optimal alignments of the read to a given location to the genome. In addition, this approach applies a likelihood ratio test that provides researchers with straightforward SNV calling cutoffs based on a p-value cutoff or a false discovery control. Reads were aligned and SNVs called for the five samples. SNV calls for III-4, brother, and uncle were made assuming a haploid genome (because the calls are on the X chromosome) whereas heterozygous calls were allowed for the mother and grandmother. SNVs were selected based on a p-value cutoff of 0.001. Due to the X-linked nature of the disease, candidate SNVs were selected that are heterozygous in the mother and grandmother, and different between the uncle/brother and III-4.

***VAASTAnalysis Method.*** SNV filtering in Table 9 was performed using the SST (simple selection tool) module in VAAST based on the SNV position. Our analysis applied a disease model that did not require complete penetrance or locus homogeneity. We restricted the expected allele frequency of putative disease-causing variants within the control genomes to 0.1% or lower. The background file used in the analysis is composed of variants from dbSNP (version 130), 189 genomes from the 1000 Genomes Project, the 10Gen dataset, 184 Danish exomes, and 40 whole-genomes from the Complete Genomics Diversity Panel. VAAST candidate gene prioritization analysis was performed using the likelihood ratio test under the dominant inheritance model, assuming an expected allele frequency of 0.1% or lower for the causal variant in the general population. After masking out loci of potentially low variant quality, SNVs in each gene were scored as a group. The significance level was assessed using individual permutation tests (the following VAAST analysis parameters: "-m lrt -c X -g 4 -d 1.E8 -r 0.001 -x 35bp_se.wig -less_ram - inheritance d").

**Table 9. Coverage Statistics in Family 1. Based on GNUMAP**

| Region | RefSeq Transcripts | Unique Exons | Percent Exon Coverage ≥1X | Percent Exon Coverage ≥10X | Unique Genes | Average Base Coverage | VAAST Candidate SNVs |
|---|---|---|---|---|---|---|---|
| X-chromosome | 1,959 | 7,486 | 97.8 | 95.6 | 913 | 214.6 | 1 (*NAA10*) |
| chrX: 10054434-40666673 | 262 | 1,259 | 98.1 | 95.9 | 134 | 213.5 | 0 |
| chrX: 138927365-153331900 | 263 | 860 | 97.1 | 94.9 | 132 | 177.1 | 1 (*NAA10*) |

[a]On chromosome X, there are 8,222 unique RefSeq exons. Of these exons, 736 were excluded from the SureSelect X-Chromosome Capture Kit because they were designated as pseudoautosomal or repetitive sequences (UCSC genome browser).

## RESULTS

### *VAAST Analysis*

VAAST (Variant Annotation, Analysis and Selection Tool) which identifies disease-causing variants, was used to analyze the exon capture data from Family 1. VAAST annotated the SNVs for their effect on coding sequences, selected the variants compatible with the pedigree, and performed a statistical analysis on the X chromosome genes to identify the variant(s) most likely to be disease-causing. In the candidate gene identification step, VAAST uses a likelihood ratio test that incorporates both amino acid substitution frequencies as well as allele frequencies to prioritize candidate genes based on SNVs present in those genes. The analyses by VAAST of the variant sets generated from the three variant calling pipelines yielded similar results and identified the same causal mutation in NAA10. With SNVs from the affected child (III-4) alone, VAAST was able to narrow the candidate gene list to fewer than five genes (Table 10). We then filtered the data by only selecting SNVs shared by mother (II-2) and the affected child (III-4). This subset resulted in three, two, and two candidate genes in the GATK, Samtools, and GNUMAP datasets, respectively (Table 10). Next, we filtered the data by selecting SNVs shared by the mother (II-2), maternal grandmother (1-2) and the affected child (III-4). This subset resulted in two, two, and one hits in the three datasets, and NAA10 ranked first in all three lists. When we further excluded the SNVs that were present in the unaffected brother and uncle, VAAST identified a single candidate disease-causing variant in NAA10 in the GATK and the Samtools datasets, and two candidate disease-causing variants in NAA10 and RENBP in the GNUMAP dataset (Table 10).

**Table 10. Summary of the filtering procedure and candidate genes using VAAST**

| SNV calling pipeline | GATK | Samtools | GNUMAP |
|---|---|---|---|
| III-4 (total SNVs) | 1546 | 1499 | 2168 |
| III-4 (nsSNVs) | 146 | 114 | 155 |
| VAAST candidate genes (NAA10 ranking) | 4 (3) | 3 (2) | 5 (2) |
| Present in III-4 and mother II-2 (nsSNVs) | 122 | 107 | 116 |
| VAAST candidate genes (NAA10 ranking) | 3 (2) | 2 (1) | 2 (2) |
| Present in III-4, mother II-2, and grandmother I-2 (nsSNVs) | 115 | 95 | 104 |
| VAAST candidate genes (NAA10 ranking) | 2 (1) | 2 (1) | 1 (1) |
| Present in III-4, II-2, and 1-2, absent in brother III-2 and uncle II-8 (nsSNVs) | 8 | 6 | 8 |
| VAAST candidate genes (NAA10 ranking) | 1 (1) | 1 (1) | 2 (1) |

### *Genome-wide Significance*

We next assessed whether adding members of Family 2 would improve the power of the VAAST analysis. We combined variants from III-4 in family 1 with the obligate carrier mother (II-2) in family 2 and performed VAAST analysis on the 216 coding SNVs present in either of the two individuals. After incorporating the mother from family 2, NAA10 was the only candidate gene, and the result was statistically significant (p-value 3.8x10-5, Bonferroni corrected p-value 3.8 x 10-5 x 729 = 0.028).

## Discussion

The results demonstrate that a probabilistic disease-causing variant discovery algorithm can readily identify and characterize the genetic basis of a previously unrecognized X-linked syndrome. The algorithm, when used in parallel with high-throughput sequencing, can identify variants with high prioritization for causing disease using as few as two individuals. In this instance, ~150 variants distributed among ~2000 transcripts on the X-chromosome in one sample from III-4 in Family 1 were screened. With no prior filtering, 3-5 possible candidate genes were prioritized, with the mutation in NAA10 ranked second overall (Table 10). Including exon capture data from relatives increased NAA10's ranking to first overall in just one family. Furthermore, after combining variants from the proband in family 1 with the obligate carrier mother in family 2, VAAST identified NAA10 as the only statistically significant candidate.

**Example 12: Applications of VAAST**

**Rare genetic disease gene discovery and treatment using family-based sequencing.** Discovery of the Ogden Syndrome, where VAAST was used to discover the disease-causing variant in a novel disease gene *NAA10.* In this case an extensive family tree of affected and non-affected individuals where sequenced and then analyzed with VAAST, which scored, ranked and identified the causative mutation on Chromosome X in this family analysis. This is a classical example of a severe, rare genetic disease. Greater than 1% of all children born in the US have a severe heritable abnormality, and might be candidates for such an analysis. In our case, all genes on Chromosome X were sequenced, but in other studies exomes (all genes) or whole-genome sequencing has been applied. As a consequence, for future pregnancies the mother can now be screened for the mutation causing Ogden Syndrome and she is able to have a health boy. Other approaches produced 25+ "hits", which can be too expensive for a clinical follow-up validation test such as family genotyping or functional assays. The use of VAAST can enable a scientist or clinician to reduce the time and effort required to validate identified genes. VAAST can be more like a clinical diagnostic test.

**Rare genetics disease gene discovery and treatment based on a set of rare genetic disease phenotypes. Table 3** in the application shows an example how VAAST was applied to a set of individuals (6) with severe phenotypes and where running it against a background database of "healthy" or "average" individuals identified the disease causing genes. It also shows the use of a P-value as an indicator or statistically significance and ranking of the findings.

**Common disease searches for genes and alleles responsible for the disease.** As described in Yandell et al 2011 GR June 23rd, and specifically in **Figure 6** in the patent application above, VAAST can also be used to search for genes and alleles responsible for common diseases such as Crohn's Disease; other examples-but not limited to-include Autism, and cardiovascular diseases. Because VAAST has an explicit model for locus heterogeneity, its probabilistic model makes it a more powerful analysis tool than existing GWAS methodologies (Yandell et al GR 2011 July 7th) for these analyses.

**Tumor growth mutation detection.** VAAST methodology can be used to search target genome(s) of tumors ("somatic variant files") against a database of germline genomes to identify the most relevant somatic tumor variants, sometimes also called "driver mutations". These somatic tumor mutations could then be linked to potential pathways and treatment options. The tumor target genomes can be from the same type of cancer or a mixture of cancers.

**Tumor growth speed and metastasis mutations in somatic variant files.** VAAST can be used to search a database of fast growing tumors versus slow growing tumors (background), or metastatic tumors vs. non-mestatic tumors, to identify the variants that cause the different growth rates. In addition, family tree information can be added as described in the application above. It can also be used with a set of tumor genomes extracted at different time points or different tumor tissue samples (dealing with tumor heterogeneity).

**Application of VAAST with oncogenic pathway option.** Using the pathway option, which restricts the scoring only to oncogenic pathways, for which drugs might exist, VAAST can be used to identify variants in a target tumor genome versus a background genome where a pathway is a feature so a highly perturbed pathway might require a certain drug or even an adjusted dose of treatment. In this instance VAAST scores the pathway as a feature and not only a single gene.

**Analysis of tumor genomes for the identification of novel targets.** VAAST methodology can be used to score the mutational profile of a certain sub-type of tumor growth by analyzing the higher order rearrangement of a genomic region in a case control type. Analyzing higher order rearrangements with respect to the underlying genes involved can identify by VAAST scoring the mutations/rearrangements with the highest effect on tumor growth, progression severness. VAAST can in this way identify subtypes of higher-order tumor genomes that might respond to a certain drug the same way, and therefore personalized treatment for the subtype can be prescribed. mutation severity is estimated by VAAST by analyzing target and background genomes.

**Model organism-based cancer/tumorgenesis studies.** Model organism-based cancer/ tumorgenesis studies involving searches for both causative alleles and secondary mutations promoting metastasis and adverse outcomes. In these analyses, suitable background files would be obtained by re-sequencing of reference strains of model organisms; target data would be obtained by sequencing either strains with known predispositions to genetic disease/cancer/tumors or direct sequencing of tumors/metastases from these animals.

**Agricultural analyses.** VAAST is used to identify the genes and variants underlying the characteristic traits of plant cultivars, animal breeds, and wild populations of flora and fauna. In these analyses, background data would be obtained from sequencing of appropriate reference stains/cultivars for purposes of comparisons to target datasets consisting of individuals manifesting the desired trait.

**Pharmacogenomic searches for genes and alleles responsible for adverse drug reactions or varying drug efficacy effects.** These analyses would be carried out using the standard approaches outlined in Yandell et al 2011 GR June 23rd. Target data would be derived from resequencing of individuals who had experienced an adverse reaction, or varying efficacy responses, to a drug of interest.

**Chemotherapeutic applications.** Broadly speaking, these applications would include the use of VAAST to assay sequenced cancer genomes to look for mutations in Cancers/tumors that have occurred in response to previous rounds of chemotherapy. This information would be used to tailor subsequent therapies in order to avoid administration of drugs to which the cancer/tumor has already acquired resistant mutations. The goals of these analyses would be to custom tailor the patient's chemotherapy for maximum efficacy.

**Pathway restricted VAAST analysis.** For an individual patient, VAAST analysis can be restricted to a certain disease pathway or set of disease genes. With a background database of healthy genomes (at least for the selected disease) VAAST will identify the mutations in the pathway/gene sets that are most significant for an individuals and predict risk for this individual and even identifying the most dangerous variants within that pathway on an individual basis.

**Centennials analysis.** VAAST can be used to identify variants in personal genomes that promote health and long life by comparing variant files from individuals lived for a long time versus variants files from individuals that had a shorter life span. This can be used to identify variants and genes that contribute to long life and vice versa short life or disease.

## Claims

1. A computer implemented method for identifying disease-causing genetic variants within a feature, comprising:

    a) providing a genome variant file from at least one individual;
    b) annotating the variants, including annotation with respect to their impact on existing genome annotations, wherein the annotating is in respect to:

        - whether the variant is coding, non-coding, synonymous, non-synonymous, loss-of-function, stop codon causing, changes top regulatory elements, splice sites, or
        - known variant frequencies in a population;

    c) selecting a feature, wherein the feature comprises any span or collection of spans of a genome sequence or transcriptome sequence, and wherein the feature comprises a gene, a transcript, an exon, an intron, a UTR, a genetic locus, an extended gene region including regulatory elements, a list of 2 or more genes, a genetic pathway, an ontology category, or a combination thereof; and
    d) scoring the variants within the feature(s), wherein the scoring:

        i) compares composite allele frequencies in target genome(s) for the selected features in the genome variant file with the corresponding allele frequencies in a background database for selected features;
        ii) computes a statistical probability of each variant and as a composite in the selected feature; and
        iii) ranks variants and their corresponding features according to the statistical probability of each variant and/or of composite feature genotype(s);

    and wherein the scoring is based on calculating a composite likelihood ratio.

2. The method of Claim 1, wherein the genome variant file is from whole genome sequencing, exome sequencing, transcriptome sequencing, chip and bead-type genotyping or a combination of these.

3. The method of Claim 1, wherein the genome variant files are from multiple individuals.

4. The method of Claim 3, wherein the multiple individuals are genetically related.

5. The method of Claim 4, wherein a pedigree information is analysed.

6. The method of Claim 1, wherein the genome variant file comes from a tumor sample.

7. The method of Claim 6, wherein the background database comes from a normal genome.

8. The method of Claim 2, wherein the genome variant file is from a set of cases all with a known phenotype.

9. The method of Claim 8, wherein the background database is from a set of controls without the known phenotype.

10. The method of any one of Claims 1-9, wherein the known variant frequencies are derived from various disease variant databases such as OMIM.

11. The method of any one of Claims 1-10, wherein ontology and pathway information is incorporated into the scoring.

12. The method of any one of Claims 1-11, wherein the disease is a human disease.

13. The method of Claim 12, wherein the variants identify disease genes.

## Patentansprüche

1. Ein computerimplementiertes Verfahren für die Identifizierung von krankheitserregenden genetischen Varianten in einem Merkmal, umfassend:

   a) Bereitstellen einer Datei zu Genomvarianten von mindestens einem Individuum;
   b) Annotation der Varianten, einschließlich der Annotation hinsichtlich ihrer Auswirkung auf bestehende Genom-Annotationen, wobei die Annotation in folgendem Bezug erfolgt:

      - ob die Variante codierend, nicht-codierend, synonym, nicht-synonym, eine Funktionsverlustmutation ist, Stopcodon verursacht, regulatorische Kopfelemente ändert, eine Spleißstellenmutation oder eine
      - bekannte Variantenhäufigkeit in einer Population ist;

   c) Auswählen eines Merkmals, wobei das Merkmal jeden Abschnitt oder jede Sammlung von Abschnitten einer Genomsequenz oder Transkriptomsequenz umfasst, und wobei das Merkmal ein Gen, ein Transkript, ein Exon, ein Intron, ein UTR, einen Genlocus, eine erweiterte Genregion, einschließlich regulatorischer Elemente, eine Liste von 2 oder mehr Genen, einen genetischen Pfad, eine ontologische Kategorie oder eine Kombination daraus umfasst; und
   d) Auswerten der Varianten innerhalb des Merkmals/der Merkmale, wobei das Auswerten:

      i) die gesamten Allelhäufigkeiten im/in den Zielgenom(en) für die ausgewählten Merkmale in der Genomvarianten-Datei mit den entsprechenden Allelhäufigkeiten in einer Hintergrunddatenbank für ausgewählte Merkmale vergleicht;
      ii) eine statistische Wahrscheinlichkeit für jede Variante und eine Gesamtheit in dem ausgewählten Merkmal berechnet; und
      iii) die Varianten und ihre entsprechenden Merkmale gemäß der statistischen Wahrscheinlichkeit jeder Variante und/oder der Gesamtheit des Merkmal-Genotyps/der Merkmal-Genotypen klassifiziert;

   und wobei die Auswertung auf der Berechnung eines Gesamtwahrscheinlichkeits-Verhältnisses basiert.

2. Das Verfahren nach Anspruch 1, wobei die Genomvarianten-Datei von der gesamten Genom-Sequenzierung, Exom-Sequenzierung, Transkriptom-Sequenzierung, chip- und perlenartigen Genotypisierung oder einer Kombination daraus stammt.

3. Das Verfahren nach Anspruch 1, wobei die Genomvarianten-Dateien von mehreren Individuen stammen.

4. Das Verfahren nach Anspruch 3, wobei die mehrere Individuen genetisch verwandt sind.

5. Das Verfahren nach Anspruch 4, wobei eine Stammbauminformation analysiert wird.

6. Das Verfahren nach Anspruch 1, wobei die Genomvarianten-Datei von einer Tumorprobe stammt.

7. Das Verfahren nach Anspruch 6, wobei die Hintergrunddatenbank von einem anomalen Genom stammt.

8. Das Verfahren nach Anspruch 2, wobei die Genomvarianten-Datei von einem Satz von Fällen stammt, die alle einen bekannten Phänotyp haben.

9. Das Verfahren nach Anspruch 8, wobei die Hintergrunddatenbank von einem Satz von Kontrollen stammt, die den bekannten Phänotyp nicht aufweisen.

**10.** Das Verfahren nach einem der Ansprüche 1-9, wobei die bekannten Variantenhäufigkeiten von verschiedenen Datenbanken zu Krankheitsvarianten, wie OMIM, stammen.

**11.** Das Verfahren nach einem der Ansprüche 1-10, wobei Ontologie und Pfadinformationen in die Auswertung aufgenommen werden.

**12.** Das Verfahren nach einem der Ansprüche 1-11, wobei die Krankheit eine menschliche Krankheit ist.

**13.** Das Verfahren nach Anspruch 12, wobei die Varianten Krankheitsgene identifizieren.

**Revendications**

**1.** Procédé mis en œuvre par ordinateur destiné à identifier les variantes génétiques responsables de maladies au sein d'une caractéristique, comprenant :

a) la fourniture d'un fichier de variantes génomiques provenant d'au moins un individu ;
b) l'annotation des variantes, comportant l'annotation par rapport à leur impact sur les annotations existantes du génome, dans lequel l'annotation concerne :

- si oui ou non la variante est codante, non codante, synonyme, non synonyme, provoquant une perte de fonction, un codon d'arrêt, change les principaux éléments réglementaires, les sites d'épissure, ou
- les fréquences de variante connues dans une population ;

c) la sélection d'une caractéristique, dans lequel la caractéristique comprend toute travée ou collection de travées d'une séquence génomique ou d'une séquence transcriptomique et dans lequel la caractéristique comprend un gène, une transcription, un exon, un intron, une région non traduite (UTR), un locus génétique, une région étendue de gènes comportant des éléments de régulation, une liste de 2 gènes ou plus, une voie génétique, une catégorie d'ontologie ou une combinaison de ceux-ci ; et
d) la notation des variantes au sein de la ou des caractéristiques, dans lequel la notation :

i) compare les fréquences allèliques composites dans le ou les génomes cibles pour les caractéristiques sélectionnées dans le fichier de variantes de génome avec les fréquences allèliques correspondantes dans une base de données d'arrière-plan pour les caractéristiques sélectionnées ;
ii) calcule une probabilité statistique de chaque variante et en tant que composite dans la caractéristique sélectionnée ; et
iii) classe les variantes et leurs caractéristiques correspondantes en fonction de la probabilité statistique de chaque variante et/ou du ou des génotypes de la caractéristique composite ;

et dans lequel la notation est basée sur le calcul d'un rapport de probabilité composite.

**2.** Procédé selon la revendication 1, dans lequel le fichier de variantes génomiques est en provenance du séquençage du génome entier, du séquençage de l'exome, du séquençage du transcriptome, du génotypage du type puce et perle ou d'une combinaison de ceux-ci.

**3.** Procédé selon la revendication 1, dans lequel les fichiers de variantes génomiques proviennent de multiples individus.

**4.** Procédé selon la revendication 3, dans lequel les multiples individus sont apparentés génétiquement.

**5.** Procédé selon la revendication 4, dans lequel une information d'ascendance est analysée.

**6.** Procédé selon la revendication 1, dans lequel le fichier de variantes génomiques provient d'un échantillon de tumeur.

**7.** Procédé selon la revendication 6, dans lequel la base de données d'arrière-plan provient du génome anormal.

**8.** Procédé selon la revendication 2, dans lequel le fichier de variantes génomiques est en provenance d'un ensemble de cas comportant tous un phénotype connu.

**9.** Procédé selon la revendication 8, dans lequel la base de données d'arrière-plan est en provenance d'un ensemble de contrôles sans le phénotype connu.

**10.** Procédé selon l'une quelconque des revendications 1 à 9, dans lequel les fréquences de variantes connues sont dérivées depuis diverses bases de données variantes de maladies telles que OMIM.

**11.** Procédé selon l'une quelconque des revendications 1 à 10, dans lequel les informations d'ontologie et de voie sont incorporées dans la notation.

**12.** Procédé selon l'une quelconque des revendications 1 à 11, dans lequel la maladie est une maladie humaine.

**13.** Procédé selon la revendication 12, dans lequel les variantes identifient les gènes de la maladie.

FIG. 1

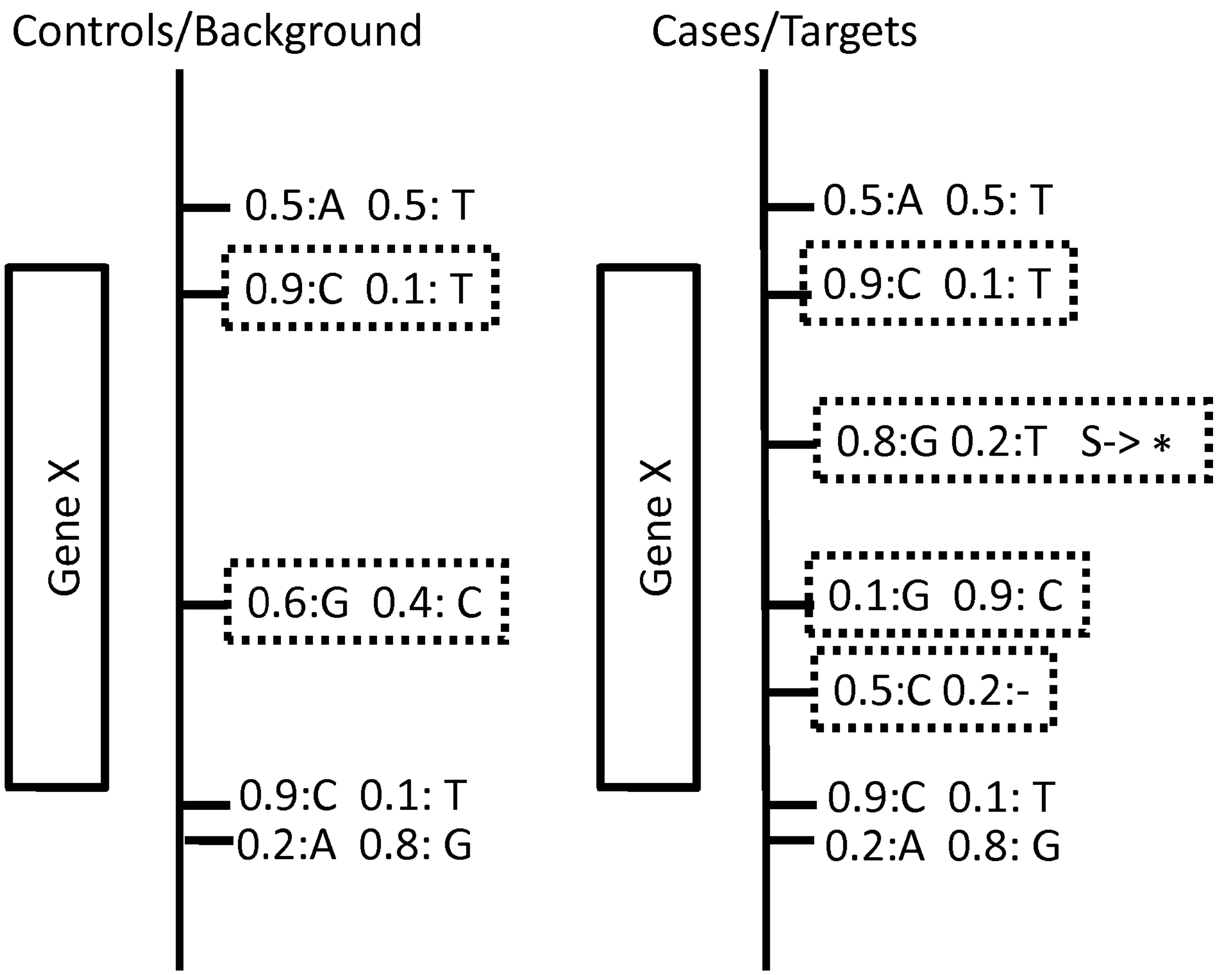
Controls/Background
Cases/Targets
Gene X
0.5:A 0.5: T
0.9:C 0.1: T
0.6:G 0.4: C
0.9:C 0.1: T
0.2:A 0.8: G
Gene X
0.5:A 0.5: T
0.9:C 0.1: T
0.8:G 0.2:T S-> *
0.1:G 0.9: C
0.5:C 0.2:-
0.9:C 0.1: T
0.2:A 0.8: G

FIG. 2

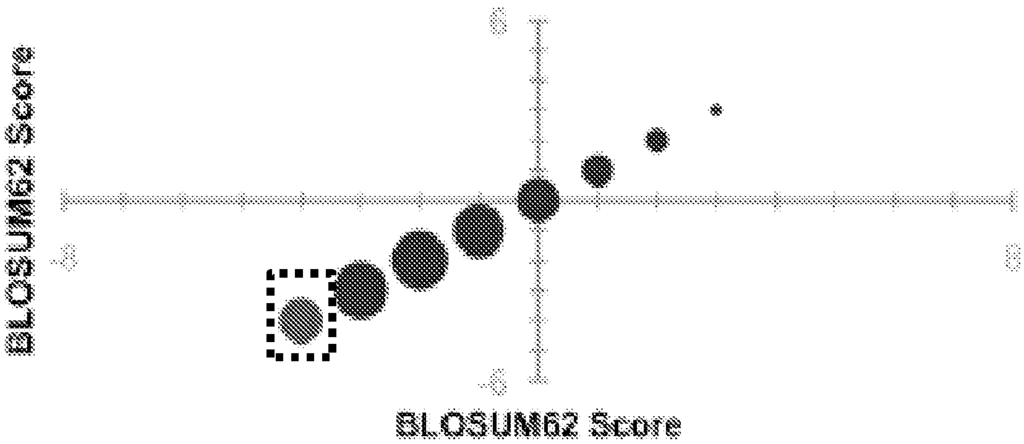


A. Blosum 62 *versus* Blosum 62

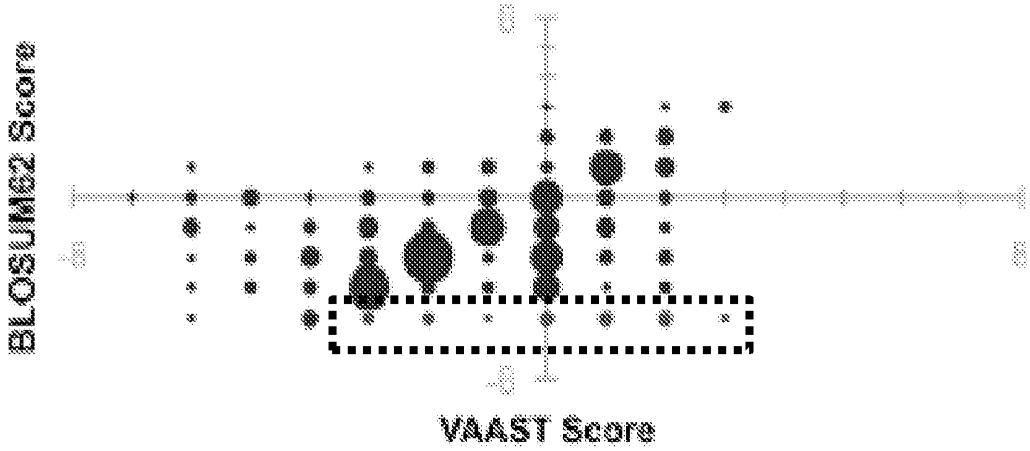


B. Healthy genomes *versus* Blosum 62

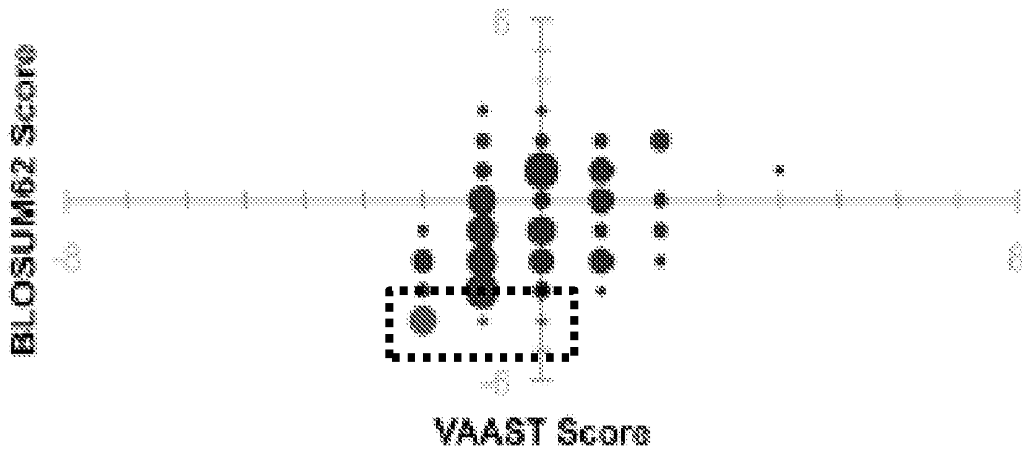


C. OMIM alleles *versus* Blosum 62

FIG. 3

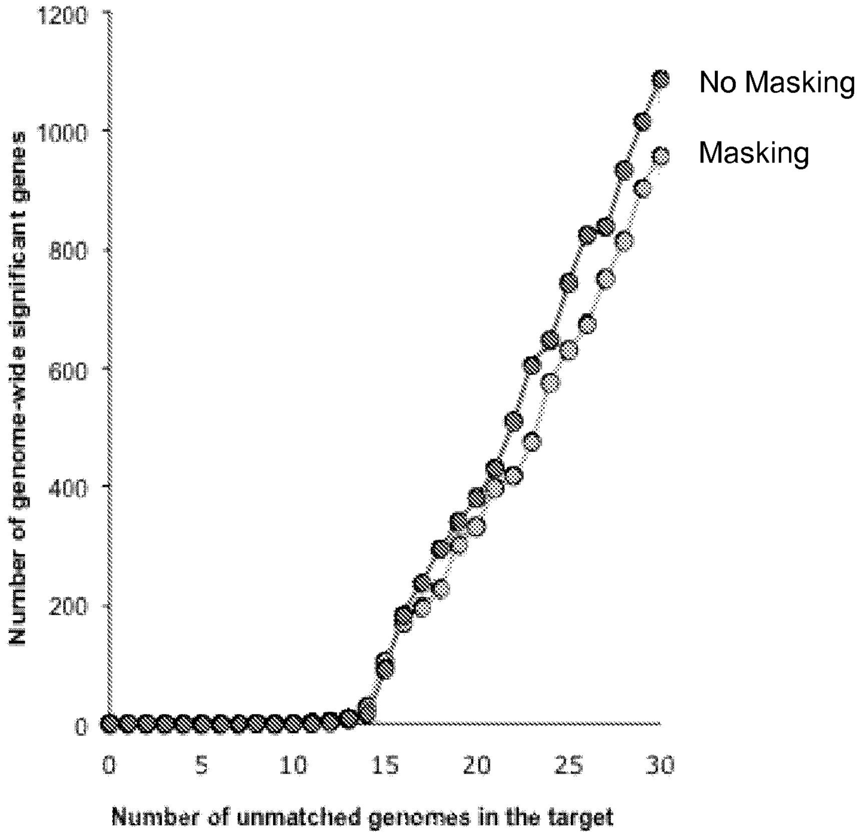
No Masking
Masking
Number of genome-wide significant genes
Number of unmatched genomes in the target
0
200
400
600
800
1000
1200
0
5
10
15
20
25
30

A.

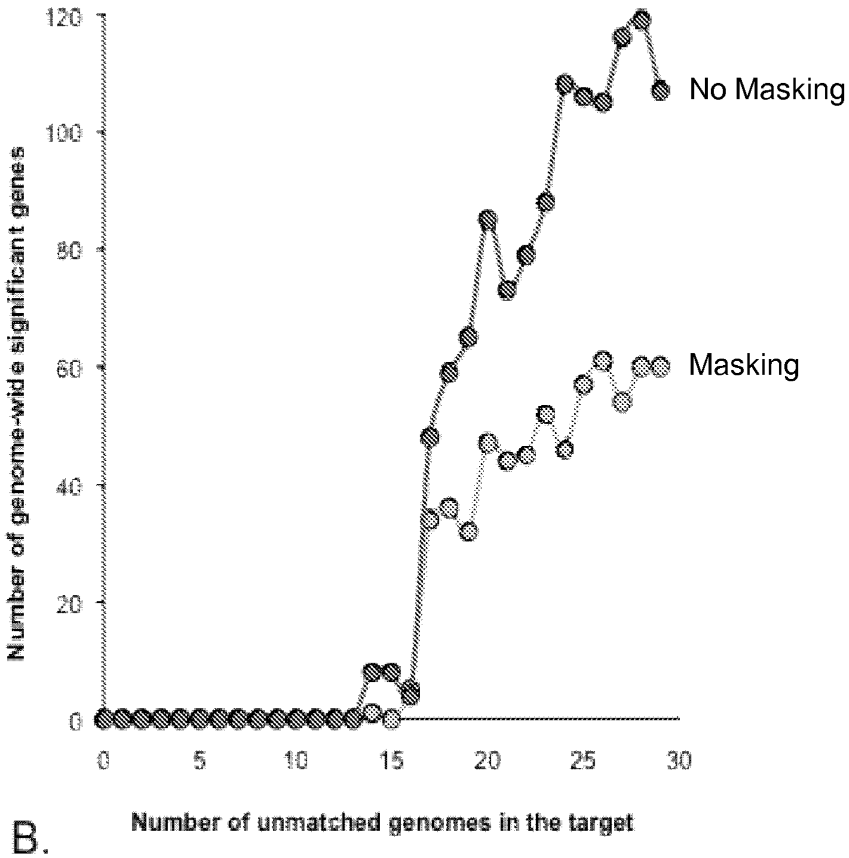
No Masking
Masking
Number of genome-wide significant genes
Number of unmatched genomes in the target
0
20
40
60
80
100
120
0
5
10
15
20
25
30

B.

**FIG. 4**

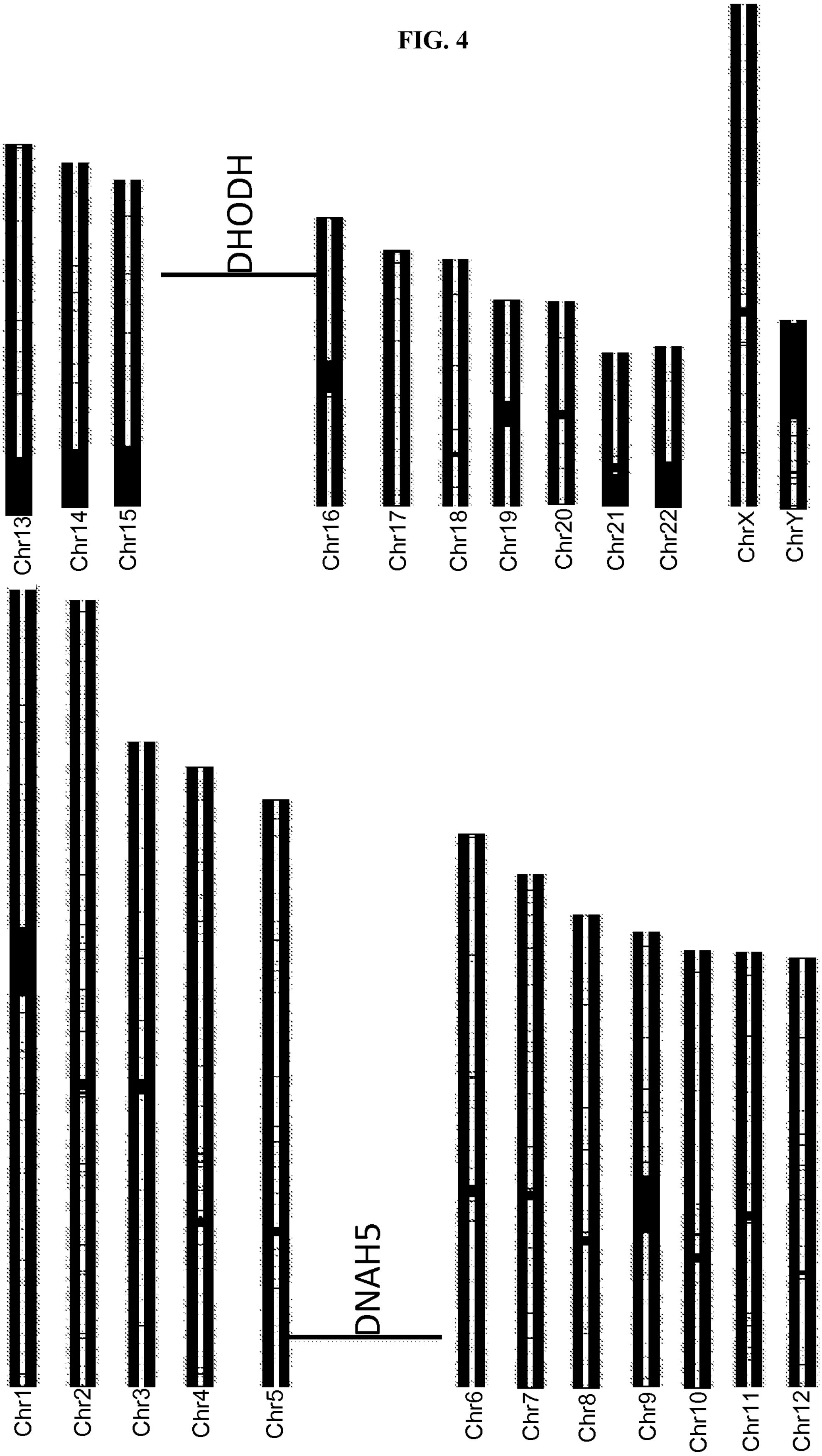
DHODH
Chr13
Chr14
Chr15
Chr16
Chr17
Chr18
Chr19
Chr20
Chr21
Chr22
ChrX
ChrY
DNAH5
Chr1
Chr2
Chr3
Chr4
Chr5
Chr6
Chr7
Chr8
Chr9
Chr10
Chr11
Chr12

FIG. 5

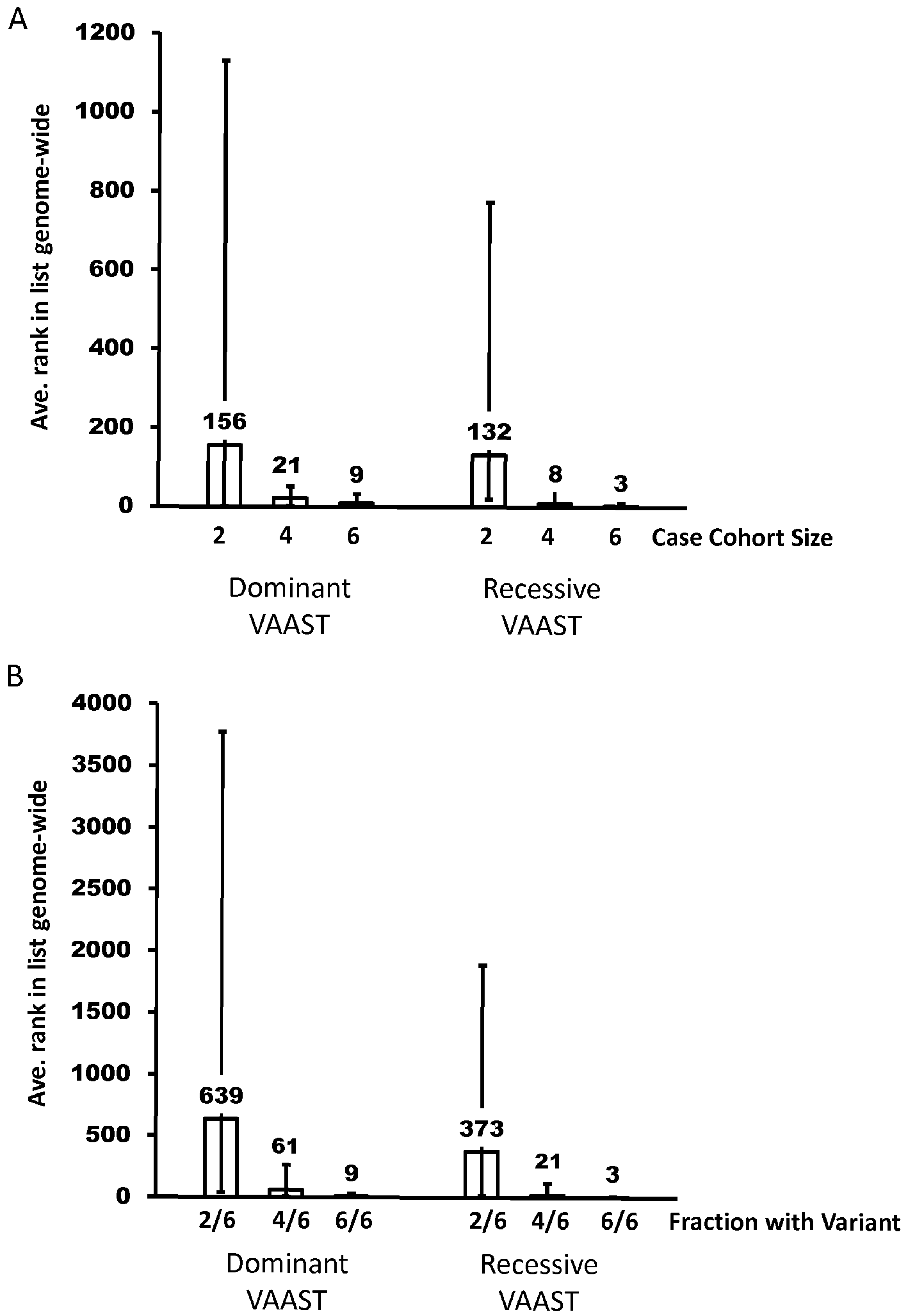
A
Ave. rank in list genome-wide
1200
1000
800
600
400
200
0
156
21
9
132
8
3
2
4
6
2
4
6
Case Cohort Size
Dominant
VAAST
Recessive
VAAST
B
Ave. rank in list genome-wide
4000
3500
3000
2500
2000
1500
1000
500
0
639
61
9
373
21
3
2/6
4/6
6/6
2/6
4/6
6/6
Fraction with Variant
Dominant
VAAST
Recessive
VAAST

FIG. 5 Con't

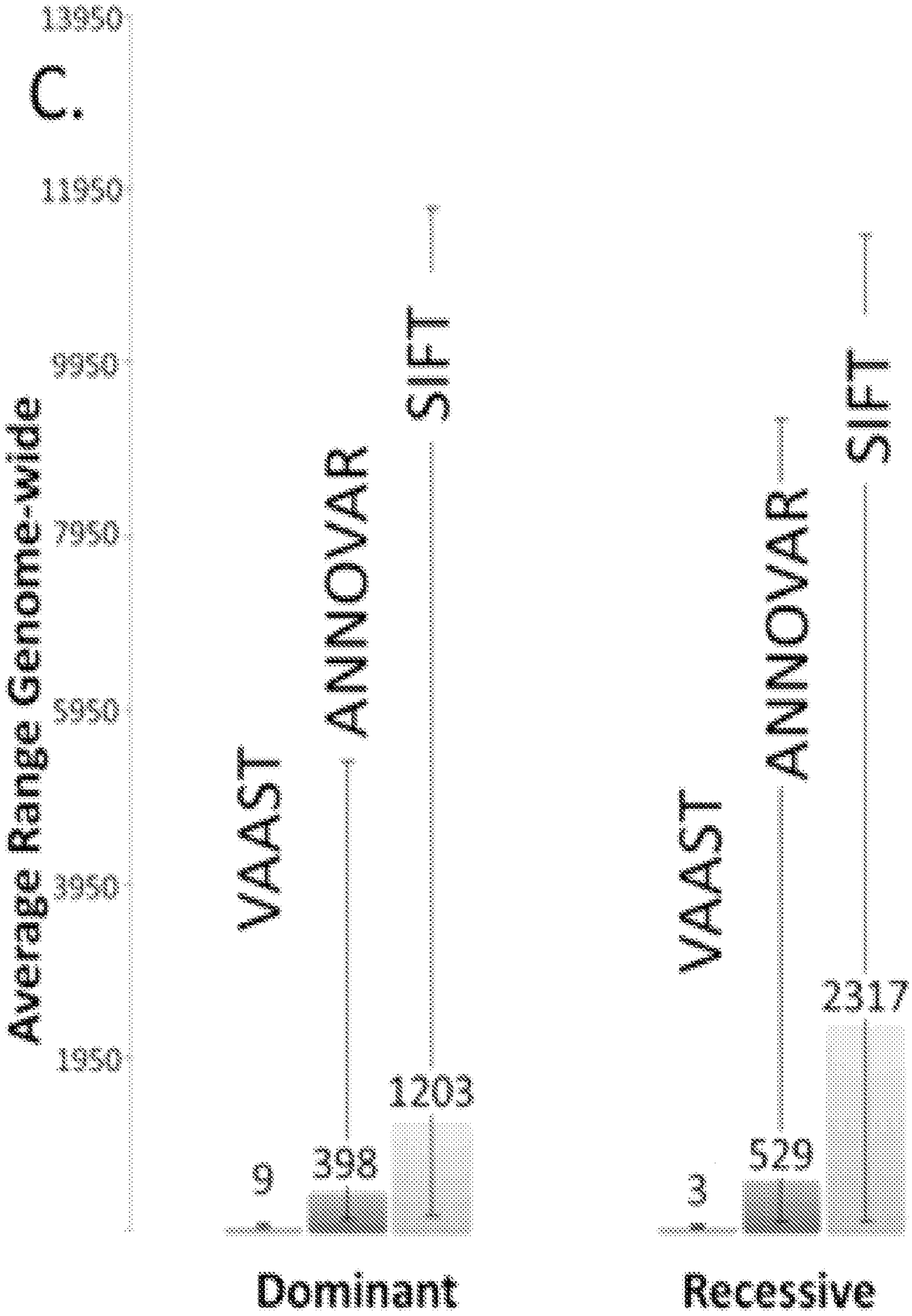
C.
Average Range Genome-wide
13950
11950
9950
7950
5950
3950
1950
VAAST
ANNOVAR
SIFT
9
398
1203
3
529
2317
Dominant
Recessive

FIG. 6

A

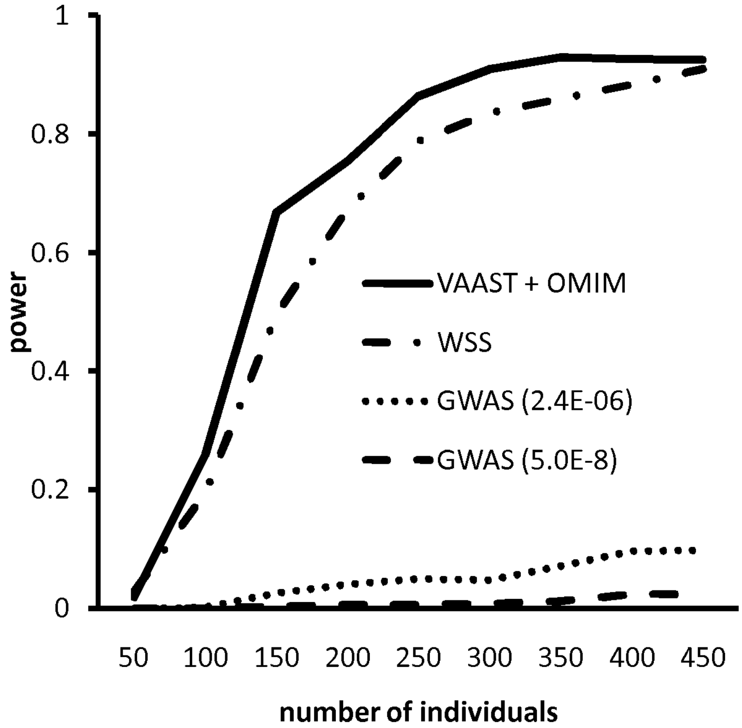


Crohn Disease (CARD15)

B

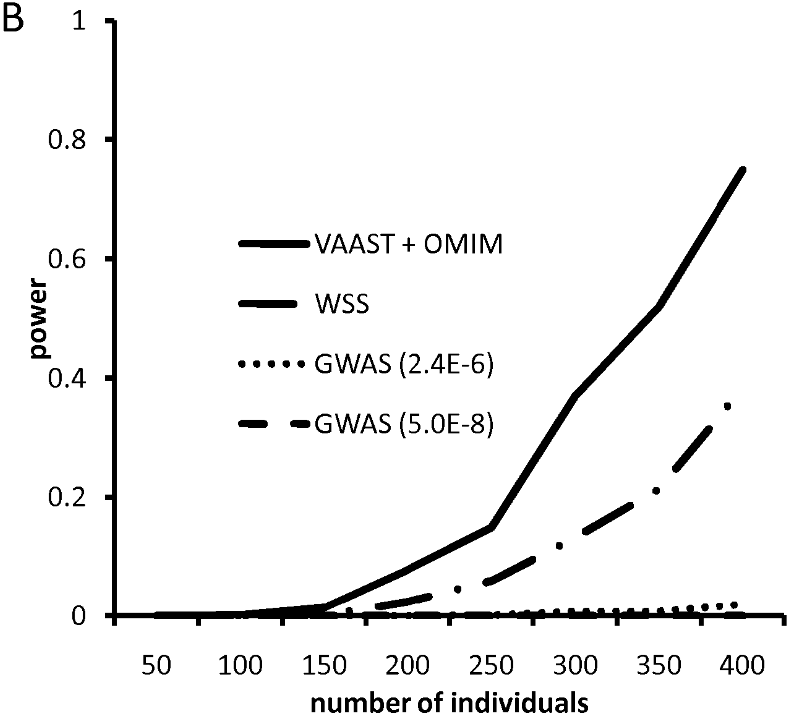


hypertriglyceridemia (LPL)

FIG. 7

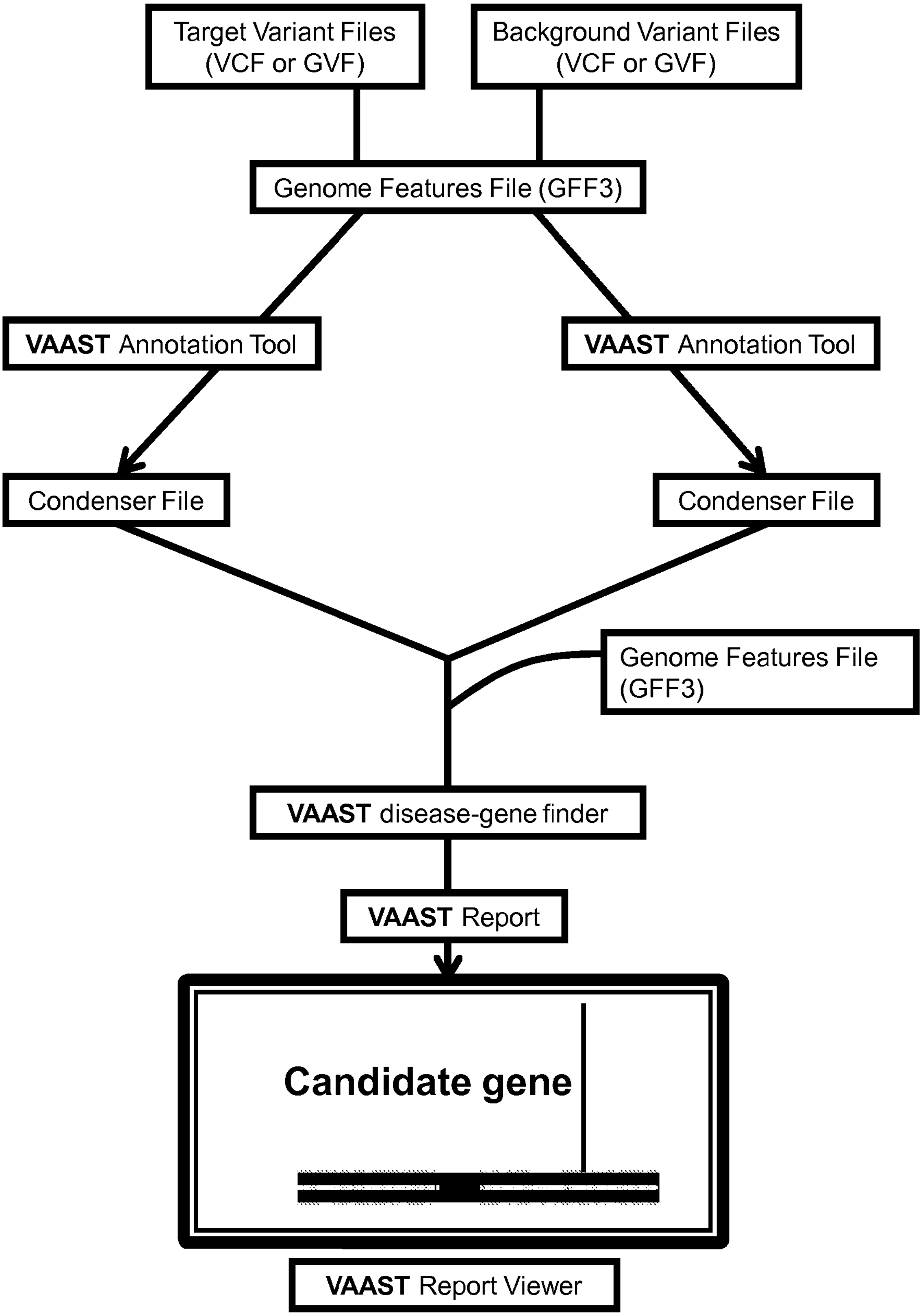
Target Variant Files (VCF or GVF)
Background Variant Files (VCF or GVF)
Genome Features File (GFF3)
VAAST Annotation Tool
VAAST Annotation Tool
Condenser File
Condenser File
Genome Features File (GFF3)
VAAST disease-gene finder
VAAST Report
Candidate gene
VAAST Report Viewer

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2009183268 A1


### Non-patent literature cited in the description


- **M. YANDELL et al.** *Genome Research,* 23 June 2011, vol. 21 (9
- **MADSEN ; BROWNING.** *PLoS Genet,* 2009, 5
- **LESAGE et al.** *Am J Hum Genet,* 2002, vol. 70, 845-857
- **JOHANSEN et al.** *Nat Genet,* 2010, vol. 42, 684-687
- *Nat Genet.,* January 2010, vol. 42 (1), 30-5
- *Nat Methods.,* May 2010, vol. 7 (5), 350
- **MADSEN ; BROWNING.** *PLoS Genet.,* February 2009, vol. 5 (2), e1000384
- **YANDELL M et al.** *Genome Res.,* 07 July 2011
- **MORGENTHALER ; THILLY.** *Mutat Res,* 2007, vol. 615, 28-56
- **LI ; LEAL.** *Am J Hum Genet,* 2008, vol. 83, 311-321
- **LIU ; LEAL.** *PLoS Genet,* 2010, vol. 6, e1001156
- **REESE et al.** *Genome Biol,* 2010, vol. 11, R88
- **CONSORTIUM.** *Nature,* 2010, vol. 467, 1061-1073
- **LAUSCH et al.** *Am J Hum Genet,* 2008, vol. 83 (5), 649-55
- **KUMAR et al.** *Nat Protoc,* 2009, vol. 4, 1073-1081
- **ROPE, A.F. et al.** *American Journal of Human Genetics,* 2011